# EUROPEAN PATENT APPLICATION

(11) **EP 2 517 726 A2**
(43) Date of publication of application: **31.10.2012**
(21) Application number: 10838441.3
(22) Date of filing: 22.12.2010
(51) Int. Cl.: A61K 39/12, C12N 15/86, C12N 15/49, C12N 15/66, A61P 31/18

(54) **LENTIVIRUS VACCINE BASED ON THE RECOMBINANT VIRAL VACCINE AGAINST YELLOW FEVER**

(30) Priority: 23.12.2009 BR 09120090
(71) Applicant: Fundação Oswaldo Cruz (Fiocruz), 21040-900 Rio de Janeiro, RJ (BR); Wisconsin Alumni Research Foundation - WARF, Madison, Wisconsin 53762 (US)
(72) Inventor: BONALDO, Myrna Cristina, 22743-720 Rio de Janeiro, RJ (BR); GALLER, Ricardo, Niterói, RJ, CEP: 24320-000 (BR); WATKINS. David Ian, Arena WI 53503 (US); SACHA, Jonah Bradley, Madison WI 53717 (US)
(74) Representative: Fritz, Edmund Lothar
(86) International application number: PCT/BR2010/000430
(87) International publication number: WO 2011/075806

(57) **Abstract**

The present invention relates to an attenuated recombinant vaccine virus of the yellow fever which expresses heterologous sequences of a lentivirus and is used as an immunization agent to induce an immune response to lentivirus.

## Description

### FIELD OF THE INVENTION

The present invention relates to the development of a vaccine against lentivirus, and more specifically against primate lentiviruses such as HIV (Human Immunodeficiency Virus) and SIV (Simian Immunodeficiency Virus).

The present invention concerns the use of attenuated recombinant yellow fever vaccine virus 17D for expression of HIV and SIV antigens for immunization. The invention provides immunization schemes using yellow fever virus expressing HIV or SIV antigens. For example, the recombinant virus may be used in a "prime-boost" protocol immunizing an individual with a recombinant polynucleotide or recombinant Bacillus Calmette-Guérin (rBCG) vaccine, which expresses one or more HIV or SIV antigens, followed by booster dose with recombinant Yellow Fever virus 17D, which expresses one or more HIV or SIV antigens.

Part of the described material has been developed with the financial support of the National Institutes of Health of the United States of America under number [identification code] R01 AI076114. Thus, the North American government may have certain rights concerning the described technical material.

### BACKGROUNG OF THE ART

Over the 28-year period since the scientists identified the human immunodeficiency virus (HIV) as the cause of the acquired immunodeficiency syndrome (AIDS), the virus has spread inexorably, giving rise to one of the most devastating pandemics ever recorded in history. More than 20 million people have already died of AIDS and about 33 million people are living with HIV infection.

However, none of the vaccine regimens tested in HIV vaccine efficacy trials to date has reduced HIV infection or replication rates.

Structural aspects and the enormous variability of the Envelope glycoprotein have frustrated the efforts to broadly induce reactive neutralizing antibodies against HIV [1]. Investigators have therefore focused their attention on T-cell-based vaccines. The recent trial of a recombinant adenovirus[Ad5]-vectored vaccine was widely seen as an important test of this concept [3,4]. Unfortunately, vaccinees became infected at higher rates than control groups [3].

Several studies have shown the key role of CD+8 T cells in the control of HIV infections as well as of simian immunodeficiency virus (SIV) infections. As a result, various modalities of response-inducing vaccines regarding CD8+ T-cell effector response have been currently investigated and developed [5, 6, 7, 8].

Hence, it is also desirable that new approaches are developed in order to reduce the incidence of HIV infection or improve the consequences of the infection. As regards this aspect, vaccines are particularly relevant.

### SUMMARY OF THE INVENTION

The technical matter described herein relates to pharmaceutical compositions that may be used as immunogenic compounds or vaccines against lentivirus, and more specifically as immunogenic compounds or vaccines against HIV (Human Immunodeficiency Virus) or SIV (Simian Immunodeficiency Virus).

### BRIEF DESCRIPTION OF THE FIGURES

**Figure 1** shows viral growth curves in Vero cells.
**Figure 2** shows that YF17D replicates and induces **neutralizing** antibodies, virus-specific CD8+ T cells, and CD8+ T-cell activation in rhesus monkeys.
**Figure 3** shows the study of infection and immunogenicity of YF17D/SIVGag45-269 virus in rhesus monkeys.
**Figure 4** shows that vaccination with rYF17D/SIVGag45-269 yielded a robust expansion of Gag-specific responses in a monkey initially vaccinated with rBCG.
**Figure 5** shows that the result of the vaccination with rYF17D/SIVGag45-269 of r01056 [sic].

### DETAILED DESCRIPTION OF THE INVENTION

The described compositions typically include a recombinant polynucleotide or a panel of recombinant polynucleotides. Adequate HIV or SIV polynucleotides may encode at least one fragment of a HIV or SIV polypeptide including Gag, Pol, Rev, Tat, Nef, Vif, Vpx, Vpr, Env, Vpu,[sic](preferably at least one fragment of Gag, Vif, and Nef). The recombinant polynucleotide or panel of recombinant polynucleotides may be present in a recombinant virus or in a panel of recombinant viruses which express polypeptides encoded by the recombinant polynucleotides. The disclosed compositions may be used in methods for inducing immune response against HIV, which may include HIV-1 or HIV-2, or SIV (for example, response based on cytotoxic T cells (CTC), and, optionally, humoral or antibody-based response.[sic]

Disclosed compositions may include a mixture of recombinant polynucleotides, which may be present in a viral vector or in other nucleotide vector such as a plasmid or a bifunctional vector ("shuttle" vectors). Typically, the polynucleotides are present in expression vectors to express the polypeptides encoded by the polynucleotides. Adequate vectors may include but are not limited to viral vectors (for example, attenuated recombinant viruses). The pharmaceutical composition may include free recombinant nucleic acid or recombinant nucleic acid packaged into one or more virus particles (for example, replication-defective viral particles or attenuated virus). For instance, the recombinant polynucleotides contemplated herein may be present in one or more recombinant viruses (for example, recombinant yellow fever viruses) and the compositions may include a mixture of recombinant viruses.

The compositions may include a polynucleotide or a panel of polynucleotides (for example, at least about 2-9 polynucleotides) present in one or more expression vectors (for example, a viral vector or other expression vector,[sic] which encode the complete Gag polypeptide or a fragment/part of it. Optionally, the compositions may include a polynucleotide or a panel of polynucleotides present in one or more expression vectors which encode the complete Vif polypeptide or a fragment/part of it. Optionally, the compositions may include a polynucleotide or a panel of polynucleotides present in one or more expression vectors which encode the complete Nef polypeptide or a fragment/part of it. Optionally, the compositions may include a polynucleotide or a panel of polynucleotides present in one or more expression vectors which encode the complete Tat polypeptide or a fragment/part of it. Optionally, the compositions may include a polynucleotide or a panel of polynucleotides present in one or more expression vectors which encode Rev polypeptide or a fragment/part of it. Optionally, the compositions may include a polynucleotide or a panel of polynucleotides present in one or more expression vectors which encode the complete Pol polypeptide or a fragment/part of it. Optionally, the compositions may include a polynucleotide or a panel of polynucleotides present in one or more expression vectors which encode the complete Vpr polypeptide or a fragment/part of it. Optionally, the compositions may include a polynucleotide or a panel of polynucleotides present in one or more expression vectors which encode the complete Vpx polypeptide or a fragment/part of it. The polynucleotides or panels of polynucleotides may encode different polypeptides or different fragments of polypeptides. The encoded polypeptides or fragments of polypeptides may overlap (for example, in about 9-20 amino acids). The fragments are typically in the length range of 5-100 amino acids (or from 5-50, 10-50, 10-25, 5-15, or 10-15 amino acids) and include at least one epitope of the polypeptide from which the fragment is derived. In some embodiments, a fragment may include an N-terminal amino acid which is not normally present in the complete polypeptide from which the fragment is derived.

The pharmaceutical composition may comprise an effective amount or concentration of recombinant polynucleotides or recombinant viruses to induce a protective or therapeutic immune response against HIV infection in humans or SIV infection in simians. Inducing a protective or therapeutic immune response may include the potentiation of a CD8+ and/or CD4+ response to one or more HIV or SIV epitopes. Inducing a protective response may include the induction of sterilizing immunity against HIV or SIV. Inducing a therapeutic response may include the reduction of the viral load of an individual, for example, as determined by the measurement of the amount of circulating virus before and after delivering the composition. In some embodiments, the viral load is reduced to less than about 10,000 vRNA copies/mL (preferably to less than about 5,000 vRNA copies/mL, more preferably to less than about 2000 vRNA copies/mL, much more preferably to less than about 1500 vRNA copies/mL, and even more preferably to less than about 1000 vRNA copies/mL) at least about 3 months after the composition administration. The induction of a therapeutic response may include the increase in CD4+ T-cell count of the individual (for example, at least a twofold, threefold, or fourfold increase) at least 3 months after the composition administration.

Methods inducing protective or therapeutic immune responses against HIV or SIV infection through the administration of the pharmaceutical compositions described herein (for example, as immunogenic compounds or vaccines) to an individual who needs them, which may include a human who has already acquired or is at risk of acquiring a lentivirus infection, are also disclosed.

As used herein, a "nucleic acid vaccine" or "free DNA vaccine" relates to a vaccine which includes one or more expression vectors which encode B-cell and/or T-cell epitopes and provide an immunoprotective response to the individual who is being vaccinated. Nucleic acid vaccines as defined here, which may include expression vector plasmids, are not typically encapsidated into a viral particle. The vaccine nucleic acid is directly introduced into the cells of the individual who is subjected to the vaccinal regimen. This approach is described, for example, in U.S. Pat. Nos. 5.580.859; 5.589.466; 5.804.566; 5.739.118; 5.736.524; 5.679.647; and WO 98/04720. Examples of DNA-based administration technologies may include pure DNA ("naked DNA") administration, facilitated distribution (for example, through the use of bupivacaine, polymers, or peptides), and administration in the presence of cationic lipid complex or liposomes. Nucleic acids may be delivered using ballistic administration (for example, as described in U.S. Pat. No. 5.204.253), by pressure (for example, as described in U.S. Pat. No. 5.922.687), or by electroporation.

The nucleic acids used in the compositions and vaccines disclosed herein may encode one or more viral proteins or portions of one or more viral proteins. In some embodiments, the nucleic acids may encode an epitope (for example, a T-cell epitope) which is not part of a known HIV or SIV functional protein or which is encoded by a HIV or SIV alternative open reading frame (for example, a "cryptic" epitope). (See, for example, Maness et al., J. Exp. Med. (2007), 204:2505-12; and Cardinaud et al., J. Exp. Med. (2004), 199:1053-1063, which are all incorporated by reference herein in their entireties). A cryptic epitope may be located in an open reading frame (ORF) whose translation is not typically observed in the course of HIV or SIV viral replication. For example, Maness et al. discloses a cryptic epitope in SIVmac239 called "cRW9", which is located in the +2 reading frame relative to the ORF encoding the envelope protein. This cryptic epitope is located in the same ORF that encodes exon 1 of the Rev protein, but it is downstream of the only known splice donor site and so is not predicted to be translated under "normal" biological circumstances.

The pharmaceutical compositions disclosed herein may be formulated as vaccines for administration to an individual who needs them. These compositions may be formulated and/or administered at dosages and through techniques that are well known by physicians, taking into account factors such as age, gender, weight, particular conditions of the patient, and route of administration. The compositions may include pharmaceutical carriers, diluents, or excipients as they are known [in the art]. Moreover, the compositions may include preservatives (for example, antimicrobial or antibacterial agents such as benzalkonium chloride) or adjuvants.

The pharmaceutical compositions may be administered prophylactically or therapeutically. In prophylactic administration, vaccines may be administered in a sufficient amount to induce CD8+, CD4+, and/or antibody responses for protection against infection. In therapeutic applications, vaccines are administered to a patient in a sufficient amount to induce a therapeutic effect (for example, CD8+, CD4+, and/or antibody responses for HIV or SIV antigens or epitopes encoded by the polynucleotides of the composition [sic], which heals or at least partially suspends or delays the symptoms and/or complications of HIV or SIV infection (i.e., a "therapeutically effective dose").

The compositions disclosed herein may include recombinant polynucleotides or recombinant viruses which encode and express complete HIV or SIV polypeptides or their fragments. As used herein, a "minigene" encodes only part of a gene. For example, a minigene for HIV Gag may encode only 5-100 amino acids of the Gag polypeptide (or 5-50, 10-50, 5-25, 10-25, 5-15, or 10-15 amino acids). Minigenes which encode only part of a gene are described in the U.S. Patent Application No. 12/022.530, filed on January 30, 2008, which claims the benefit of U.S. Provisional Application No. 60/898.644, filed on January 31, 2007, the contents of which are incorporated by reference herein in their entireties. For example, a minigene for HIV Gag may encode a Gag fragment which includes only 5-100 amino acids of the Gag polypeptide (or 5-50, 10-50, 5-25, 10-25, 5-15, or 10-15 amino acids). In some embodiments, the recombinant polynucleotide or recombinant viruses may encode and express a SIV polypeptide fragment as disclosed in Table 1 or a corresponding HIV polypeptide fragment.

**Table 1. Epitope-specific sequences of amino acids.**

| Protein | Name | Sequence | MHC class I restriction |
|---|---|---|---|
| Tat | SL8 | STPESANL | A*01 |
| Nef | IW9 | IRYPKTFGW | B*17 |
| | MW9 | MHPAQTSQW | B*17 |
| | YY9 | YTSGPGIRY | A*02 |
| | YY9* | YTYEAYVRY | A*02 |
| | AL11 | ARRHRILDIYL | B*08 |
| | ML10 | MRRSRPSGDL | B*08 |
| | RL10 | RRRLTARGLL | B*08 |
| Vif | HW8 | HLEVQGYW | B*17 |
| | WY8 | WTDVTPNY | A*02 |
| | RL8 | RRDNRRGL | B*08 |
| | RL9 | RRAIRGEQL | B*08 |
| Ver | KL9 | KRLRLIHLL | B*08 |
| | RL10 | RRRWQQLLAL | B*08 |
| Pol p10 | LV10 | LGPHYTPKIV | A*01 |
| protease | YL8 | YHSNVKEL | A*07 |
| Env gp120 | CL9 | CAPPGYALL | A*01 |
| Env gp41 | TL9 | TVPWPNASL | A*01 |
| Env gp41 | RY8 | RTLLSRVY | A*02 |
| Env gp41 | FW9 | FHEAVQAVW | B*17 |
| Gag p17 | GY9 | GSENLKSLY | A*02 |
| matrix | CM9 | CTPYDINQM | A*01 |
| Gag p27 | QI9 | QNPIPVGNI | A*01 |
| capsid | LF8 | LAPVPIPF | A*01 |
| Gag p27 | RW9 | RAPRRQGCW | B*17 |
| capsid | | | |
| Vpx | II11 | IPPGNSGEETI | A*01 |

In some embodiments, the recombinant polynucleotide or recombinant viruses may comprise a "minigenes" of SIV or HIV and express a SIV polypeptide fragment or a corresponding HIV polypeptide fragment as disclosed in Tables 2-6.

**Table 2. SIV and HIV Gag Protein Fragments**

| **Protein** | **Sequence** |
|---|---|
| 1 (a) SIV Gag p17 | SVLSGKKADELEKIRLRPNGKKKYMLKHVVWAANELDRFGLAESLLE |
| 1 (b) HIV Gag p17 | SVLSGGKLDKWEKIRLRPGGKKTYQLKHIVWASRELERFAVNPGLLE |
| 2 (a) SIV Gag p17 | FGLAESLLENKEGCQKILSVLAPLVPTGSENLKSLYNTVDV |
| 2 (b) HIV Gag p17 | FAVNPGLLETGGGCKQILVQLQPSLQTGSEELKSLYNAVAT |
| 3 (a) SIV Gag p17 | NKEGCQKILSVLAPLVPTGSENLKSLYNTVCVIWCIHAEEKVKHTEEA KQIVQRHLVVETGTTETMPKTSR |
| 3 (b) HIV Gag p17 | TGGGCKQILVQLQPSLQTGSEELKSLYNAVATLYCVHQGIEVRDTKEA LDKIEEEQNKSKKKAQQAAA |
| 4 (a) SIV Gag p27 | GNYVHLPLSPRTLNAWVKLIEEKKFGAEVVPGFQALSEGCTPYDI |
| 4 (b) HIV Gag p24 | GQMVHQAISPRTLNAWVKVIEEKAFSPEVIPMFSALSEGATPQDL |
| 5 (a) SIV Gag p27 | SEGCTPYDINQMLNCVGDHQAAMQIIRDIINEEAADWDLQHPQPAPQQ GQLREPSGSDIAGTTSSVDEQIQWMYRQQNPIP |
| 5 (b) HIV Gag p24 | SEGATPQDLNTMLNTVGGHQAAMQMLKETINEEAAEWDRLHPAHAGPN APGQMREPRGSDIAGTTSTLQEQIGWMTSNPPVP |
| 6 (a) | SEGCTPYDINQMLNCVGDHQAAMQIIRDIINEEAADWDLQHPQPAPQQ |
| SIV Gag p27 | GQ |
| 6 (b) HIV Gag p24 | SEGATPQDLNTMLNTVGGHQAAMQMLKETINEEAAEWDRLHPAHAGPN APGQ |
| 7 (a) SIV Gag p27 | MYRQQNPIPVGNIYRRWIQLGLQKCVRMYNPTNIPDVKQGPKEPFQSY VDRFYKSLRAEQTDAAVKNWMTQTLLIQNANPDCKLVLKGLGVNPTLE EMLTACQGVGGPGQKARLMAEALKEALAPVPIPFAAAQQRGPRKPIKC WNCGKEGHSARQCRAPRRQGCW |
| 7 (b) HIV Gag p24 | MTSNPPVPVGEIYKRWIILGLNKIVRMYSPVSILDIRQGPKEPFRDYV DRFYKTLRAEQASQDVKNWMTETLLVQNANPDCKTILKALGPAATLEE MMTACQGVGGPSHKARILAEAMSQVTSPANIMMQRGNFRNQRKTIKCF NCGKEGHLARHCRAPRKKGCW |
| 8 (a) SIV Gag p27 | TTSSVDEQIQWMYRQQNPIPVGNIYRRWIQLGLQKCVRMYNPTNIPDV KQGPKEPFQSYVDRFYKSLRAEQTDAAVKNWMTQTLLIQNANPDCKLV LKGLGVNPTLEEMLTACQGVGGPG |
| 8 (b) HIV Gag p24 | TTSTLQEQIGWMTSNPPVPVGEIYKRWIILGLNKIVRMYSPVSILDIR QGPKEPFRDYVDRFYKTLRAEQASQDVKNWMTETLLVQNANPDCKTIL KALGPAATLEEMMTACQGVGGPS |

**Table 3. SIV and HIV Vif Protein Fragments**

| | |
|---|---|
| 10 (a) SIV Vif | MEEEKRWIAVPTWRIPERLERWHSLIKYLKYKTKDLQKVCYVPHFKVGWA WWTCSRVIFPLQEGSHLEVQGYWHLTPEKGWLSTYAVRITWYSKNFWTDV TPNYADILLH |
| 10 (b) HIV Vif | MENRWQVMIVWQVDRMRIKTWKSLVKHHMYISKKAKEWVYRHHYESTHPR ISSEVHIPLGDAKLVITTYWGLHTGEREWHLGQGVSIEWRKKRYNTQVDP DLADKLIH |
| 11 (a) SIV Vif | PNYADILLHSTYFPCFTAGEVRRAIRGEQLLSCCRFPRAHKYQVPSLQYL ALKVVSDVRSQGENPTWKQWRRDNRRGLRMAKQNSRGDKQRGGKPPTKGA NFPGLAKVLGILA |
| 11 (b) HIV Vif | PDLADKLIHLHYFDCFSDSAIRHAILGHRVRPKCEYQAGHNKVGSLQYLA LTALITPKKIKPPLPSVRKLTEDRWNKPQKTKGHRGSHTMNGH |
| 12 (a) SIV Vif | MEEEKRWIAVPTWRIPERLERWHSLIKYLKYKTKDLQKVCYVPHFKVGWA WWTCSRVIFP |
| 12 (b) HIV Vif | MENRWQVMIVWQVDRMRIKTWKSLVKHHMYISKKAKEWVYRHHYESTHPR ISSEVHIP |
| 13 (a) SIV Vif | WWTCSRVIFPLQEGSHLEVQGYWHLTPEKGWLSTYAVRITWYSKNFWTDV TPNYADILLH |
| 13 (b) HIV Vif | ESTHPRISSEVHIPLGDAKLVITTYWGLHTGEREWHLGQGVSIEWRKKRY NTQVDPDLADKLIH |
| 14 (a) SIV Vif | PNYADILLHSTYFPCFTAGEVRRAIRGEQLLSCCRFPRAHKYQVPSLQYL ALKVVSDVR |
| 14 (b) | PDLADKLIHLHYFDCFSDSAIRHAILGHRVRPKCEYQAGHNKVGSLQYLA |
| HIV Vif | LTALITPKK |
| 15 (a) SIV Vif | LALKVVSDVRSQGENPTWKQWRRDNRRGLRMAKQNSRGDKQRGGKPPTKG ANFPGLAKVLGILA |
| 15 (b) HIV Vif | LALTALITPKKIKPPLPSVRKLTEDRWNKPQKTKGHRGSHTMNGH |

**Table 4. SIV and HIV Nef Protein Fragments**

| | |
|---|---|
| 16 (a) SIV Nef | GLDKGLSSLSCEGQKYNQGQYMNTPWRNPAEEREKLAYRKQNMDDIDEED DDLVGVSVRPKVPLRTMSYKLAIDMSHFIKEKGGLEGIYYSARRHRILDI YLEKEEGIIPDWQDYTSGPGIRYPKTFGWLWKLVPVNVSDEAQEDEEHYL MHPAQTSQWDDPWGEV |
| 16 (b) HIV Nef | MGGKWSKSSKAGWPQVREKIKQTPPAAEGVGAVSQDLDKHGAITSSNMNN ADCVWLQAQEDEEVGFPVRPQVPLRPMTFKGAFDLSFFLKEKGGLDGLIY SRKRQEILDLWVYNTQGFFPDWQNYTPGPGVRLPLCFGWCFKLVPVDPRE VEEDNKGENNCLLHPLSQHGMEDEHKEV |
| 17 (a) SIV Nef | GLDKGLSSLSCEGQKYNQGQYMNTPWRNPAEEREKLAYRKQNMDDIDEED DD |
| 17 (b) HIV Nef | MGGKWSKSSKAGWPQVREKIKQTPPAAEGVGAVSQDLDKHGAITSSNMNN ADCVWLQAQEDEE |
| 18 (a) SIV Nef | DDIDEEDDDLVGVSVRPKVPLRTMSYKLAIDMSHFIKEKGGLEGIYYSAR RH |
| 18 (b) HIV Nef | DCVWLQAQEDEEVGFPVRPQVPLRPMTFKGAFDLSFFLKEKGGLDGLIYS RKR |
| 19 (a) SIV Nef | GIYYSARRHRILDIYLEKEEGIIPDWQDYTSGPGIRYPKTFGWLWKLVPV |
| 19 (b) HIV Nef | GLIYSRKRQEILDLWVYNTQGFFPDWQNYTPGPGVRLPLCFGWCFKLVPV |
| 20(a) SIV Nef | GWLWKLVPVNVSDEAQEDEEHYLMHPAQTSQWDDPWGEV |
| 20 (b) HIV Nef | GWCFKLVPVDPREVEEDNKGENNCLLHPLSQHGMEDEHKEV |

**Table 5. SIV and HIV Tat Protein Fragments**

| | |
|---|---|
| 21(a) SIV Tat | METPLREQENSLESSNERSSCISEADASTPESANLGEEILSQLYRPLEA |
| 21 (b) HIV Tat | MEPVDPRLEPWKHPGSQPKTPCTKCYCKKCCLHCQVCFMTKGLGISYGRK |
| 22 (a) SIV Tat | SQLYRPLEACYNTCYCKKCCYHCQFCFLKKGLGICYEQSRKRRRTPKKAK |
| 22 (b) HIV Tat | SQPKTPCTKCYCKKCCLHCQVCFMTKGLGISYGRKKRRQRRRAPQDNKNH |

**Table 6. SIV and HIV Rev Protein Fragments**

| | |
|---|---|
| 23 (a) SIV Rev | MSNHEREEELRKRLRLIHLLHQTNPYPTGPGTANQRRQRKRRWRRRWQQ |
| 23 (b) HIV Rev | MAGRSGSTDEELLRAVRIIKILYQSNPYPSSEGTRQARRNRRRRWRARQR |
| 24 (a) SIV Rev | RRWRRRWQQLLALADRIYSFPDPPTDTPLDLAIQQLQNLAIESIPDPPTN TPEALCDPTEDSRSPQD |
| 24 (b) HIV Rev | RRNRRRRWRARQREICALSERILSSCLGRPTEPVPLPLPPLERLTLDCSE DCGTSGTQQSQGTETGVGRPQISGE |

A minigene of SIV or a minigene of HIV may encode a polypeptide comprising a SIV or HIV polypeptide fragment and also comprise an N-terminal methionine. A minigene of SIV or a minigene of HIV may include codon sequences which are optimized for expression (for example, in animal, organism or recombinant virus as contemplated herein). Exemplary SIV minigenes including codon sequences which are optimized for expression by the yellow fever virus are depicted in Table 7.

**Table 7. Optimized-Sequence Minigenes**

| Name | 1. SIVmac239Gag(6-52) |
|---|---|
| DNA Seq. | 5′- GTCGACAGGAGCCACCATGAGCGTGCTGAGCGGCAAGAAGGCCGACGAGC TGGAGAAGATCCGGCTGCGGCCCAACGGCAAGAAGAAATACATGCTGAAG CACGTGGTGTGGGCCGCCAACGAGCTGGACCGGTTCGGCCTGGCCGAGAG CCTGCTGGAGTGAGAATTCGCGGCCGC-3′ |
| AA Seq. | MSVLSGKKADELEKIRLRPNGKKKYMLKHVVWAANELDRFGLAESLLE |
| Name | 2. SIVmac239Gag(44-84) |
| DNA Seq. | 5′- GTCGACAGGAGCCACCATGTTCGGCCTGGCCGAGAGCCTGCTGGAGAACA AGGAGGGCTGCCAGAAGATCCTGAGCGTGCTGGCCCCTCTGGTGCCCACC GGCAGCGAGAACCTGAAGAGCCTGTACAACACCGTGTGCGTGTGAGAATT CGCGGCCGC-3′ |
| AA Seq. | MFGLAESLLENKEGCQKILSVLAPLVPTGSENLKSLYNTVCV |
| Name | 3. SIVmac239Gag(76-123) |
| DNA Seq. | 5′- GTCGACAGGAGCCACCATGAAGAGCCTGTACAACACCGTGTGCGTGATCT GGTGCATCCACGCCGAGGAGAAGGTGAAGCACACCGAGGAGGCCAAGCAG ATCGTGCAGCGGCACCTGGTGGTGGAGACCGGCACCACCGAGACCATGCC CAAGACCAGCCGCTGAGAATTCGCGGCCGC-3′ |
| AA Seq. | MKSLYNTVCVIWCIHAEEKVKHTEEAKQIVQRHLVVETGTTETMPKTSR |
| Name | 4. SIVmac239Gag(142-186) |
| DNA Seq. | 5′- GTCGACAGGAGCCACCATGGGCAACTACGTGCACCTGCCCCTGAGCCCCC GGACCCTGAACGCCTGGGTGAAGCTGATCGAGGAGAAGAAGTTCGGCGCC GAGGTGGTGCCCGGCTTCCAAGCCCTGAGCGAGGGCTGCACCCCCTACGA CATCTGAGAATTCGCGGCCGC-3′ |
| AA Seq. | MGNYVHLPLSPRTLNAWVKLIEEKKFGAEVVPGFQALSEGCTPYDI |

| Name | 5. SIVmac239Gag(178-258) |
|---|---|
| DNA Seq. | 5′- GTCGACAGGAGCCACCATGAGCGAGGGCTGCACCCCCTACGACATCAACC AGATGCTGAACTGCGTGGGCGACCACCAGGCCGCCATGCAGATCATCCGG GACATCATCAACGAGGAGGCCGCCGACTGGGACCTGCAGCACCCCCAGCC CGCCCCCCAGCAAGGCCAGCTGCGGGAGCCCAGCGGCAGCGACATCGCCG GCACCACCAGCAGCGTGGACGAGCAGATCCAGTGGATGTACCGGCAGCAG AACCCCATCCCCTGAGAATTCGCGGCCGC-3′ |
| AA Seq. | MSEGCTPYDINQMLNCVGDHQAAMQIIRDIINEEAADWDLQHPQPAPQQG QLREPSGSDIAGTTSSVDEQIQWMYRQQNPIP |

| Name | 6. SIVmac239Gag(250-415) |
|---|---|
| DNA Seq. | 5′- GTCGACAGGAGCCACCATGTACCGGCAGCAGAACCCCATCCCCGTGGGCA ACATCTACCGGCGGTGGATCCAGCTGGGCCTGCAGAAATGCGTGCGGATG TACAACCCCACCAACATCCTGGACGTGAAGCAGGGCCCCAAGGAGCCCTT CCAGAGCTACGTGGACCGGTTCTACAAGAGCCTGCGGGCCGAGCAGACCG ACGCCGCCGTGAAGAACTGGATGACCCAGACCCTGCTGATCCAGAACGCC AACCCCGACTGCAAGCTGGTGCTGAAGGGCCTGGGCGTGAACCCCACCCT GGAGGAGATGCTGACCGCCTGCCAGGGCGTGGGCGGCCCCGGCCAGAAGG CCCGGCTGATGGCCGAGGCCCTGAAGGAGGCCCTGGCCCCCGTGCCCATC CCCTTCGCCGCCGCCCAGCAGCGGGGCCCCCGGAAGCCCATCAAGTGCTG GAACTGCGGCAAGGAGGGCCACAGCGCCCGGCAGTGCCGGGCCCCCCGGC GGCAGGGCTGCTGGTGAGAGAATTCGCGGCCGC-3′ |
| AA Seq. | MYRQQNPIPVGNIYRRWIQLGLQKCVRMYNPTNILDVKQGPKEPFQSYVD RFYKSLRAEQTDAAVKNWMTQTLLIQNANPDCKLVLKGLGVNPTLEEMLT ACQGVGGPGQKARLMAEALKEALAPVPIPFAAAQQRGPRKPIKCWNCGKE GHSARQCRAPRRQGCW |
| Name | 7. SIVmac239Vif(1-110) |
| DNA Seq. | 5′- GTCGACAGGAGCCACCATGGAGGAGGAGAAGCGGTGGATCGCCGTGCCCA CCTGGCGGATCCCCGAGCGGCTGGAGCGGTGGCACAGCCTGATCAAGTAC CTGAAGTACAAGACCAAGGACCTGCAGAAGGTGTGCTACGTGCCCCACTT CAAAGTGGGCTGGGCCTGGTGGACCTGCAGCCGGGTGATCTTCCCCCTGC AAGAGGGCAGCCACCTGGAGGTGCAGGGCTACTGGCACCTGACCCCCGAG AAGGGCTGGCTGAGCACCTACGCCGTGCGGATCACCTGGTACAGCAAGAA CTTCTGGACCGACGTGACCCCCAACTACGCCGACATCCTGCTGCACTGAG AATTCGCGGCCGC-3′ |
| AA Seq. | MEEEKRWIAVPTWRIPERLERWHSLIKYLKYKTKDLQKVCYVPHFKVGWA WWTCSRVIFPLQEGSHLEVQGYWHLTPEKGWLSTYAVRITWYSKNFWTDV TPNYADILLH |
| Name | 8. SIVmac239Vif(102-214) |
| DNA Seq. | 5′- GTCGACAGGAGCCACCATGCCCAACTACGCCGACATCCTGCTGCACAGCA CCTACTTCCCCTGCTTCACCGCCGGCGAGGTCCGGCGGCCATCCGGGGC GAGCAGCTGCTGAGCTGCTGCCGGTTCCCCCGGGCCCACAAGTACCAGGT GCCCAGCCTGCAGTACCTGGCCCTGAAGGTGGTGAGCGACGTGCGGAGCC AGGGCGAGAACCCCACCTGGAAGCAGTGGCGGCGGGACAACCGGCGGGGC CTGCGGATGGCCAAGCAGAACAGCCGGGGCGACAAGCAGCGGGGCGGCAA GCCTCCCACCAAGGGCGCCAACTTCCCCGGCCTGGCCAAGGTGCTGGGCA TCCTGGCCTGAGAATTCGCGGCCGC-3′ |
| AA Seq. | MPNYADILLHSTYFPCFTAGEVRRAIRGEQLLSCCRFPRAHKYQVPSLQY LALKVVSDVRSQGENPTWKQWRRDNRRGLRMAKQNSRGDKQRGGKPPTKG ANFPGLAKVLGILA |

| Name | 9. SIVmac239Nef(45-210) |
|---|---|
| DNA Seq. | 5′- GTCGACAGGAGCCACCATGGGCCTGGACAAGGGCCTGAGCAGCCTGAGCT GCGAGGGCCAGAAGTACAACCAGGGCCAGTACATGAACACCCCCTGGCGG AACCCCGCCGAGGAGCGGGAGAAGCTGGCCTACCGGAAGCAGAACATGGA CGACATCGACGAGGAGGACGACGACCTGGTGGGCGTGAGCGTGCGGCCCA AGGTGCCCCTGCGGACCATGAGCTACAAGCTGGCCATCGACATGAGCCAC TTCATCAAGGAGAAGGGCGGCCTGGAGGGCATCTACTACAGCGCCCGGCG GCACCGGATCCTGGACATCTACCTGGAGAAGGAGGAGGGCATCATCCCCG ACTGGCAGGACTACACCAGCGGCCCCGGCATCCGGTACCCCAAGACCTTC GGCTGGCTGTGGAAGCTGGTGCCCGTGAACGTGAGCGACGAGGCCCAGGA GGACGAGGAGCACTACCTGATGCACCCCGCCCAGACCAGCCAGTGGGACG ACCCCTGGGGCGAGGTGTGAGAATTCGCGGCCGC-3′ |
| AA Seq. | MGLDKGLSSLSCEGQKYNQGQYMNTPWRNPAEEREKLAYRKQNMDDIDEE DDDLVGVSVRPKVPLRTMSYKLAIDMSHFIKEKGGLEGIYYSARRHRILD IYLEKEEGIIPDWQDYTSGPGIRYPKTFGWLWKLVPVNVSDEAQEDEEHY LMHPAQTSQWDDPWGEV |

The viral vectors, recombinant bacteria, and nucleic acids used in the compounds and vaccines disclosed herein may encode one or more proteins of HIV or SIV or portions of one or more proteins of HIV or SIV. In some embodiments, nucleic acids may encode an epitope (for example, a T-cell epitope) which is not part of a known HIV or SIV functional protein or which is encoded by a HIV or SIV alternative open reading frame (i.e., a "cryptic" epitope). (See, for example, Maness et al., J. Exp. Med. (2007), 204:2505-12; and Cardinau et al., J. Exp. Med. (2004), 199:1053-1063, which are all incorporated by reference herein in their entireties). A cryptic epitope may be located in an open reading frame (ORF) whose translation is not typically observed in the course of HIV or SIV viral replication. For example, Maness et al. discloses a cryptic epitope in SIVmac239 called "cRW9", which is located in the +2 reading frame relative to the ORF encoding the envelope protein. This cryptic epitope is located in the same ORF that encodes exon 1 of the Rev protein, but it is at the 3' side ("downstream") of the donor processing site ("donor splicing site") and so its translation would not be expected under "normal" biological conditions.

Any of the conventional vectors used for expression in eukaryotic cells may be used to directly immunize an individual with nucleic acid. Expression vectors containing regulatory elements of eukaryotic viruses may be used in eukaryotic expression vectors (for example, vectors containing SV40, CMV, or retroviral enhancers or promoters). Exemplary vectors include vectors expressing proteins under the direction of such promoters as SV40 early promoter, SV40 late promoter, metallothionein promoter, human cytomegalovirus promoter, murine mammary tumor virus promoter, and Rous sarcoma virus promoter. Industrial amounts for therapeutic and prophylactic use of plasmid DNA may be yielded, for example, by fermentation in *E. coli,* followed by purification. Aliquots from the working cell bank are used to inoculate a culture medium, and cultured until saturation in shake flasks or in a bioreactor according to widely known techniques. Plasmid DNA may be purified by standard bioseparation technologies such as solid phase ion exchange resins. If required, the DNA may be isolated from the open-circular and linear forms using gel electrophoresis or other methods.

The purified plasmid DNA may be prepared for injection using a wide range of formulations, such as, for example, lyophilized DNA which may be reconstituted in phosphate-buffered sterile saline (PBS) solution. The purified DNA may be introduced into an individual through any appropriate method; for example, by intramuscular (IM) or intradermal (ID) administration.

In order to maximize the immunotherapy effects of DNA vaccines, alternative methods may be desirable to formulate purified plasmid DNA. A variety of methods has been described, and new techniques may become available. For example, cationic lipids may be used in the formulation of the pharmaceutical composition disclosed herein (for example, as described in WO 93/24640; U.S. Pat. No. 5.279.833; and WO 91/06309). Protective, interactive, noncondensing compounds (PINC) may be used in the formulation of the pharmaceutical composition disclosed herein (for example, glycolipids, fusogenic liposomes, and peptides).

The term "vector" refers to a few means by which nucleic acid may be introduced into a host organism or tissue. There are several types of vectors, including viruses, plasmids, bacteriophages, cosmids, and bacteria. As used herein, a "recombinant virus" or "viral vector" relates to the recombinant viral nucleic acid which has been genetically modified to express a heterologous polypeptide (for example, a HIV polypeptide or a fragment thereof). The recombinant viral nucleic acid typically includes cis-acting elements for the expression of the heterologous polypeptide. Typically, the recombinant viral nucleic acid can be packaged into a virus which is able to infect a host cell. For example, the recombinant viral nucleic acid may include cis-acting elements for packaging. Generally, the viral vector is not able to replicate or is attenuated. An "attenuated recombinant virus" refers to a virus which has been genetically modified by modern molecular biological methods, making it less virulent than the wild type, usually by mutation or deletion of specific genes. For example, the recombinant viral nucleic acid may not have an essential gene for an efficient production or for the production of the infectious virus.

The recombinant viral nucleic acid may function as a vector for an immunogenic retroviral protein in view of the fact that the recombinant viral nucleic acid comprises exogenous nucleic acid. The recombinant virus may be introduced in a vaccinated human by standard methods for vaccination using live vaccines. A live vaccine as contemplated herein may be administered using, for example, from about 10⁴ to 10⁸ units of viral particles/dose, or 10⁶ to 10⁹ pfu/dose. The actual dosages of this vaccine may be readily determined by any expert in the vaccine field.

Numerous virus species may be used as recombinant virus vectors for the pharmaceutical composition disclosed herein. Preferred recombinant virus for a viral vaccine is the yellow fever vaccine virus (for example, 17-D or similar).

In some embodiments, attenuated recombinant bacteria or mycobacteria may be used as vectors in the pharmaceutical compositions and vaccines disclosed herein (for example, Bacillus, Shigella, Salmonella, Listeria or Yersinia, attenuated recombinant bacteria). Recombinant bacterial vaccine vectors are described by Daudel et al., "Use of attenuated bacteria as delivery vectors for DNA vaccines", Expert Review of Vaccines, Volume 6, Number 1, February, 2007, pp. 97-110(14); Shata et al., "Recent advances with recombinant bacterial vaccine vectors", Molec. Med. Today (2000), Volume 6, Number 2, February 1, 2000, pages 66-71; Clare & Dougan, "Live Recombinant Bacterial Vaccines", Novel Vaccination Strategies, April 16, 2004 (Editor Stefan H. E. Kaufman); Gentaschev et al., "Recombinant Attenuated Bacteria for the Delivery of Subunit Vaccines", Vaccine, Volume 19, Numbers 17-19, March 21, 2001, pages 2621-2628; Garmory et al., "The Use of live attenuated bacteria as a delivery system for heterologous antigens", J. Drug Target. 2003; 11 (8-10): 471-9; U.S. Patent No. 6.383.496; and U.S. Patent No. 6.923.958 (which are all incorporated by reference herein in their entireties).

In some embodiments, the recombinant bacterium or mycobacterium is the recombinant Bacillus Calmette-Guérin (or Bacillus Calmette-Guérin, BCG). The BCG is an attenuated strain of the live bovine tuberculosis bacillus, *Mycobacterium bovis,* which lost its virulence in humans by being specially cultured in an artificial medium over years. The use of a recombinant BCG (rBCG) expressing the codon-optimized HIV-1 Gag for mice immunization in a "prime-boost" regimen has been described. Promkhatkaaew D. et al., "Prime-boost immunization of codon optimized HIV-1 CRF01_AE Gag in BCG with recombinant vaccinia virus elicits MHC class I and II immune responses in mice", Immunol Invest. 2009; 38(8):762-79.

The compositions included in the vaccine regimen as contemplated herein may be coadministered or administered sequentially with another immunological antigenic vaccine or in therapeutic compositions, including an adjuvant, a biological or chemical agent given in combination with or genetically fused to an antigen to increase the antigen immunogenicity. Additional therapeutic agents may include but are not limited to CD40 or interleukin-2 (IL-2) ligand in a sufficient amount to further potentiate CD8+ T cell, CD4+ T cell, and antibody responses. These other compositions may also include purified antigens of the immunodeficiency virus or the expression of such antigens by a second recombinant vector system which can give rise to additional therapeutic compositions.

The pharmaceutical composition disclosed herein may be delivered by various routes. The typical delivery routes include parenteral administration (for example, intradermal, intramuscular, or subcutaneous delivery). Other routes include oral, intranasal, intravaginal, and intrarectal administration. The pharmaceutical composition may be formulated for intranasal or pulmonary delivery. The pharmaceutical composition formulations may include liquid formulations (for example, for oral, nasal, anal, or vaginal administration, among others, including suspensions, syrups, or elixirs) and preparations for parenteral, subcutaneous, intradermal, intramuscular, or intravenous administration (for example, injectable administration) such as sterile suspensions or emulsions.

As used herein, the term "adjuvant" refers to a compound or mixture which increases the immune response to an antigen. An adjuvant may serve as a tissue depot that slowly releases the antigen and also as a lymphoid system activator which nonspecifically amplifies the immune response. Examples of adjuvants which may be employed include the adjuvant MPL-TDM (Monophosphoryl lipid A/synthetic trehalose dicorynomycolate; for example, available from GSK Biologicals). Another appropriate adjuvant is the AS021/AS02 immunostimulatory adjuvant (GSK). These immunostimulatory adjuvants are formulated to promote an intense T-cell response and include QS-21, a saponin from Quillay saponaria, the TL4 ligand, a monophosphoryl lipid A, together in a lipid or liposomal carrier. Other adjuvants include but are not limited to nonionic block copolymer adjuvants (for example, CRL1005), aluminum phosphates (for example, A1PO4), R-848 (a Th1-like adjuvant), imiquimod, PAM3CYS, poly (I:C), loxoribine, potentially useful human adjuvants such as BCG (Bacillus Calmette-Guérin) and *Corynebacterium parvum,* C p G oligodeoxynucleotides (ODN), antigens derived from cholera toxin (for example, CTA1-DD), lipopolysaccharide adjuvants, complete Freund's adjuvant, incomplete Freund's adjuvant, saponin, mineral gels such as aluminum hydroxide, surface active substances such as lysolecithin, pluronic polyols, polyanions, peptides, oil or hydrocarbon emulsions in water (for example, MF59 available from Novartis Vaccines or Montanide ISA 720), keyhole-limpet hemocyanins, and dinitrophenol.

The pharmaceutical compositions disclosed herein may also or additionally comprise at least one antiviral chemotherapy compound. Nonlimiting examples may be selected from at least one compound of the group consisting of gamma globulin, amantadine, guanidine, benzimidazole hydroxide, interferons, interleukin-16, thiosemicarbazones, methisazones, rifampicin, ribavirin, pyrimidine analogs (for example, AZT and/or 3TC), purine analogs, foscarnet, phosphonoacetic acid, acyclovir, dideoxynucleosides, protease inhibitors (for example, saquinavir; indinavir; ritonavir; AG 1343; and VX-2/78), chemokines, such as RANTES, MIP1x or MIP1b, or ganciclovir.

As used herein, a "prime-boost vaccination regimen" refers to a regimen in which a first composition is administered to an individual one or more times (for example, two or three times with about 2, 3, or 4 weeks between administrations), and, after a certain period of time (for example, about two weeks, about four weeks, about two months, about three months, about four months, about five months, about six months, or more), a second composition is administered to the individual, which may be equal to or different from the first composition. The second composition may also be administered more than once, with at least 2, 3, or 4 weeks between administrations. In some embodiments, the individual is given the first composition, which comprises the recombinant yellow fever virus 17D expressing one or more HIV or SIV polypeptides, or their fragments, or rBCG expressing one or more HIV or SIV polypeptides, or their fragments. Subsequently, the individual receives the second composition, which comprises the recombinant yellow fever virus 17D expressing one or more HIV or SIV polypeptides, or their fragments, DNA encoding one or more HIV or SIV polypeptides, or their fragments, or rBCG expressing one or more HIV or SIV polypeptides or their fragments. The first and the second compositions may be the same or different.

The pharmaceutical compositions disclosed herein may be delivered to individuals at risk of developing HIV or SIV infection or to individuals who are already infected with HIV or SIV. To evaluate the efficacy of the vaccine, the immune response may be assessed by measuring the induction of CD8+ and antibody responses for particular epitopes. CD8+ T-cell responses may be measured, for example, by using cultured or fresh PBMC tetramer staining, ELISPOT assays, or functional cytotoxicity assays, which are well known among experts, and are described herein. Antibody responses may be measured by widely known assays such as ELISA. Determinations of viral titers or loads and CD4+ T cells after immunization may be performed in individuals who have already been infected. For example, individuals may present a decline in the viral load and an increase in the CD4+ cell count after immunization (for example, at least a twofold, threefold, or fourfold increase in the CD4+ cell count in relation to the count obtained prior to immunization).

The invention will now be described in detail regarding certain embodiments of the invention.

In some embodiments of the recombinant viruses contemplated herein, the virus is a yellow fever vaccinal virus engineered to express a heterologous polypeptide. The heterologous polypeptide may include a HIV polypeptide (HIV-1 or HIV-2), a SIV polypeptide, or a fragment of them (for example, a fragment consisting of at least 8, 9, 10, 20, 30, 40, 50, 100, or 200 contiguous amino acids of a HIV Gag polypeptide or a SIV Gag polypeptide). In other embodiments, the heterologous polypeptide is a polypeptide with at least 90%, 95%, 96%, 97%, 98%, or 99% of sequence identity with a HIV polypeptide, a SIV polypeptide, or a fragment of them (for example, a fragment consisting of at least 8, 9, 10, 20, 30, 40, 50, 100, or 200 contiguous amino acids of a HIV Gag polypeptide or a SIV Gag polypeptide). Sequences for HIV-1 strains and several genes of HIV-1 strains (including the gene for Gag polypeptide) are publicly available in GenBank; for example, the sequences available under accession numbers: NC_ 001802; EU293448, EU293447, EU293446, EU293445, EU293444, FJ195091, FJ195090, FJ195089, FJ195088, FJ195087, FJ195086, EU884501, EU786681, EU786680, EU786679, EU786678, EU786677, EU786676, EU786675, EU786674, EU786673, EU786672, EU786671, EU786670, EU697909, EU697908, EU697907, EU697906, EU697905, EU697904, U71182, EU69324, EU861977, FJ213783, FJ213782, FJ213781, FJ213780, AB428562, AB428561, AB428560, AB428559, AB428558, AB428557, AB428556, AB428555, AB428554, AB428553, AB428552, AB428551, DQ295195, DQ295196, DQ295194, DQ295193, DQ295192, EU446022, EU735540, EU735539, EU735538, EU735537, EU735536, EU735535, EU110097, EU110096, EU110095, EU110094, EU110093, EU110092, EU110091, EU110090, EU110089, EU110088, EU110087, EU110086, EU110085, AF193277, AF193276, AF049337, EU541617, EU220698, EF469243, DQ912823, DQ912822, EU000516, EU000515, EU000514, EU000513, EU000512, EU000511, EU000510, EU000509, EU000508, EU000507, and EU884500; whose submissions to GenBank are incorporated by reference herein in their entireties. Primary and field isolates of HIV are available from the National Institute of Allergy and Infectious Diseases (NIAID), which has contracted the services of Quality Biological (Gaithersburg, MD.) to make these strains available. Strains are also available from the World Health Organization (WHO), Geneva, Switzerland. In a preferred embodiment, the selected polypeptide has a consensus sequence as determined by the comparison of three or more HIV strains. As disclosed herein, the polynucleotides encoding a HIV polypeptide or a SIV polypeptide may be "optimized" for expression in a human cellular environment or simian cellular environment, respectively. Thus, the use of polynucleotides specifically including HIV genes that are codon-optimized for expression in a human cellular environment is contemplated herein. Optimized synthetic HIV gag genes are described in U.S. Patent No. 6.696.291.

The recombinant yellow fever viruses contemplated herein are prepared through the insertion of the sequence encoding the heterologous polypeptide into the genome of the yellow fever virus or vector which can replicate the yellow fever virus. Adequate yellow fever viruses include attenuated strains such as YF17D. Adequate insertion sites for the sequence encoding the heterologous polypeptide include sites between the sequences encoding viral proteins E and NS1. The sequence encoding the heterologous polypeptide may be codon-optimized based on the use of the codon for the yellow fever virus genome.

In another embodiment of the recombinant viruses described herein, the virus is a genetically modified vaccinal YF 17D virus which expresses amino acids 45-269 of SIVmac239Gag (rYF17D/SIVGag45-269), and is prepared through the insertion of a codon-optimized sequence based on YF genome between the genes which encode viral proteins E and NS1.

In one example, the antigen used for expression by the yellow fever 17D virus was a fragment of a Gag protein comprising amino acid residues 45 to 269. Gag is a precursory protein that, on being processed by the viral protease, generates the internal structural proteins of the mature virion. Gag protein induces a strong protective immune response mediated by CD8+ T cells in rhesus monkeys and, on a smaller scale, in humans. This immune response is very important since it may provide protection against viral infection or reduce the viral load and delay the onset of disease.

In some embodiments, the following criteria may be used to select a Gag fragment to be cloned and expressed in the E/NS1 region of 17D virus genome. First, the presence of CD8+ T-cell epitopes in the Gag polypeptide may be taken into consideration based on immunological studies in rhesus monkeys. Furthermore, preferably, the insertion of the Gag polypeptide should not disturb the translocation of the YF precursory protein into the endoplasmic reticulum, as well as its processing by viral and cell proteases, if these steps occur during virus morphogenesis and assembly. Thus, preferably, the Gag polypeptide fragment should not be hydrophobic or contain a transmembrane motif, which might produce a rupture or significantly destabilize the correct topology of the membrane and precursory protein processing. Therefore, the selected Gag polypeptide fragment is hydrophilic and does not include apparent transmembrane domains. Besides, a very flexible motif, KESSIG, is fused to the C-terminal of the Gag sequence. This sort of motif is present in all flaviviruses and proteins binding domain III and the anchorage region ("stem anchor") of E protein. It is a very flexible motif and was used here as a separating sequence to connect and move away the Gag fragment and alpha helices of the anchorage domain ("stem anchor") of truncated dengue 4 virus E protein in the recombinant cassette.

Moreover, the virus vaccines presently disclosed differ from the virus vaccines of the state of the art by using an attenuated vector structure and emphasizing the generation of cell immune responses for specific viral epitopes in certain proteins. The strategy for vector virus replication is maximizing the production of antigens in an appropriate cell location to induce an appropriate response in terms of specificity and magnitude.

Vaccination with a single dose of attenuated yellow fever virus 17D causes a limited viral infection and promotes the generation of neutralizing antibodies as well as T-cell responses which confer protective immunity to more than 95% of vaccinees, providing immunity for over 30 years. The yellow fever 17D vaccine is regarded as one of the most successful vaccines available. Vaccine production concerns a well-established process and, since 1937, over 540 million doses have been used in humans with a minimal incidence of severe side effects [9,10].

The current methods are based on the methodology that was previously developed by two of the present inventors. The methodology is described in WO07051267, which is incorporated by reference herein.

The method for the production of recombinant viruses with nucleotide sequences totally or partially encoding the heterologous proteins, as described in WO07051267, may comprise the following steps:
(a) modification of the heterologous nucleotide sequences in such a way that, when they are cloned in the vector virus, they present, in the 5' region, nucleotides of the 5' end of the vector virus NS1 gene or functionally equivalent sequences, and, in its 3' end, the totality or part of the anchorage genome region ("stem anchor") of E protein of the vector virus or functionally equivalent sequences, which do not compromise the structure and replication of said vector virus;
(b) insertion of the modified heterologous sequences as described in (a) into the intergenic region between the genes encoding E structural protein and NS1 nonstructural protein of the vector virus;
(c) regeneration of the nonpathogenic recombinant virus, having the heterologous sequences stably integrated into the viral genome at the insertion point described in (b), and expression of the heterologous sequence in such a way that the corresponding protein induces an appropriate immune response.

Thus, the inventors found that the strategy for E/NS1 insertion may be applied to generate recombinant vaccines against lentiviruses, particularly the HIV (Human Immunodeficiency Virus).
In one embodiment of a recombinant virus as contemplated herein, an attenuated recombinant virus is a yellow fever vaccinal virus 17D expressing a sequence of SIVmac239 Gag sequences which is used as a viral vector to originate SIV-specific CD8+ T-cell responses in rhesus monkeys. The model of Asian rhesus monkey was employed to validate the expression of HIV antigens in the yellow fever 17D virus vector because the SIV-infected rhesus monkey model reproduces, in many aspects, the immunodeficiency that occurs in HIV-infected humans. First, several SIV strains are closely related to HIV-1 strains and infect CD4+ T cells, giving rise to a decline in their counts over time. SIV infection causes a disease that is similar to AIDS in most of the monkeys infected for one year after inoculation. Moreover, many genes of the monkeys which encode key proteins in the immune system are similar to those of humans. Equivalents of the genes for HLA class, class II [sic] and T-cell receptor (TCR) are found in the monkeys. Orthologs of HLA-A, -B, -E and -F locations were isolated in rhesus monkeys. In both infections, CD8+ T cells select the major part of the sequence variation outside the envelope protein and the natural control of viral replication is associated with certain MHC-I alleles [11,12].

Consequently, as disclosed herein, the vaccination of rhesus monkeys with different formulations containing recombinant yellow fever virus was assessed so as to determine the potential use of 17D vector in the development of new vaccines against HIV. The recombinant yellow fever virus obtained expresses a SIVmac239 Gag fragment encompassing amino acids 45 to 269. Gag is a precursory protein and, in the course of viral replication, it undergoes proteolytic splitting by the viral protease, producing the internal structural proteins of the mature virion. It was determined that multiple Gag-specific responses are important in the control of SIV replication. This may be due to infected cells which present Gag-derived CD8+ T-cell epitopes right after the viral entry. Gag-specific CD8+ T cells recognize infected cells in up to 2 hours after the infection. Six hours after the infection, Gag-specific CD8+ T cells eliminate the infected cells, both before and after the integration of proviral DNA and viral protein synthesis. These findings demonstrate that Gag-specific CD8+ T cells do not require productive cell infection or even protein synthesis "again" to recognize the infected cells and eliminate them. This suggests that Gag-specific CD8+ T cells may be much more important than previously imagined.

In order to reach the goals disclosed herein, the following sequences were used:
**SEQ. ID. NO.: 1** 
MGARNSVLSGKKADELEKIRLRPNGKKKYMLKHVVWAANELDRFGLAESLLENKEGCQK
ILSVLAPLVPTGSENLKSLYNTVCVIWCIHAEEKVKHTEEAKQIVQRHLVVETGTTETM
PKTSRPTAPSSGRGGNYPVQQIGGNYVHLPLSPRTLNAWVKLIEEKKFGAEVVPGFQAL
SEGCTPYDINQMLNCVGDHQAAMQIIRDIINEEAADWDLQHPQPAPQQGQLREPSGSDI
AGTTSSVDEQIQWMYRQQNPIPVGNIYRRWIQLGLQKCVRMYNPTNILDVKQGPKEPFQ
SYVDRFYKSLRAEQTDAAVKNWMTQTLLIQNANPDCKLVLKGLGVNPTLEEMLTACQGV
GGPGQKARLMAEALKEALAPVPIPFAAAQQRGPRKTIKCWNCGKEGHSARQCRAPRRQG
CWKCGKMDHVMAKCPDRQAGFLGLGPWGKKPRNFPMAQVHQGLMPTAPPEDPAVDLLKN
YMQLGKQQREKQRESRXKPYKEVTEDLLHLNSLFGGDQ. (TO BE CONFIRMED,
 but in several seqs. of the GENBANK submitted by David's group, the seqs. have 510 amino acids - the final Q residue is missing).
SEQ. ID. NO.: 2 is a sequence of amino acids of the recombinant protein Gag 45-269, cloned and expressed in the E/NS1 intergenic region by the yellow fever 17D virus. Gag45-269 fragment is shaded in gray.
   **SEQ. ID. NO.: 2** 
DQGSAINFGRGLAESLLENKEGCQKILSVLAPLVPTGSENLKSLYNTVCVIWCIHAEEK
VKHTEEAKQIVQRHLVVETGTTETMPKTSRPTAPSSGRGGNYPVQQIGGNYVHLPLSPR
TLNAWVKLIEEKKFGAEVVPGFQALSEGCTPYDINQMLNCVGDHQAAMQIIRDIINEEA
ADWDLQHPQPAPQQGQLREPSGSDIAGTTSSVDEQIQWMYRQQNPIPVGNIYRRWIQLK
ESSIGTSLGKAVHQVFGSVYTTMFGGVSWMIRILIGFLVLWIGTNSRNTSMAMTCIAVG
GITLFLGFTVGA
 Wherein the other sequences present in SEQ. ID. NO.: 2 are sequences of the yellow fever virus and correspond to:
   **SEQ. ID. NO.:3 -** DQGSAINFGR
SEQ. ID. NO.: 3 is a variation of 10 amino acid residues of the N-terminal sequence of NS1 protein of the yellow fever virus (DQGCAINFGK); YF genome position from 2453 to 2482) [sic] in which an amino acid replacement was introduced (from cysteine to serine, shaded in gray).
**SEQ. ID. NO.: 4 -** KESSIG
   SEQ. ID. NO.: 4 is the sequence of amino acids corresponding both to the sequence of nucleotides of the position from 2145 to 2164 of the yellow fever virus genome and the motif comprising the amino acid residues 391 to 397 of E protein of the yellow fever virus.
**SEQ. ID. NO.:.5 -** 
TSLGKAVHQVFGSVYTTMFGGVSWMIRILIGFLVLWIGTNSRNTSMAMTCIAVGGITLF

LGFTVGA
 SEQ. ID. NO.: 5 is a variation of the last -terminal [sic] amino acid residues of E protein of dengue virus serotype 4 (dengue virus serotype 4 genome position from 2226 to 2423; GenBank AF326825). SEQ. ID. NO.: 5 includes a replacement relative to the original sequence where the amino acid terminal sequence VQA is changed to VGA.
SEQ. ID. NO.: 6 is a nucleotide sequence (924 nucleotides) of the cassette of recombinant Gag45-269, cloned in the E/NS1 intergenic region of the yellow fever 17D virus. Gag 45-269 fragment is shaded in gray.
   **SEQ. ID. NO.: 6** 
5´GATCAAGGAAGCGCCATCAACTTCGGCCGAGGACTGGCTGAATCACTGCTGGAAAAC
AAAGAAGGATGTCAGAAAATCCTGTCAGTGCTGGCTCCACTGGTGCCAACAGGATCAGA
AAACCTGAAGTCACTGTACAACACAGTGTGTGATCTGGTGTATCCATGCTGAAGAAA
AAGTGAAGCATACAGAAGAGGCCAAGCAGATCGTGCAGAGGCATCTGGTGGTGGAAACA
GGGACAACAGAAACAATGCCAAAGACATCAAGGCCAACAGCTCCATCATCAGGAAGGGG
GGGAAACTACCCAGTGCAGCAGATCGGAGGAACTACGTGCATCTGCCACTGTCACCAA
 
GGACACTGAACGCTTGGGTGAAACTGATCGAAGAAAAGAAATTTGGAGCTGAAGTGGTG
CCAGGATTTCAGGCTCTGTCAGAAGGATGTACACCATACGACATCAACCAGATGCTGAA
CTGTGTGGGAGATCATCAGGCTGCTATGCAGATCATTAGGGACATCATCAACGAAGAAG
CTGCAGATTGGGATCTGCAACATCCACAGCCAGCTCCACAGCAGGACAGCTGAGGGAA
CCATCAGGATCAGACATTGCTGGAACAACATCATCAGTGGACGAACAGATCCAGTGGAT
GTACAGGCAGCAGAACCCAATCCCAGTGGAAACATCTACAGAAGATGGATCCAGCTGA
AAGAGGGAAGCTCAATAGGAACATCATTGGGAAAGGCTGTGCACCAGGTTTTTGGAAGT
GTGTATACAACCATGTTTGGAGGAGTCTCATGGATGATTAGAATCCTAATTGGGTTCTT
AGTGTTGTGGATTGGCACGAACTCCAGGAACACTTCAATGGCTATGACGTGCATAGCTG
TTGGAGGAATCACTCTGTTTCTGGGCTTCACAGTTGGCGCC 3′
SEQ. ID. NO.: 7 is a sequence of 11, 785 [sic] nucleotides of the recombinant virus genome taking cassette Gag 45-269, called rYF17D/SIVGag45-269.
   **SEQ. ID. NO.: 7** 
5′
AGTAAATCCTGTGTGCTAATTGAGGTGCATTGGTCTGCAAATCGAGTTGCTAGGCAATA
AACACATTTGGATTAATTTTAATCGTTCGTTGAGCGATTAGCAGAGAACTGACCAGAAC
ATGTCTGGTCGTAAAGCTCAGGGAAAAACCCTGGGCGTCAATATGGTACGACGAGGAGT
TCGCTCCTTGTCAAACAAAATAAAACAAAAAACAAAACAAATTGGAAACAGACCTGGAC
CTTCAAGAGGTGTTCAAGGATTTATCTTTTTCTTTTTGTTCAACATTTTGACTGGAAAA
AAGATCACAGCCCACCTAAAGAGGTTGTGGAAAATGCTGGACCCAAGACAAGGCTTGGC
TGTTCTAAGGAAAGTCAAGAGAGTGGTGGCCAGTTTGATGAGAGGATTGTCCTCAAGGA
AACGCCGTTCCCATGATGTTCTGACTGTGCAATTCCTAATTTTGGGAATGCTGTTGATG
ACCGGTGGAGTGACCTTGGTGCGGAAAAACAGATGGTTGCTCCTAAATGTGACATCTGA
GGACCTCGGGAAAACATTCTCTGTGGGCACAGGCAACTGCACAACAAACATTTTGGAAG
CCAAGTACTGGTGCCCAGACTCAATGGAATACAACTGTCCCAATCTCAGTCCAAGAGAG
GAGCCAGATGACATTGATTGCTGGTGCTATGGGGTGGAAAACGTTAGAGTCGCATATGG
TAAGTGTGACTCAGCAGGCAGGTCTAGGAGGTCAAGAAGGGCCATTGACTTGCCTACGC
ATGAAAACCATGGTTTGAAGACCCGGCAAGAAAAATGGATGACTGGAAGAATGGGTGAA
 
AGGCAACTCCAAAAGATTGAGAGATGGTTCGTGAGGAACCCCTTTTTTGCAGTGACGGC
TCTGACCATTGCCTACCTTGTGGGAAGCAACATGACGCAACGAGTCGTGATTGCCCTAC
TGGTCTTGGCTGTTGGTCCGGCCTACTCAGCTCACTGCATTGGAATTACTGACAGGGAT
TTCATTGAGGGGGTGCATGGAGGAACTTGGGTTTCAGCTACCCTGGAGCAAGACAAGTG
TGTCACTGTTATGGCCCCTGACAAGCCTTCATTGGACATCTCACTAGAGACAGTAGCCA
TTGATAGACCTGCTGAGGCGAGGAAAGTGTGTTACAATGCAGTTCTCACTCATGTGAAG
ATTAATGACAAGTGCCCCAGCACTGGAGAGGCCCACCTAGCTGAAGAGAACGAAGGGGA
CAATGCGTGCAAGCGCACTTATTCTGATAGAGGCTGGGGCAATGGCTGTGGCCTATTTG
GGAAAGGGAGCATTGTGGCATGCGCCAAATTCACTTGTGCCAAATCCATGAGTTTGTTT
GAGGTTGATCAGACCAAAATTCAGTATGTCATCAGAGCACAATTGCATGTAGGGGCCAA
GCAGGAAAATTGGAATACCAGCATTAAGACTCTCAAGTTTGATGCCCTGTCAGGCTCCC
AGGAAGTCGAGTTCATTGGGTATGGAAAAGCTACACTGGAATGCCAGGTGCAAACTGCG
GTGGACTTTGGTAACAGTTACATCGCTGAGATGGAAACAGAGAGCTGGATAGTGGACAG
ACAGTGGGCCCAGGACTTGACCCTGCCATGGCAGAGTGGAAGTGGCGGGGTGTGGAGAG
AGATGCATCATCTTGTCGAATTTGAACCTCCGCATGCCGCCACTATCAGAGTACTGGCC
CTGGGAAACCAGGAAGGCTCCTTGAAAACAGCTCTTACTGGCGCAATGAGGGTTACAAA
GGACACAAATGACAACAACCTTTACAAACTACATGGTGGACATGTTTCTTGCAGAGTGA
AATTGTCAGCTTTGACACTCAAGGGGACATCCTACAAAATATGCACTGACAAAATGTTT
TTTGTCAAGAACCCAACTGACACTGGCCATGGCACTGTTGTGATGCAGGTGAAAGTGTC
AAAAGGAACCCCCTGCAGGATTCCAGTGATAGTAGCTGATGATCTTACAGCGGCAATCA
ATAAAGGCATTTTGGTTACAGTTAACCCCATCGCCTCAACCAATGATGATGAAGTGCTG
ATTGAGGTGAACCCACCTTTTGGAGACAGCTACATTATCGTTGGGAGAGGAGATTCACG
TCTCACTTACCAGTGGCACAAAGAGGGAAGCTCAATAGGAAAGTTGTTCACTCAGACCA
TGAAAGGCGTGGAACGCCTGGCCGTCATGGGAGACACCGCCTGGGATTTCAGCTCCGCT
GGAGGGTTCTTCACTTCGGTTGGGAAAGGAATTCATACGGTGTTTGGCTCTGCCTTTCA
GGGGCTATTTGGCGGCTTGAACTGGATAACAAAGGTCATCATGGGGGCGGTACTCATAT
GGGTTGGCATCAACACAAGAAACATGACAATGTCCATGAGCATGATCTTGGTAGGAGTG
ATCATGATGTTTTTGTCTCTAGGAGTTGGGGCGGATCAAGGAAGCGCCATCAACTTCGG
CCGAGGACTGGCTGAATCACTGCTGGAAAACAAAGAAGGATGTCAGAAAATCCTGTCAG
 
TGCTGGCTCCACTGGTGCCAACAGGATCAGAAAACCTGAAGTCACTGTACAACACAGTG
TGTGTGATCTGGTGTATCCATGCTGAAGAAAAAGTGAAGCATACAGAAGAGGCCAAGCA
GATCGTGCAGAGGCATCTGGTGGTGGAAACAGGGACAACAGAAACAATGCCAAAGACAT
CAAGGCCAACAGCTCCATCATCAGGAAGGGGGGAAACTACCCAGTGCAGCAGATCGGA
GGGAACTACGTGCATCTGCCACTGTCACCAAGGACACTGAACGCTTGGGTGAAACTGAT
CGAAGAAAAGAAATTTGGAGCTGAAGTGGTGCCAGGATTTCAGGCTCTGTCAGAAGGAT
GTACACCATACGACATCAACCAGATGCTGAACTGTGTGGGAGATCATCAGGCTGCTATG
CAGATCATTAGGGACATCATCAACGAAGAAGCTGCAGATTGGGATCTGCAACATCCACA
GCCAGCTCCACAGCAGGGACAGCTGAGGGAACCATCAGGATCAGACATTGCTGGAACAA
CATCATCAGTGGACGAACAGATCCAGTGGATGTACAGGCAGCAGAACCCAATCCCAGTG
GGAAACATCTACAGAAGATGGATCCAGCTGAAAGAGGGAAGCTCAATAGGAACATCATT
GGGAAAGGCTGTGCACCAGGTTTTTGGAAGTGTGTATACAACCATGTTTGGAGGAGTCT
CATGGATGATTAGAATCCTAATTGGGTTCTTAGTGTTGTGGATTGGCACGAACTCCAGG
AACACTTCAATGGCTATGACGTGCATAGCTGTTGGAGGAATCACTCTGTTTCTGGGCTT
CACAGTTGGCGCcGATCAAGGATGCGCCATCAACTTTGGCAAGAGAGAGCTCAAGTGCG
GAGATGGTATCTTCATATTTAGAGACTCTGATGACTGGCTGAACAAGTACTCATACTAT
CCAGAAGATCCTGTGAAGCTTGCATCAATAGTGAAAGCCTCTTTCGAAGAAGGGAAGTG
TGGCCTAAATTCAGTTGACTCCCTTGAGCATGAGATGTGGAGAAGCAGGGCAGATGAGA
TTAATACCATTTTTGAGGAAAACGAGGTGGACATTTCTGTTGTCGTGCAGGATCCAAAG
AATGTTTACCAGAGAGGAACTCATCCATTTTCCAGAATTCGGGATGGTCTGCAGTATGG
TTGGAAGACTTGGGGTAAGAACCTTGTGTTCTCCCCAGGGAGGAAGAATGGAAGCTTCA
TCATAGATGGAAAGTCCAGGAAAGAATGCCCGTTTTCAAACCGGGTCTGGAATTCTTTC
CAGATAGAGGAGTTTGGGACGGGAGTGTTCACCACACGCGTGTACATGGACGCAGTCTT
TGAATACACCATAGACTGCGATGGATCTATCTTGGGTGCAGCGGTGAACGGAAAAAAGA
GTGCCCATGGCTCTCCAACATTTTGGATGGGAAGTCATGAAGTAAATGGGACATGGATG
ATCCACACCTTGGAGGCATTAGATTACAAGGAGTGTGAGTGGCCACTGACACATACGAT
TGGAACATCAGTTGAAGAGAGTGAAATGTTCATGCCGAGATCAATCGGAGGCCCAGTTA
GCTCTCACAATCATATCCCTGGATACAAGGTTCAGACGAACGGACCTTGGATGCAGGTA
CCACTAGAAGTGAAGAGAGAAGCTTGCCCAGGGACTAGCGTGATCATTGATGGCAACTG
 
TGATGGACGGGGAAAATCAACCAGATCCACCACGGATAGCGGGAAAGTTATTCCTGAAT
GGTGTTGCCGCTCCTGCACAATGCCGCCTGTGAGCTTCCATGGTAGTGATGGGTGTTGG
TATCCCATGGAAATTAGGCCAAGGAAAACGCATGAAAGCCATCTGGTGCGCTCCTGGGT
TACAGCTGGAGAAATACATGCTGTCCCTTTTGGTTTGGTGAGCATGATGATAGCAATGG
AAGTGGTCCTAAGGAAAAGACAGGGACCAAAGCAAATGTTGGTTGGAGGAGTAGTGCTC
TTGGGAGCAATGCTGGTCGGGCAAGTAACTCTCCTTGATTTGCTGAAACTCACAGTGGC
TGTGGGATTGCATTTCCATGAGATGAACAATGGAGGAGACGCCATGTATATGGCGTTGA
TTGCTGCCTTTTCAATCAGACCAGGGCTGCTCATCGGCTTTGGGCTCAGGACCCTATGG
AGCCCTCGGGAACGCCTTGTGCTGACCCTAGGAGCAGCCATGGTGGAGATTGCCTTGGG
TGGCGTGATGGGCGGCCTGTGGAAGTATCTAAATGCAGTTTCTCTCTGCATCCTGACAA
TAAATGCTGTTGCTTCTAGGAAAGCATCAAATACCATCTTGCCCCTCATGGCTCTGTTG
ACACCTGTCACTATGGCTGAGGTGAGACTTGCCGCAATGTTCTTTTGTGCCATGGTTAT
CATAGGGGTCCTTCACCAGAATTTCAAGGACACCTCCATGCAGAAGACTATACCTCTGG
TGGCCCTCACACTCACATCTTACCTGGGCTTGACACAACCTTTTTTGGGCCTGTGTGCA
TTTCTGGCAACCCGCATATTTGGGCGAAGGAGTATCCCAGTGAATGAGGCACTCGCAGC
AGCTGGTCTAGTGGGAGTGCTGGCAGGACTGGCTTTTCAGGAGATGGAGAACTTCCTTG
GTCCGATTGCAGTTGGAGGACTCCTGATGATGCTGGTTAGCGTGGCTGGGAGGGTGGAT
GGGCTAGAGCTCAAGAAGCTTGGTGAAGTTTCATGGGAAGAGGAGGCGGAGATCAGCGG
GAGTTCCGCCCGCTATGATGTGGCACTCAGTGAACAAGGGGAGTTCAAGCTGCTTTCTG
AAGAGAAAGTGCCATGGGACCAGGTTGTGATGACCTCGCTGGCCTTGGTTGGGGCTGCC
CTCCATCCATTTGCTCTTCTGCTGGTCCTTGCTGGGTGGCTGTTTCATGTCAGGGGAGC
TAGGAGAAGTGGGGATGTCTTGTGGGATATTCCCACTCCTAAGATCATCGAGGAATGTG
AACATCTGGAGGATGGGATTTATGGCATATTCCAGTCAACCTTCTTGGGGGCCTCCCAG
CGAGGAGTGGGAGTGGCACAGGGAGGGGTGTTCCACACAATGTGGCATGTCACAAGAGG
AGCTTTCCTTGTCAGGAATGGCAAGAAGTTGATTCCATCTTGGGCTTCAGTAAAGGAAG
ACCTTGTCGCCTATGGTGGCTCATGGAAGTTGGAAGGCAGATGGGATGGAGAGGAAGAG
GTCCAGTTGATCGCGGCTGTTCCAGGAAAGAACGTGGTCAACGTCCAGACAAAACCGAG
CTTGTTCAAAGTGAGGAATGGGGGAGAAATCGGGGCTGTCGCTCTTGACTATCCGAGTG
GCACTTCAGGATCTCCTATTGTTAACAGGAACGGAGAGGTGATTGGGCTGTACGGCAAT
 
GGCATCCTTGTCGGTGACAACTCCTTCGTGTCCGCCATATCCCAGACTGAGGTGAAGGA
AGAAGGAAAGGAGGAGCTCCAAGAGATCCCGACAATGCTAAAGAAAGGAATGACAACTG
TCCTTGATTTTCATCCTGGAGCTGGGAAGACAAGACGTTTCCTCCCACAGATCTTGGCC
GAGTGCGCACGGAGACGCTTGCGCACTCTTGTGTTGGCCCCCACCAGGGTTGTTCTTTC
TGAAATGAAGGAGGCTTTTCACGGCCTGGACGTGAAATTCCACACACAGGCTTTTTCCG
CTCACGGCAGCGGGAGAGAAGTCATTGATGCCATGTGCCATGCCACCCTAACTTACAGG
ATGTTGGAACCAACTAGGGTTGTTAACTGGGAAGTGATCATTATGGATGAAGCCCATTT
TTTGGATCCAGCTAGCATAGCCGCTAGAGGTTGGGCAGCGCACAGAGCTAGGGCAAATG
AAAGTGCAACAATCTTGATGACAGCCACACCGCCTGGGACTAGTGATGAATTTCCACAT
TCAAATGGTGAAATAGAAGATGTTCAAACGGACATACCCAGTGAGCCCTGGAACACAGG
GCATGACTGGATCCTGGCTGACAAAAGGCCCACGGCATGGTTCCTTCCATCCATCAGAG
CTGCAAATGTCATGGCTGCCTCTTTGCGTAAGGCTGGAAAGAGTGTGGTGGTCCTGAAC
AGGAAAACCTTTGAGAGAGAATACCCCACGATAAAGCAGAAGAAACCTGACTTTATATT
GGCCACTGACATAGCTGAAATGGGAGCCAACCTTTGCGTGGAGCGAGTGCTGGATTGCA
GGACGGCTTTTAAGCCTGTGCTTGTGGATGAAGGGAGGAAGGTGGCAATAAAAGGGCCA
CTTCGTATCTCCGCATCCTCTGCTGCTCAAAGGAGGGGGCGCATTGGGAGAAATCCCAA
CAGAGATGGAGACTCATACTACTATTCTGAGCCTACAAGTGAAAATAATGCCCACCACG
TCTGCTGGTTGGAGGCCTCAATGCTCTTGGACAACATGGAGGTGAGGGGTGGAATGGTC
GCCCCACTCTATGGCGTTGAAGGAACTAAAACACCAGTTTCCCCTGGTGAAATGAGACT
GAGGGATGACCAGAGGAAAGTCTTCAGAGAACTAGTGAGGAATTGTGACCTGCCCGTTT
GGCTTTCGTGGCAAGTGGCCAAGGCTGGTTTGAAGACGAATGATCGTAAGTGGTGTTTT
GAAGGCCCTGAGGAACATGAGATCTTGAATGACAGCGGTGAAACAGTGAAGTGCAGGGC
TCCTGGAGGAGCAAAGAAGCCTCTGCGCCCAAGGTGGTGTGATGAAAGGGTGTCATCTG
ACCAGAGTGCGCTGTCTGAATTTATTAAGTTTGCTGAAGGTAGGAGGGGAGCTGCTGAA
GTGCTAGTTGTGCTGAGTGAACTCCCTGATTTCCTGGCTAAAAAAGGTGGAGAGGCAAT
GGATACCATCAGTGTGTTCCTCCACTCTGAGGAAGGCTCTAGGGCTTACCGCAATGCAC
TATCAATGATGCCTGAGGCAATGACAATAGTCATGCTGTTTATACTGGCTGGACTACTG
ACATCGGGAATGGTCATCTTTTTCATGTCTCCCAAAGGCATCAGTAGAATGTCTATGGC
GATGGGCACAATGGCCGGCTGTGGATATCTCATGTTCCTTGGAGGCGTCAAACCCACTC
 
ACATCTCCTATGTCATGCTCATATTCTTTGTCCTGATGGTGGTTGTGATCCCCGAGCCA
GGGCAACAAAGGTCCATCCAAGACAACCAAGTGGCATACCTCATTATTGGCATCCTGAC
GCTGGTTTCAGCGGTGGCAGCCAACGAGCTAGGCATGCTGGAGAAAACCAAAGAGGACC
TCTTTGGGAAGAAGAACTTAATTCCATCTAGTGCTTCACCCTGGAGTTGGCCGGATCTT
GACCTGAAGCCAGGAGCTGCCTGGACAGTGTACGTTGGCATTGTTACAATGCTCTCTCC
AATGTTGCACCACTGGATCAAAGTCGAATATGGCAACCTGTCTCTGTCTGGAATAGCCC
AGTCAGCCTCAGTCCTTTCTTTCATGGACAAGGGGATACCATTCATGAAGATGAATATC
TCGGTCATAATGCTGCTGGTCAGTGGCTGGAATTCAATAACAGTGATGCCTCTGCTCTG
TGGCATAGGGTGCGCCATGCTCCACTGGTCTCTCATTTTACCTGGAATCAAAGCGCAGC
AGTCAAAGCTTGCACAGAGAAGGGTGTTCCATGGCGTTGCCAAGAACCCTGTGGTTGAT
GGGAATCCAACAGTTGACATTGAGGAAGCTCCTGAAATGCCTGCCCTTTATGAGAAGAA
ACTGGCTCTATATCTCCTTCTTGCTCTCAGCCTAGCTTCTGTTGCCATGTGCAGAACGC
CCTTTTCATTGGCTGAAGGCATTGTCCTAGCATCAGCTGCCTTAGGGCCGCTCATAGAG
GGAAACACCAGCCTTCTTTGGAATGGACCCATGGCTGTCTCCATGACAGGAGTCATGAG
GGGGAATCACTATGCTTTTGTGGGAGTCATGTACAATCTATGGAAGATGAAAACTGGAC
GCCGGGGGAGCGCGAATGGAAAAACTTTGGGTGAAGTCTGGAAGAGGGAACTGAATCTG
TTGGACAAGCGACAGTTTGAGTTGTATAAAAGGACCGACATTGTGGAGGTGGATCGTGA
TACGGCACGCAGGCATTTGGCCGAAGGGAAGGTGGACACCGGGGTGGCGGTCTCCAGGG
GGACCGCAAAGTTAAGGTGGTTCCATGAGCGTGGCTATGTCAAGCTGGAAGGTAGGGTG
ATTGACCTGGGGTGTGGCCGCGGAGGCTGGTGTTACTACGCTGCTGCGCAAAAGGAAGT
GAGTGGGGTCAAAGGATTTACTCTTGGAAGAGACGGCCATGAGAAACCCATGAATGTGC
AAAGTCTGGGATGGAACATCATCACCTTCAAGGACAAAACTGATATCCACCGCCTAGAA
CCAGTGAAATGTGACACCCTTTTGTGTGACATTGGAGAGTCATCATCGTCATCGGTCAC
AGAGGGGGAAAGGACCGTGAGAGTTCTTGATACTGTAGAAAAATGGCTGGCTTGTGGGG
TTGACAACTTCTGTGTGAAGGTGTTAGCTCCATACATGCCAGATGTTCTTGAGAAACTG
GAATTGCTCCAAAGGAGGTTTGGCGGAACAGTGATCAGGAACCCTCTCTCCAGGAATTC
CACTCATGAAATGTACTACGTGTCTGGAGCCCGCAGCAATGTCACATTTACTGTGAACC
AAACATCCCGCCTCCTGATGAGGAGAATGAGGCGTCCAACTGGAAAAGTGACCCTGGAG
GCTGACGTCATCCTCCCAATTGGGACACGCAGTGTTGAGACAGACAAGGGACCCCTGGA
 
CAAAGAGGCCATAGAAGAAAGGGTTGAGAGGATAAAATCTGAGTACATGACCTCTTGGT
TTTATGACAATGACAACCCCTACAGGACCTGGCACTACTGTGGCTCCTATGTCACAAAA
ACCTCAGGAAGTGCGGCGAGCATGGTAAATGGTGTTATTAAAATTCTGACATATCCATG
GGACAGGATAGAGGAGGTCACCAGAATGGCAATGACTGACACAACCCCTTTTGGACAGC
AAAGAGTGTTTAAAGAAAAAGTTGACACCAGAGCAAAGGATCCACCAGCGGGAACTAGG
AAGATCATGAAAGTTGTCAACAGGTGGCTGTTCCGCCACCTGGCCAGAGAAAAGAGCCC
CAGACTGTGCACAAAGGAAGAATTTATTGCAAAAGTCCGAAGTCATGCAGCCATTGGAG
CTTACCTGGAAGAACAAGAACAGTGGAAGACTGCCAATGAGGCTGTCCAAGACCCAAAG
TTCTGGGAACTGGTGGATGAAGAAAGGAAGCTGCACCAACAAGGCAGGTGTCGGACTTG
TGTGTACAACATGATGGGGAAAAGAGAGAAGAAGCTGTCAGAGTTTGGGAAAGCAAAGG
GAAGCCGTGCCATATGGTATATGTGGCTGGGAGCGCGGTATCTTGAGTTTGAGGCCCTG
GGATTCCTGAATGAGGACCATTGGGCTTCCAGGGAAAACTCAGGAGGAGGAGTGGAAGG
CATTGGCTTACAATACCTAGGATATGTGATCAGAGACCTGGCTGCAATGGATGGTGGTG
GATTCTACGCGGATGACACCGCTGGATGGGACACGCGCATCACAGAGGCAGACCTTGAT
GATGAACAGGAGATCTTGAACTACATGAGCCCACATCACAAAAAACTGGCACAAGCAGT
GATGGAAATGACATACAAGAACAAAGTGGTGAAAGTGTTGAGACCAGCCCCAGGAGGGA
AAGCCTACATGGATGTCATAAGTCGACGAGACCAGAGAGGATCCGGGCAGGTAGTGACT
TATGCTCTGAACACCATCACCAACTTGAAAGTCCAATTGATCAGAATGGCAGAAGCAGA
GATGGTGATACATCACCAACATGTTCAAGATTGTGATGAATCAGTTCTGACCAGGCTGG
AGGCATGGCTCACTGAGCACGGATGTAACAGACTGAAGAGGATGGCGGTGAGTGGAGAC
GACTGTGTGGTCCGGCCCATCGATGACAGGTTCGGCCTGGCCCTGTCCCATCTCAACGC
CATGTCCAAGGTTAGAAAGGACATATCTGAATGGCAGCCATCAAAAGGGTGGAATGATT
GGGAGAATGTGCCCTTCTGTTCCCACCACTTCCATGAACTACAGCTGAAGGATGGCAGG
AGGATTGTGGTGCCTTGCCGAGAACAGGACGAGCTCATTGGGAGAGGAAGGGTGTCTCC
AGGAAACGGCTGGATGATCAAGGAAACAGCTTGCCTCAGCAAAGCCTATGCCAACATGT
GGTCACTGATGTATTTTCACAAAAGGGACATGAGGCTACTGTCATTGGCTGTTTCCTCA
GCTGTTCCCACCTCATGGGTTCCACAAGGACGCACAACATGGTCGATTCATGGGAAAGG
GGAGTGGATGACCACGGAAGACATGCTTGAGGTGTGGAACAGAGTATGGATAACCAACA
ACCCACACATGCAGGACAAGACAATGGTGAAAAAATGGAGAGATGTCCCTTATCTAACC
 
AAGAGACAAGACAAGCTGTGCGGATCACTGATTGGAATGACCAATAGGGCCACCTGGGC
CTCCCACATCCATTTAGTCATCCATCGTATCCGAACGCTGATTGGACAGGAGAAATACA
CTGACTACCTAACAGTCATGGACAGGTATTCTGTGGATGCTGACCTGCAACTGGGTGAG
CTTATCTGAAACACCATCTAACAGGAATAACCGGGATACAAACCACGGGTGGAGAACCG
GACTCCCCACAACCTGAAACCGGGATATAAACCACGGCTGGAGAACCGGGCTCCGCACT
TAAAATGAAACAGAAACCGGGATAAAAACTACGGATGGAGAACCGGACTCCACACATTG
AGACAGAAGAAGTTGTCAGCCCAGAACCCCACACGAGTTTTGCCACTGCTAAGCTGTGA
GGCAGTGCAGGCTGGGACAGCCGACCTCCAGGTTGCGAAAAACCTGGTTTCTGGGACCT
CCCACCCCAGAGTAAAAAGAACGGAGCCTCCGCTACCACCCTCCCACGTGGTGGTAGAA
AGACGGGGTCTAGAGGTTAGAGGAGACCCTCCAGGGAACAAATAGTGGGACCATATTGA
CGCCAGGGAAAGACCGGAGTGGTTCTCTGCTTTTCCTCCAGAGGTCTGTGAGCACAGTT
TGCTCAAGAATAAGCAGACCTTTGGATGACAAACACAAAACCAC 3′
SEQ. ID. NO.: 8 is a precursory sequence of the polyprotein of virus rYF17D/SIVGag45-269. The recombinant protein is shaded in gray.
   **SEQ. ID. NO.: 8**

SGRKAQGKTLGVNMVRRGVRSLSNKIKQKTKQIGNRPGPSRGVQGFIFFFLFNILTGKK
ITAHLKRLWKMLDPRQGLAVLRKVKRVVASLMRGLSSRKRRSHDVLTVQFLILGMLLMT
GGVTLVRKNRWLLLNVTSEDLGKTFSVGTGNCTTNILEAKYWCPDSMEYNCPNLSPREE
PDDIDCWCYGVENVRVAYGKCDSAGRSRRSRRAIDLPTHENHGLKTRQEKWMTGRMGER
QLQKIERWFVRNPFFAVTALTIAYLVGSNMTQRVVIALLVLAVGPAYSAHCIGITDRDF
IEGVHGGTWVSATLEQDKCVTVMAPDKPSLDISLETVAIDRPAEARKVCYNAVLTHVKI
NDKCPSTGEAHLAEENEGDNACKRTYSDRGWGNGCGLFGKGSIVACAKFTCAKSMSLFE
VDQTKIQYVIRAQLHVGAKQENWNTSIKTLKFDALSGSQEVEFIGYGKATLECQVQTAV
DFGNSYIAEMETESWIVDRQWAQDLTLPWQSGSGGVWREMHHLVEFEPPHAATIRVLAL
GNQEGSLKTALTGAMRVTKDTNDNNLYKLHGGHVSCRVKLSALTLKGTSYKICTDKMFF
VKNPTDTGHGTVVMQVKVSKGTPCRIPVIVADDLTAAINKGILVTVNPIASTNDDEVLI
EVNPPFGDSYIIVGRGDSRLTYQWHKEGSSIGKLFTQTMKGVERLAVMGDTAWDFSSAG
GFFTSVGKGIHTVFGSAFQGLFGGLNWITKVIMGAVLIWVGINTRNMTMSMSMILVGVI

MMFLSLGVGADQGSAINFGRGLAESLLENKEGCQKILSVLAPLVPTGSENLKSLYNTVC
VIWCIHAEEKVKHTEEAKQIVQRHLVVETGTTETMPKTSRPTAPSSGRGGNYPVQQIGG
NYVHLPLSPRTLNAWVKLIEEKKFGAEVVPGFQALSEGCTPYDINQMLNCVGDHQAAMQ
IIRDIINEEAADWDLQHPQPAPQQGQLREPSGSDIAGTTSSVDEQIQWMYRQQNPIPVG
NIYRRWIQLKEGSSIGTSLGKAVHQVFGSVYTTMFGGVSWMIRILIGFLVLWIGTNSRN
TSMAMTCIAVGGITLFLGFTVGADQGCAINFGKRELKCGDGIFIFRDSDDWLNKYSYYP
EDPVKLASIVKASFEEGKCGLNSVDSLEHEMWRSRADEINTIFEENEVDISVVVQDPKN
VYQRGTHPFSRIRDGLQYGWKTWGKNLVFSPGRKNGSFIIDGKSRKECPFSNRVWNSFQ
IEEFGTGVFTTRVYMDAVFEYTIDCDGSILGAAVNGKKSAHGSPTFWMGSHEVNGTWMI
HTLEALDYKECEWPLTHTIGTSVEESEMFMPRSIGGPVSSHNHIPGYKVQTNGPWMQVP
LEVKREACPGTSVIIDGNCDGRGKSTRSTTDSGKVIPEWCCRSCTMPPVSFHGSDGCWY
PMEIRPRKTHESHLVRSWVTAGEIHAVPFGLVSMMIAMEVVLRKRQGPKQMLVGGVVLL
GAMLVGQVTLLDLLKLTVAVGLHFHEMNNGGDAMYMALIAAFSIRPGLLIGFGLRTLWS
PRERLVLTLGAAMVEIALGGVMGGLWKYLNAVSLCILTINAVASRKASNTILPLMALLT
PVTMAEVRLAAMFFCAMVIIGVLHQNFKDTSMQKTIPLVALTLTSYLGLTQPFLGLCAF
LATRIFGRRSIPVNEALAAAGLVGVLAGLAFQEMENFLGPIAVGGLLMMLVSVAGRVDG
LELKKLGEVSWEEEAEISGSSARYDVALSEQGEFKLLSEEKVPWDQVVMTSLALVGAAL
HPFALLLVLAGWLFHVRGARRSGDVLWDIPTPKIIEECEHLEDGIYGIFQSTFLGASQR
GVGVAQGGVFHTMWHVTRGAFLVRNGKKLIPSWASVKEDLVAYGGSWKLEGRWDGEEEV
QLIAAVPGKNVVNVQTKPSLFKVRNGGEIGAVALDYPSGTSGSPIVNRNGEVIGLYGNG
ILVGDNSFVSAISQTEVKEEGKEELQEIPTMLKKGMTTVLDFHPGAGKTRRFLPQILAE
CARRRLRTLVLAPTRVVLSEMKEAFHGLDVKFHTQAFSAHGSGREVIDAMCHATLTYRM
LEPTRVVNWEVIIMDEAHFLDPASIAARGWAAHRARANESATILMTATPPGTSDEFPHS
NGEIEDVQTDIPSEPWNTGHDWILADKRPTAWFLPSIRAANVMAASLRKAGKSVVVLNR
KTFEREYPTIKQKKPDFILATDIAEMGANLCVERVLDCRTAFKPVLVDEGRKVAIKGPL
RISASSAAQRRGRIGRNPNRDGDSYYYSEPTSENNAHHVCWLEASMLLDNMEVRGGMVA
PLYGVEGTKTPVSPGEMRLRDDQRKVFRELVRNCDLPVWLSWQVAKAGLKTNDRKWCFE
GPEEHEILNDSGETVKCRAPGGAKKPLRPRWCDERVSSDQSALSEFIKFAEGRRGAAEV
LVVLSELPDFLAKKGGEAMDTISVFLHSEEGSRAYRNALSMMPEAMTIVMLFILAGLLT

SGMVIFFMSPKGISRMSMAMGTMAGCGYLMFLGGVKPTHISYVMLIFFVLMVVVIPEPG
QQRSIQDNQVAYLIIGILTLVSAVAANELGMLEKTKEDLFGKKNLIPSSASPWSWPDLD
LKPGAAWTVYVGIVTMLSPMLHHWIKVEYGNLSLSGIAQSASVLSFMDKGIPFMKMNIS
VIMLLVSGWNSITVMPLLCGIGCAMLHWSLILPGIKAQQSKLAQRRVFHGVAKNPVVDG
NPTVDIEEAPEMPALYEKKLALYLLLALSLASVAMCRTPFSLAEGIVLASAALGPLIEG
NTSLLWNGPMAVSMTGVMRGNHYAFVGVMYNLWKMKTGRRGSANGKTLGEVWKRELNLL
DKRQFELYKRTDIVEVDRDTARRHLAEGKVDTGVAVSRGTAKLRWFHERGYVKLEGRVI
DLGCGRGGWCYYAAAQKEVSGVKGFTLGRDGHEKPMNVQSLGWNIITFKDKTDIHRLEP
VKCDTLLCDIGESSSSSVTEGERTVRVLDTVEKWLACGVDNFCVKVLAPYMPDVLEKLE
LLQRRFGGTVIRNPLSRNSTHEMYYVSGARSNVTFTVNQTSRLLMRRMRRPTGKVTLEA
DVILPIGTRSVETDKGPLDKEAIEERVERIKSEYMTSWFYDNDNPYRTWHYCGSYVTKT
SGSAASMVNGVIKILTYPWDRIEEVTRMAMTDTTPFGQQRVFKEKVDTRAKDPPAGTRK
IMKVVNRWLFRHLAREKSPRLCTKEEFIAKVRSHAAIGAYLEEQEQWKTANEAVQDPKF
WELVDEERKLHQQGRCRTCVYNMMGKREKKLSEFGKAKGSRAIWYMWLGARYLEFEALG
FLNEDHWASRENSGGGVEGIGLQYLGYVIRDLAAMDGGGFYADDTAGWDTRITEADLDD
EQEILNYMSPHHKKLAQAVMEMTYKNKVVKVLRPAPGGKAYMDVISRRDQRGSGQVVTY
ALNTITNLKVQLIRMAEAEMVIHHQHVQDCDESVLTRLEAWLTEHGCNRLKRMAVSGDD
CVVRPIDDRFGLALSHLNAMSKVRKDISEWQPSKGWNDWENVPFCSHHFHELQLKDGRR
IVVPCREQDELIGRGRVSPGNGWMIKETACLSKAYANMWSLMYFHKRDMRLLSLAVSSA
VPTSWVPQGRTTWSIHGKGEWMTTEDMLEVWNRVWITNNPHMQDKTMVKKWRDVPYLTK
RQDKLCGSLIGMTNRATWASHIHLVIHRIRTLIGQEKYTDYLTVMDRYSVDADLQLGEL

Other appropriate sequences to prepare the recombinant yellow fever viruses contemplated herein include corresponding sequences in HIV (for example, complete Gag polypeptide or a fragment thereof). For example, the heterologous polypeptide expressed by the recombinant yellow fever viruses considered here may comprise a fragment of **SEQ. ID. NO.: 9,** or a related protein (for example, a protein having at least about 50%, 60%, 70%, 80%, 90%, 95%, 96%, 97%, 98%, or 99% of amino acid sequence identity with SEQ. ID. NO.: 9).
SED.[sic] ID. NO.: 9

MGARASVLSG GKLDKWEKIR LRPGGKKTYQ LKHIVWASRE LERFAVNPGL LETGGGCKQI

LVQLQPSLQT GSEELKSLYN AVATLYCVHQ GIEVRDTKEA LDKIEEEQNK SKKKAQQAAA

DTGNSSQVSQ NYPIVQNLQG QMVHQAISPR TLNAWVKVIE EKAFSPEVIP MFSALSEGAT

PQDLNTMLNT VGGHQAAMQM LKETINEEAA EWDRLHPAHA GPNAPGQMRE PRGSDIAGTT

STLQEQIGWM TSNPPVPVGE IYKRWIILGL NKIVRMYSPV SILDIRQGPK EPFRDYVDRF

YKTLRAEQAS QDVKNWMTET LLVQNANPDC KTILKALGPA ATLEEMMTAC QGVGGPSHKA

RILAEAMSQV TSPANIMMQR GNFRNQRKTI KCFNCGKEGH LARHCRAPRK KGCWKCGREG

HQMKDCTERQ ANFLGKIWPS HKGRPGNFLQ SRPEPTAPPE ESFRFGEETT TPPQKQEPLP

SQKQETIDKD LYPLASLKSL FGNDPSLQ  formatting

A fragment, as considered here, may include at least 8, 9, 10, 20, 30, 40, 50 [sic] 100, or 200 contiguous amino acids of SEQ. ID. NO.: 9. In one embodiment, the fragment comprises amino acids 45-269 of SEQ. ID. NO.: 9. The heterologous polypeptide may still comprise a terminal amino acid sequence comprising KESSIG.

### EXAMPLES

The present invention will now be described in detail by the examples presented below. It is worth emphasizing that the invention is not limited to these examples, and also includes variations and modifications within the limits allowing its action.

### Example 1: Proliferation properties of recombinant rYF17D/SIVGag45-269 in Vero cells

The growth capacity of recombinant virus rYF17D/SIVGag45-269 was assessed and compared with other two viruses, YF 17DD and YF17D/G1/2 T3 vaccine (the latter corresponding to the parental vector virus, that is, heterologous insertion). Three independent experiments of virus growth in monolayers of Vero cells were performed. All experiments were performed in low MOI of 0.02 with densities of Vero cells of 62,500 cells per cm².

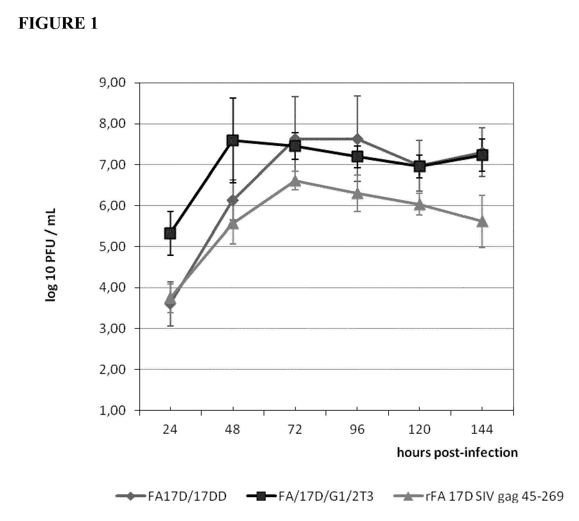

The recombinant virus rYF17D/SIVGag45-269 presented a lower growth rate compared with the viruses of 17D vaccine, and had its peak at 72 hours after the infection, exhibiting a titer of 6.61 ± 0.23 log10 PFU/mL. **Figure 1** shows the viral growth curves in Vero cells. Cells were infected with YF 17D control (gray lozenges), YF17D/G1/2T3 virus (black squares), or with the recombinant virus rYF17D/SIVGag45-269 (gray triangles in MOI of 0.02). Each time point represents the mean titer obtained for these three experiments separately, with the respective standard deviations.
**Table 8** below shows the viral growth curves in Vero cells. Viral titers (log10 PFU/mL) are shown in each time point. Viral growth peaks are shown in bold.

**TABLE 8**

| Virus | 24 h | 48 h | 72 h | 96 h | 120 h | 144 h |
|---|---|---|---|---|---|---|
| YF17D/17DD | 3.60 ± 0.54 | 6.14 ± 0.49 | **7.62 ± 1.02** | 7.63 ± 1.03 | 6.97 ± 0.62 | 7.30 ± 0.59 |
| YF/7D/G1/2T3 | 5.32 ± 0.53 | **7.60 ± 1.03** | 7.45 ± 0.33 | 7.19 ± 0.27 | 6.95 ± 0.27 | 7.23 ± 0.40 |
| rYF 17D SIV Gag 45-269 | 3.74 ± 0.35 | 5.57 ± 0.51 | **6.61 ± 0.23** | 6.30 ± 0.45 | 6.04 ± 0.27 | 5.61 ± 0.64 |

### Example 2 - Preliminary immunological studies of the YF17D vaccine virus infection in Mamu-A*01-positive rhesus monkeys

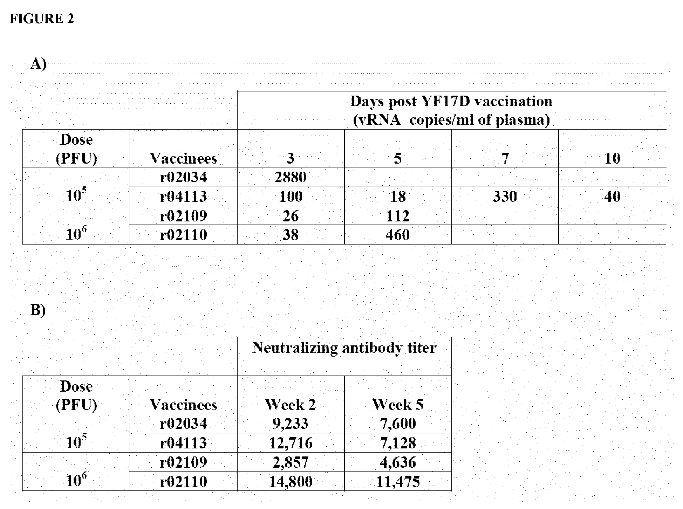

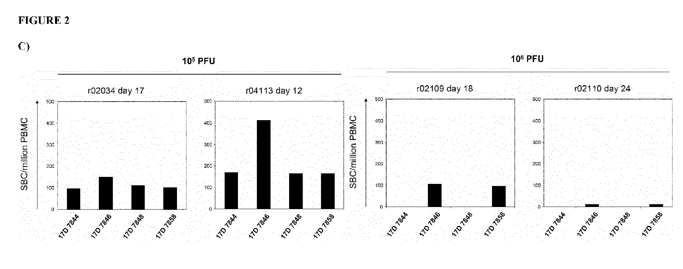

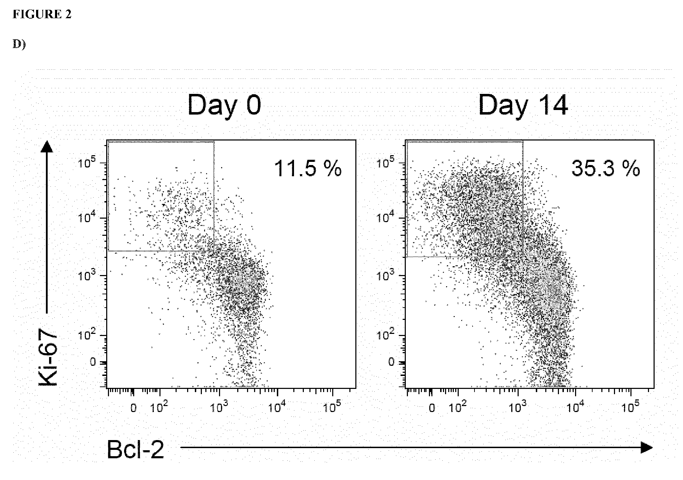

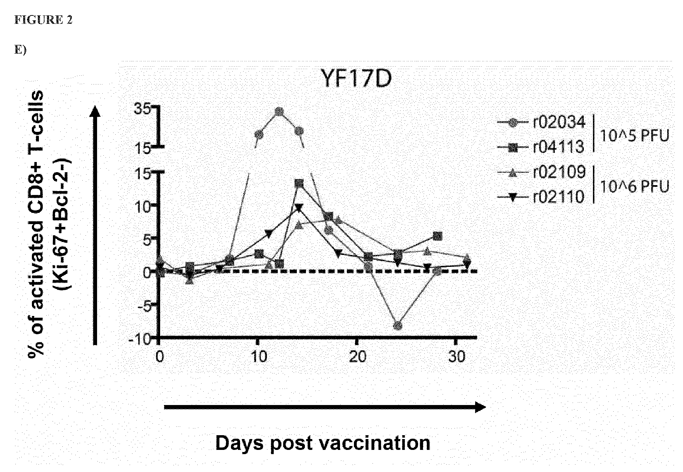

First, the YF17D vaccine virus was used to infect four Mamu-A*01-positive monkeys. The vaccine virus replicated in these four animals and induced neutralizing antibodies in all four monkeys by two weeks after vaccination (Figs. 2A and 2B). To monitor the CD8+ T-cell immune response against YF17D, its proteome was scanned for peptides that might bind to Mamu-A*01 using algorithms (MHC Pathway) [14]. The 52 YF17D-derived peptides which were most likely to bind to Mamu-A*01, based on their predicted affinity for this MHC class I molecule, were synthesized. IFNy ELISPOT assay was used to screen these peptides in YF17D-infected animals, and four Mamu-A*01-binding peptides: 17D 7844 (LTPVTMAEV; LV91285-1293), 17D 7846 (VSPGNGWMI; VI93250-3258), 17D 7850 (MSPKGISRM; MM92179-2187), and 17D 7858 (TTPFGQQRVF; TF102853-2863) were recognized *in vivo* (**Fig. 2C**). Using a previously reported protocol [15], the activation of CD8+ T cells was also observed in all four animals (**Figs. 2D** **and** **2E**). Thus, as previously observed, the YF17D vaccine virus replicates in Indian rhesus monkeys [16] and induces neutralizing antibodies, yellow fever-specific Mamu-A*01-restricted CD8+ T-cell responses, and CD8+ T-cell activation. **Figure 2** shows that YF17D replicates and induces neutralizing antibodies, virus-specific CD8+ T cells, and CD8+ T-cell activation in rhesus monkeys. **Figure 2A** shows the replication of YF17D during the first 10 days after vaccination with two different doses, as measured by Q-PCR using YF17D-specific primers. **Figure 2B** shows the titer of neutralizing antibodies determined at 2 and 5 weeks after YF17D vaccination. In **Figure 2C****,** the fresh PBMC from vaccinees (100,000 cells/well) were used in IFN-y ELISPOT assays [17] to assess T-cell responses against YF17D. Four epitopes (17D 7844, 17D 7846, 17D 7850, and 17D 7858) - predicted to bind to Mamu-A*01 as defined by the algorithm (MHC Pathway) - were selected for further studies. **Figure 2D** presents the identification of activated CD8+ T cells after vaccination with YF17D based on the expression of proliferation and proapoptotic markers Ki-67 and Bcl-2 [15]. Whole blood cells were stained with antibodies against CD3 and CD8, and then permeabilized, and subsequently labeled with Bcl-2- and Ki-67-specific antibodies. The flow cytometry graphs were gated on CD3+ and CD8+ lymphocytes. **Figure E** reveals the expression kinetics of Ki-67 and Bcl-2 in T cells after vaccination with YF17D.

### Example 3 - Immunogenicity of rYF17D/SIVGag45-269 in rhesus monkeys

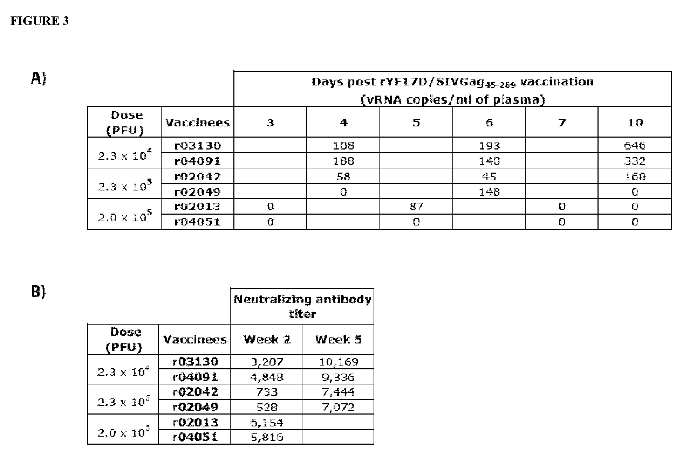

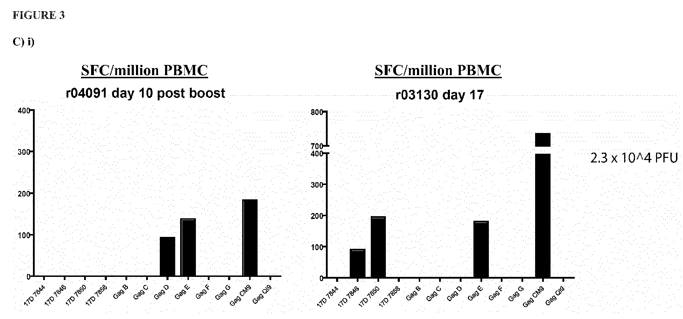

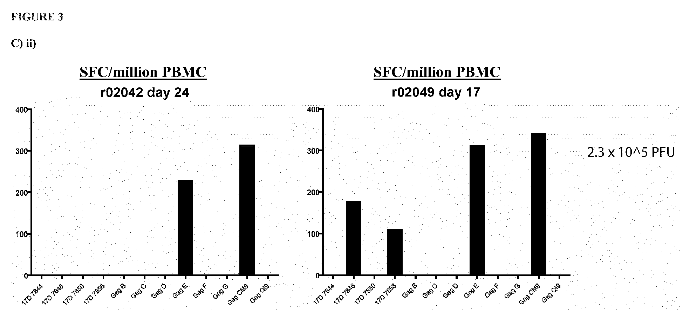

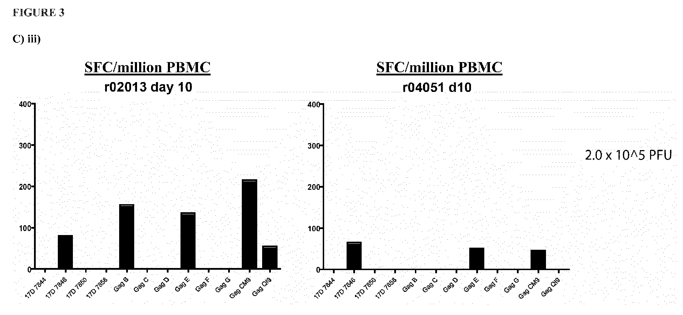

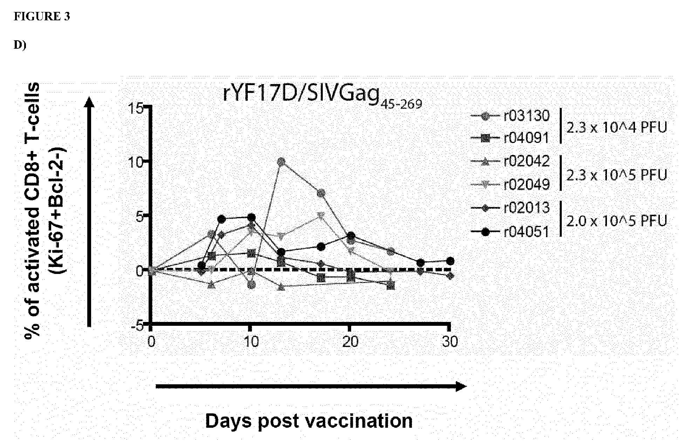

In the following step, the YF17D vaccine virus was engineered to express amino acids 45-269 of SIVmac239Gag (rYF17D/SIVGag45-269) by inserting a yellow fever codon-optimized sequence between the genes encoding the viral proteins E and NS1. This recombinant virus replicated and induced neutralizing antibodies in mice (data not shown). The rYF17D/SIVGag45-269 construct was then tested in six Mamu-A*01-positive Indian rhesus monkeys. Evidence for the viral replication of rYF17D/SIVGag45-269 was found for five of these six monkeys **(****Fig. 3A****).** Neutralizing antibodies were evident at two weeks after vaccination **(****Fig. 3****B.** [sic] A single immunization with rYF17D/SIVGag45-269 induced antigen-specific CD8+ T cells in five out of the six Mamu-A*01-positive monkeys **(****Fig. 3C****).** This recombinant virus also induced CD8+ T-cell activation in the majority of the vaccinated animals **(****Fig. 3D****).** The sixth monkey (r04091) required a booster dose 28 days after the first immunization to induce specific CD8+ T cells **(data not shown and** **Fig. 3C****).** No differences were found in vaccine-induced immune responses between the animals vaccinated with YF17D and those vaccinated with rYF17D/SIVGag45-269. There was, however, considerable animal-to-animal variability. Animal r02034, vaccinated with YF17D, exhibited massive CD8+ T-cell activation (a peak of 35% at day 12; **Fig. 2E****),** which was probably induced by the high levels of viral replication (16,800 copies/mL at day 5; **Fig. 2A****).** The comparison of neutralizing antibody responses at 2 and 5 weeks after vaccination in animals vaccinated with YF17D or rYF17D/SIVGag45-269 did not reveal detectable differences regarding the capacity of these two vaccines to induce neutralizing antibodies **(****Figs. 2B** **and** **3B****).** It was also difficult to detect differences in YF17D-specific CD8+ T-cell responses induced by these two vaccines. Peak Mamu-A*01-restricted CD8+ T-cell responses against YF17D ranged from barely detectable (r02110 at day 24; **Fig. 2C****)** to 400 SFCs/10⁶ PBMC (r04113 at day 12; **Fig. 2C****).** Two of the animals that were vaccinated with rYF17D/SIVGag45-269 did not originate CD8+ T-cell responses against the Mamu-A*01-bound YF17D peptides, whereas this kind of response was observed in four animals **(****Fig. 3C****),** with SFCs/10⁶ PBMC ranging from 50 to 200. For almost every animal vaccinated with rYF17D/SIVGag45-269, the Gag CM9-specific responses (peak in 50-750) were higher than those generated against the Mamu-A*01-restricted YF17D epitopes (peak in 0-200), suggesting that the recombinant virus replicated in a stable manner *in vivo.* Thus, the recombinant YF17D virus replicated and induced both virus-specific neutralizing antibodies and CD8+ T cells that were not demonstrably different from those induced by YF17D alone.

**Figure 3** shows that rYF17D/SIVGag45-269 replicates and induces neutralizing antibodies, virus-specific CD8+ T cells, and CD8+ T-cell activation in rhesus monkeys. **Figure 3A** shows the replication of rYF17D/SIVGag45-269 during the first 10 days after vaccination with two different doses, as measured by Q-PCR using YF17D-specific primers. **Figure 3B** shows the titer of neutralizing antibodies determined at 2 and 5 weeks after vaccination with rYF17D/SIVGag45-269. In **Figure 3C****,** fresh PBMC from vaccinees (100,00[sic] cells/well) were used in ELISPOT-IFN-γ assays to assess T-cell responses against the YF17D vector and SIV Gag (45-269) insert. YF17D-specific responses were measured using the same epitopes described in **Figure 2****.** For SIV Gag-specific responses, 6 sets of peptides of 15 mer and with 11 mer overlapping were used, covering the whole extension of Gag 45-269 insert of SIVmac239. In addition, Mamu-A*01-restricted responses against the dominant GagCM9181-189 and subdominant GagQI9254-262 epitopes were measured. The peak of CD8+ T-cell responses in r04091 is depicted at 10 days after the second administration of rYF17D/SIVGag (2.0 x 10⁵ PFU), which took place on day 28 after the initial vaccination. **Figure 3D** shows the expression kinetics of Ki-67 and Bcl-2 in CD8+ T cells after vaccination with rYF17D/SIVGag45-269, as described in **Figure 2****.**

### Example 4 - Immunization of rhesus monkeys with an initial dose (prime) of recombinant Mycobacterium bovis BCG followed by a booster dose ("boost") of rYF17D/SIVGag45-269 virus

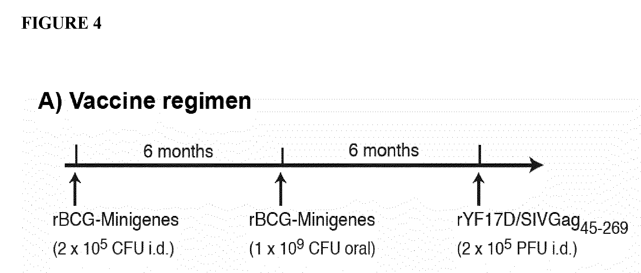

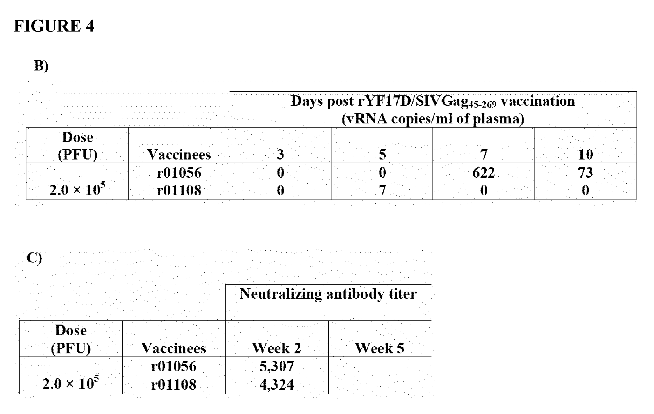

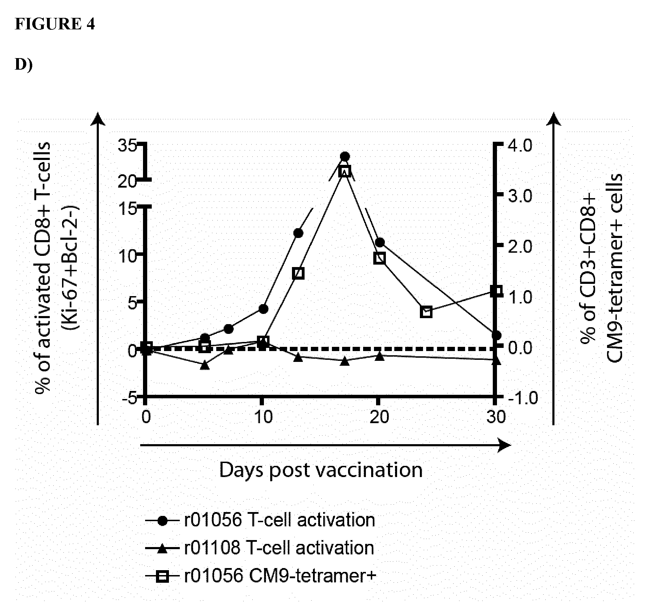

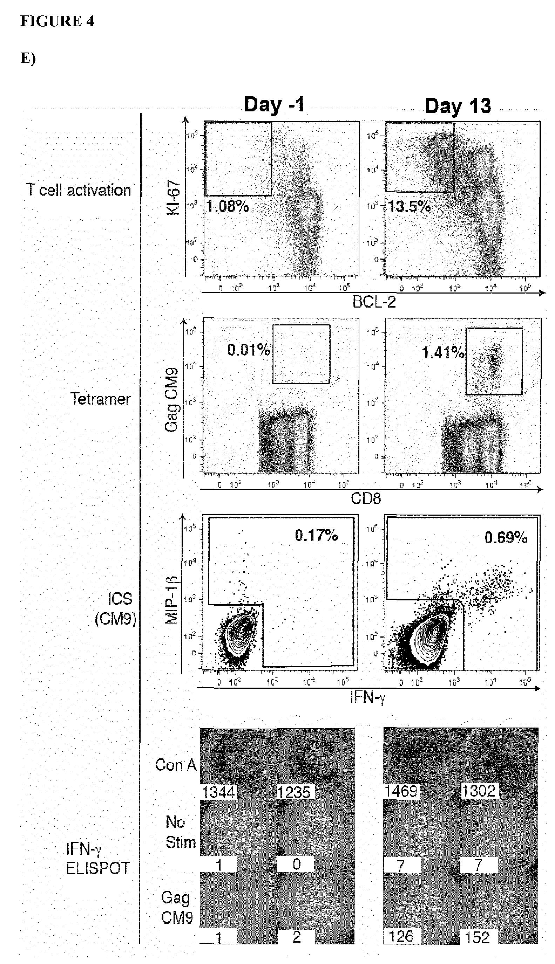

Most viral vectors are usually more efficient after an initial dose [prime] with DNA or rBCG [18, 19, 20, 21]. Thus, two monkeys that had been primed with rBCG expressing SIV proteins **(****Fig. 4A****)** were given a booster dose. No SIV-specific response was detected after either of the two priming rBCG vaccinations. Unfortunately, only a low level of rYF17D/SIVGag45-269 replication was observed at day 5 after vaccination in one of the rBCG-primed animals (r01108, 7 copies/mL; **Fig. 4B****).** However, both animals generated neutralizing antibodies at 2 weeks after vaccination **(****Fig. 4C****).** Fortunately, high-frequency CD8+ T-cell responses were detected in the Mamu-A*01-positive monkey (r01056) after a booster dose with rYF17D/SIVGag45-269 **(****Figs. 4D** **and** **4E****).** The booster dose induced massive activation of CD8+ T cells in the Mamu-A*01-positive animal r01056, peaking at 35% at 17 days after vaccination **(****Figs. 4D** **and** **4E****).** A frequency of 3.5% of the CD8+ T cells of this animal responded to the Mamu-A*01 GagCM9181-189 epitope at day 17 after vaccination **(****Figs. 4D** **and** **4E****).** These CD8+ T cells synthesized IFN γ, TNFα, MIP-1β and degranulated **(****Fig. 4E** **and data not shown).**

Thus, an rBCG prime followed by a recombinant yellow fever 17D boost induced polyfunctional antigen-specific CD8+ T cells.

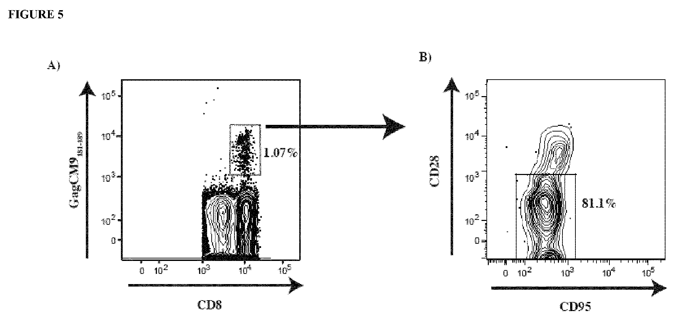

Vaccine-induced CD8+ T cells may be central memory T cells (TCM) or effector memory T cells (TEM). These two subsets of CD8+ T cells differ in function and surface markers [22]. Repeated boosting drives CD8+ T cells toward the TEM subset [22]. Consequently, we evaluated whether an initial dose of rBCG followed by a booster dose of rYF17D/SIVGag45-269 induced TCM or TEM CD8+ T cells. Staining revealed that the SIV-specific CD8+ T cells were largely TEM cells since most of them were CD28+ **(****Fig. 5A****).** It was recently suggested that TEM cells residing in mucosas can effectively control infection after a low-dose challenge with SIVmac239 [23].

After that, rYF17D/SIVGag45-269-induced CD8+ T cells were tested so as to determine if they could recognize virally infected CD8+ T cells. It was shown that CD8+ T cells stain for tetramers and produce cytokines after stimulation with synthetic peptides. Nevertheless, none of these assays determines whether these CD8+ T cells may recognize SIV-infected cells and reduce viral replication. Thus, a recently developed assay [24] was used to determine whether vaccine-induced CD8+ T cells can reduce viral replication in CD4+ T cells. Sorted tetramer(-) (GagCM9181-189) lymphocytes were incubated for 48 hours with SIVmac239-infected CD4+ T cells that expressed Mamu-A*01. The number of CD4+ T cells that expressed SIV Gag and the quantity of virus in the culture supernatant were assessed. Vaccine-induced CD8+ T cells reduced viral replication to the same extent as that seen with purified SIV-specific CD8+ T cells from three SIVmac239-infected rhesus monkeys, including an elite controller rhesus monkey 95061 **(****Fig. 5B****).**

Thus, in one aspect of the methods disclosed here, it is important that the rBCG has induced a high-frequency CD8+ T-cell response after a booster dose of rYF17D/SIVGag45-269. These CD8+ T cells reached frequencies that were similar to those induced by an initial dose of rBCG followed by a booster dose of Ad5 [19]. Even without the benefit of the initial dose of rBCG, the levels of CD8+ T cells induced by a single rYF17D/SIVGag45-269 vaccination were equivalent to those induced by our best SIV vaccine, SIVmac239ANef. Recombinant YF17D generated an average of 195 (range of 100-750) SFC/10 6 [sic] PBMC (N=6), whereas SIVmac239ANef induced an average of 238 (range of 150-320) SFC/10⁶ PBMC (N=3) [25]. It is also possible that any YF17D/HIV recombinants would likely replicate better in humans than in rhesus monkeys, thus inducing more robust immune responses. The rBCG was shown to be more effective in humans [26, 27, 28, 29] and may be more useful for inducing T-cell responses in humans than it has been in our limited study with rhesus monkeys.

**Figure 4** shows that vaccination with rYF17D/SIVGag45-269 induced a robust expansion of Gag-specific responses in an rBCG-primed monkey. **Figure 4A** shows the vaccination scheme. Two rhesus monkeys were immunized with rBCG intradermally (i.d.) (2x10⁵ CFU), rBCG orally (10⁹ CFU), and rYF17D/SIVGag45-269 subcutaneously (2x10⁵ PFU) at 6-month intervals. The rBCG was engineered to express 18 minigenes containing sequences of Gag, Vif, Nef, Ver, and Tat from SIVmac239. (See Tables 2-6, Proteins 1(a), 2(a), 3(a), 4(a), 6(a), 8(a), 12(a), 13(a), 14(a), 15(a), 17(a), 18(a), 19(a), 20(a), 21(a), 22(a), 23(a), e 24(a)). **Figure 4B** shows the replication of rYF17D/SIVGag45-269 during the first 10 days after vaccination, as measured by Q-PCR using YF17D-specific primers. **Figure 4C** shows the titer of neutralizing antibodies determined at 2 and 5 weeks after vaccination with rYF17D/SIVGag45-269. **Figure 4D** shows the kinetics of CD8+ T-cell activation (as described in **Figure 1****)** and expansion of CM9181-189-specific CD8+ T cells in r01056 after vaccination with rYF17D/SIVGag45-269. In Figure 4E, it can be seen that vaccination with rYF17D/SIVGag45-269 induces robust CD8+ T-cell responses against GagCM9181-189 in r01056. CD8+ T-cell activation (Ki-67+/Bcl-2-[sic]) for baseline and day 13 are shown. GagCM9181-189-specific responses were measured by tetramer staining, intracellular cytokine staining with antibodies against MIP-1β and IFN-γ, and ELISPOT-IFN-γ.

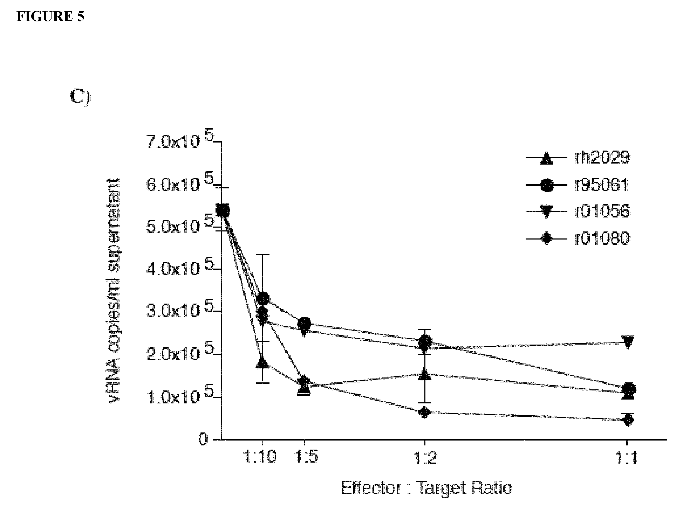

**Figure 5** illustrates that rYF17D/SIVGag45-269 vaccination of r01056 provides effector memory GagCM9181-189-specific CD8+ T cells that suppresses viral replication in CD4+ targets. **Figure 5A** shows the frequency of tetramer-positive GagCM9181-189-specific CD8+ T cells in r01056 gated on CD3+ CD8+ lymphocytes. In **Figure 5B****,** CD28 and CD95 expression profiles of tetramer-positive cells show a polarized effector memory phenotype. **Figure 5C** shows that *ex vivo* GagCM9181-189-specific CD8+ T cells from r01056 inhibit viral replication from SIVmac239-infected CD4+ T cells. GagCM9181-189-specific CD8+ T cells of three SIV-infected Manu-A*01-positive animals and r01056 vaccinated with rYF17D/SIVGag45-269 were tested for their ability to suppress viral replication from SIV-infected CD4+ T cells [24].

Forty-eight hours after the incubation of various samples containing different ratios of SIV-infected CD4+ T cells and GagCM9191-189-specific CD8+ T cells, the supernatant of these samples was removed and measured for viral RNA copies per milliliter by Q-PCR. No suppression was observed when effectors were incubated with CD4+ targets from Manu-A*01-negative animals. Rh2021 was infected with SIVmac239 (viral load ∼ 10⁵ vRNA copies/mL) containing mutations in 8 Manu-B*08-restricted epitopes as part of another study [30]. R01080 was vaccinated with a DNA/Ad5 regimen expressing gag, Rev, Tat and Nef, and later infected with SIVmac239 (viral load ∼ 10³ vRNA copies/mL) [31]. R95061 was vaccinated with a DNA/MVA regimen containing Gag CM9181-189 and was later challenged with SIVmac239 (undetectable viral load) [32].

From the facts exposed above, one may conclude that the attenuated yellow fever vaccine virus 17D expressing SIVmac239 Gag, as described herein, provided excellent immunization responses.

The material above is illustrative of the present invention, and is not intended to limit the scope of the claimed subject matter. The invention is defined by the claims below.

### REFERÊNCIAS

**[1]** - Burton, D. R., R. C. Desrosiers, R. W. Doms, W. C. Koff, P. D. Kwong, J. P. Moore, G. J. Nabel, J. Sodroski, I. A. Wilson, and R. T. Wyatt, 2004. HIV vaccine design and the neutralizing antibody problem. Nat Immunol 5:233-236.
**[2]** - Watkins, D. I., D. R. Burton, E. G. Kallas, J. P. Moore, and W. C. Koff. 2008. Nonhuman primate models and the failure of Merck HIV-1 vaccine in humans. Nat med 14:617-621.
**[3]** - Buchbinder, S. P., D. V. Mehrotra, A. Duerr, D. W. Fitzgerald, R. Mogg, D. Li, P. B. Gilbert, J. R. Lama, M. Marmor, C. Del Rio, M. J. McElrath, D. R. Casimiro, K. M. Gottesdiener, J. A. Chodakewitz, L. Corey, and M. N. Robertson.. 2008. Efficacy assessment of a cell-mediated immunity HIV-1 vaccine (the Step Study): a double-blind, randomised, placebo-controlled, test-of-concept trial. Lancet 372:1881-1893.
**[4]** - McElrath M. J., S. C. De Rosa, Z. Moodie, S. Bubey, L. Kierstead, H. Janes, O. D. Defawe, D. K. Carter, J. Hural, R. Akondy, S. P. Buchbinder, M. N. Robertson, D. V. Mehrotra, S. G. Self, L. Corey, J. W. Shiver, and D. R. Casimiro. 2008. HIV-1 vaccine-induced immunity in the test-of-concept Step Study: a case-cohort analysis. Lancet 372:1894-1905.
**[5]** - Koup, R. A., J. T. Safrit, Y. Cao, C. A. Andrews, G. McLeod, W. Borkowsky, C. Farthing, and D. D. Ho. Temporal association of cellular immune responses with the initial control of viremia in primary human immunodeficiency virus type 1 syndrome. 1994. J. Virol. 68:4650-4655.
**[6]** - Friedrich, T. C., L. E. Valentine, L. J. Yant, E. G. Rakasz, S. M. Piaskowski, J. R. Furlott, K. L. Weisgrau, B. Burwitz, G. E. May, E. J. Leon, T. Soma, G. Napoe, S. V. R. Capuano, N. A. Wilson, and D. I. Watkins. 2007. Subdominant CD8+ T-cell responses are involved in durable control of AIDS virus replication. J. Virol. 81:3465-3476.25.
**[7]** - Loffredo, J. T., E. G. Rakasz, J. P. Giraldo, S. P. Spencer, K. K. Grafton, S. R. Martin, G. Napoé, L. J. Yant, N. A. Wilson, and D. I. Watkins. 2005. Tat28-35SL8-specific CD8+ T lymphocytes are more effective than Gag181-189CM9-specific CD8+ T lymphocytes at suppressing simian immunodeficiency virus replication in a functional in vitro assay. J. Virol. 79: 14986-14991).
**[8]** - Matano, T., M. Kobayashi, H. Igarashi, A. Takeda, H. Nakamura, M. Kano,C. Sugimoto, K. Mori, A. Iida, T. Hirata, M. Hasegawa, T. Yuasa, M. Miyazawa, Y. Takahashi, M. Yasunami, A. Kimura, D. H. O'Connor, D. I. Watkins, and Y. Nagai. 2004. Cytotoxic T lymphocyte-based control of simian immunodeficiency virus in a preclinical AIDS vaccine trial. J. Exp. Med. 199:1709-1718.
**[9]** - Poland, J.D., Calisher, C.H., Monath, T.P., Downs, W.G. & Murphy, K. Persistence of neutralizing antibody 30-35 years after immunization with 17D yellow fever vaccine. Bull. World Health Organ. 1981, 59, 895-900;
**[10]** - Monath, T.P. Yellow fever vaccine. In Vaccines (Ed. Plotkin, S.A.O., W.A.) W.B. Saunders, Philadelphia, 2004. 1095-1176.
**[11]** - Bontrop RE, Watkins DI. MHC polymorphism: AIDS susceptibility in non-human primates. Trends Immunol. 2005. 26:227-233.
**[12]** - Valentine LE, Watkins DI. Relevance of studying T cell responses in SIV-infected rhesus macaques. Trends Microbiol. 2008. 16: 605-611.
**[13]** - Sacha JB, Chung C, Rakasz EG, Spencer SP, Jonas AK, Bean AT, Lee W, Burwitz, BJ, Stephany JJ, Loffredo JT, Allison DB, Adnan S, Hoji A, Wilson NA, Friedrich,TC, Lifson JD, Yang OO, Watkins DI.2007. Gag-specific CD8+ T lymphocytes recognize infected cells before AIDS-virus integration and viral protein expression. J Immunol. 178:2746-2754.
**[14]** - Peters, B., H. H. Bui, J. Sidney, Z. Weng, J. T. Loffredo, D. I. Watkins, B. R. Mothe, and A. Sette. 2005. A computational resource for the prediction of peptide binding to Indian rhesus macaque MHC class I molecules. Vaccine 23:5212-5224.
**[15]** - Miller, J. D., R. G. van der Most, R. S. Akondy, J. T. Glidewell, S. Albott, D. Masopust, K. Murali-Krishna, P. L. Mahar, S. Edupuganti, S. Lalor, S. Germon, C. Del Rio, M. J. Mulligan, S. I. Staprans, J. D. Altman, M. B. Feinberg, and R. Ahmed. 2008. Human effector and memory CD8+ T cell responses to smallpox and yellow fever vaccines. Immunity 28:710-722.
**[16]** - Trindade, G. F., R. S. Marchevsky, A. M. Fillipis, R. M. Nogueira, M. C. Bonaldo, P. C. Acero, E. Caride, M. S. Freire, and R. Galler. 2008. Limited replication of yellow fever 17DD and 17D-Dengue recombinant viruses in rhesus monkeys. An Acad Bras Cienc 80:311-321.
**[17]** - Wilson, N. A., B. F. Keele, J. S. Reed, S. M. Piaskowski, C. E. MacNair, A. J. Bett, X. Liang, F. Wang, E. Thoryk, G. J. Heidecker, M. P. Citron, L. Huang, J. Lin, S. Vitelli, C. D. Ahn, M. Kaizu, N. J. Maness, M. R. Reynolds, T. C. Friedrich, J. T. Loffredo, E. G. Rakasz, S. Erickson, D. B. Allison, M. J. Piatak, J. D. Lifson, J. W. Shiver, D. R. Casimiro, G. M. Shaw, B. H. Hahn, and D. I. Watkins. 2009. Vaccine-induced cellular heterologous challenge. J Virol 83:6508-6521.
**[18]** - Allen, T. M., T. U. Vogel, D. H. Fuller, B. R. Mothe, S. Steffen, J. E. Boyson, T. Shipley, J. Fuller, T. Hanke, A. Sette, J. D. Altman, B. Moss, A. J. McMichael, and D. I. Watkins. 2000. Induction of AIDS virus-specific CTL activity in fresh, unstimulated peripheral blood lymphocytes from rhesus macaques vaccinated with a DNA prime/modified vaccinia virus Ankara boost regimen. J Immunol 164:4968-4978.
**[19]** - Cayabyab, M. J., B. Korioth-Schmitz, Y. Sun, A. Carville, H. Balachandran, A. Miura, K. R. Carlson, A. P. Buzby, B. F. Haynes, W. R. Jacobs, and N. L. Letvin. 2009. Recombinant Mycobacterium bovis BCG prime-recombinant adenovirus boost vaccination in rhesus monkeys elicits robust polyfunctional simian immunodeficiency virus-specific T-cell responses. J Virol 83:5505-5513;
**[20]** - Hanke, T., R. V. Samuel, T. J. Blanchard, V. C. Neumann, T. M. Allen, J. E. Boyson, S. A. Sharpe, N. Cook, G. L. Smith, D. I. Watkins, M. P. Cranage, and A. J. McMichael. 1999. Effective induction of simian immunodeficiency virus-specific cytotoxic T lymphocytes in macaques by using a multiepitope gene and DNA prime-modified vaccinia virus Ankara boost vaccination regimen. J Virol 73:7524-7532.
**[21]** - Horton, H., T. U. Vogel, D. K. Carter, K. Vielhuber, D. H. Fuller, T. Shipley, J. T. Fuller, K. J. Kunstman, G. Sutter, D. C. Montefiori, V. Erfle, R. C. Desrosiers, N. Wilson, L. J. Picker, S. M. Wolinsky, C. Wang, D. B. Allison, and D. I. Watkins. 2002. Immunization of rhesus macaques with a DNA prime/modified vaccinia virus Ankara boost regimen induces broad simian immunodeficiency virus(SIV)-specific T-cell responses and reduces initial viral replication but does not prevent disease progression following challenge with pathogenic SIVmac239. J Virol 76:7187-7202.
**[22]** - Masopust, D., S. J. Ha, V. Vezys, and R. Ahmed. 2006. Stimulation history dictates memory CD8 T cell phenotype: implications for prime-boost vaccination. J Immunol 177:831-839.
**[23]** - Hansen, S. G., C. Vieville, N. Whizin, L. Coyne-Johnson, D. C. Siess, D. D. Drummond, A. W. Legasse, M. K. Axthelm, K. Oswald, C. M. Trubey, M. J. Piatak, J. D. Lifson, J. A. Nelson, M. A. Jarvis, and L. J. Picker. 2009. Effector memory T cell responses are associated with protection of rhesus monkeys from mucosal simian immunodeficiency virus challenge. Nat Med 15:293-299.
**[24]** - Vojnov, L. J. Reed, K. L. Weisgrau, E. Rakasz, J. T. Loffredo, S. Piaskowski, J. B. Sacha, H. L. Kolar, N. A. Wilson, R. P. Johnson, and D. I. Watkins. Effective SIV-specific CD8+ T-cells lack an easily detectable, shared characteristic. J Virol Manuscript in press.
**[25]** - Reynolds, M R, A. M. Weiler, K. L. Weisgrau, S. M. Piaskowski, J. R. Furlott, J. T. Weinfurther, M Kaizu, T. Soma, E. J. Leon, C. MacNair, D. P. Leaman, M. B. Zwick, E. Gostick, S. K. Musani, D. A. Price, T. C. Friedrich, E. G. Rakasz, N. A. Wilson, A. B. McDermott, R. Boyle, D. B. Allison, D. R. Burton, W. C. Koff, and D. I. Watkins. 2008. Macaques vaccinated with live-attenuated SIV control replication of heterologous virus. J Exp Med 205:2537-2550.
**[26]** - Barker, L. F., M. J. Brennan, P. K. Rosenstein, and J. C. Sadoff. 2009. Tuberculosis vaccine research: the impact of immunology. Curr Opin Immunol 21:331-338.
**[27]** - Hoft, D. F., A. Blazevic, G. Abate, W. A. Hanekom, G. Kaplan, J. H. Soler, F. Weichold, L. Geiter, J. C. Sadoff, and M. A. Horwitz. 2008. A new recombinant bacille Calmette-Guerin vaccine safely induces significantly enhanced tuberculosis-specific immunity in human volunteers. J Infect Dis 198:1491-1501.
**[28]** - Skeiky, Y. A., and J. C. Sadoff. 2006. Advances in tuberculosis vaccine strategies. Nat Rev Microbiol 4:469-476
**[29]** - Stover, C. K., G. P. Bansal, S. Langerman, and M. S. Hanson. 1994. Protective immunity elicited by rBCG vaccines. Dev Biol Stand 82:163-170.
**[30]** - Valentine, L. E., J. T. Loffredo, A. T. Bean, E. J. Leon, C. E. Macnair, D. R. Beal, S. M. Piaskowski, Y. C. Klimentidis, S. M. Lank, R. W. Wiseman, J. T. Weinfurter, G. E. May, E. G. Rakasz, N. A. Wilson, T. C. Friedrich, D. H. O'Connor, D. B. Allison, and D. I. Watkins. 2009. Infection with "escaped" virus variants impairs control of SIVmac239 replication in Mamu-B*08+ macaques. J Virol., in press.
**[31]** - Wilson, N. A., J. Reed, G. S. Napoe, S. Piaskowski, A. Szymanski, J. Furlott, E. J Gonzalez, L. J. Yant, N. J. Maness, G. E. May, T. Soma, M. R. Reynolds, E. Rakasz, R. Rudersdorf, A. B. MacDermott, D. H. O'Connor, T. C. Friedrich, D. B. Alison, A. Patki, L. J. Picker, D. R. Burton, J. Lin, L. Huang, D. Patel, G. Heindecker, J. Fan, M. Citron, M. Horton, F. Wang, X. Liang, J. W. Shiver, D. R. Casimiro, and D. I. Watkins. 2006. Vaccine-induced cellular immune responses reduce plasma viral concentrations after repeated low-dose challenge with pathogenic simian immunodeficiency virus SIVmac239. J Virol 80:5875-5885.
**[32]** - Allen, T. M., P. Jing, B. Calore, H. Horton, D. H. O'Connor, T. Hanke, M. Piekarczyk, R. Ruddersdorf, B. R. Mothe, C. Emerson, N. Wilson, J. D. Lifson, I. M. Belyakov, J. A. Berzofsky, C. Wang, D. B. Allison, D. C. Montefiori, R. C. Desrosiers, S. Wolinsky, K. J. Kunstman, J. D. Altman, A. Sette, A. J. McMichael, and D. I. Watkins. 2002. Effects of cytotoxic T lymphocytes (CTL) directed against a single simian immunodeficiency virus(SIV) Gag CTL epitope on the course of SIVmac239 infection. J Virol 76:10507-10511.

## Claims

1. Pharmaceutical composition for infection caused by lentiviruses **characterized by** comprising:(A) a recombinant yellow fever virus which expresses a heterologous polypeptide comprising at least a fragment of a lentiviral polypeptide and,(B) a pharmaceutical carrier;
wherein the composition comprises an effective amount of a recombinant yellow fever virus to induce a therapeutic or protective immune response against infection with lentivirus.

2. Composition according to claim 1 **characterized in that** the heterologous polypeptide include at least a fragment of the HIV Gag polypeptide or at least a fragment of the polypeptide of SIV Gag.

3. Composition according to claim 1 **characterized in that** the heterologous polypeptide include at least a fragment of SEQ ID NO:9.

4. Composition according to claim 1 **characterized in that** the fragment comprises at least 8, 9, 10, 20, 30, 40, 50, 100 or 200 amino acids continuous of SEQ ID NO:9.

5. Composition according to claim 1 **characterized in that** the fragment comprises amino acids 45-269 of SEQ ID NO:9.

6. Composition according to claim 1 **characterized in that** the heterologous polypeptide comprises a sequence of amino acid C-terminal comprising KESSIG.

7. Composition according to claim 1 **characterized in that** the recombinant yellow fever virus is YF17D.

8. Composition according to claim 1 **characterized in that** the recombinant yellow fever virus comprises a sequence coding for the heterologous polypeptide inserted between the sequences coding for the E polypeptide and NS1 polypeptide.

9. Composition according to claim 1 **characterized in that** the recombinant yellow fever virus comprises a sequence coding for the heterologous polypeptide, said sequence being optimized according to codon usage frequency of the yellow fever virus.

10. Pharmaceutical composition for infection caused by lentiviruses **characterized by** comprising a mixture of recombinant yellow fever viruses, where the mixture comprises:(A) a first recombinant yellow fever virus which expresses a first heterologous polypeptide comprising at least a fragment of a lentiviral Gag polypeptide;(B) a second recombinant yellow fever virus expressing a second heterologous polypeptide comprising at least a fragment of a lentiviral Vif polypeptide; (C) a third recombinant yellow fever virus that expresses a third heterologous polypeptide comprising at least a fragment of a lentiviral Nef polypeptide and, (D) a pharmaceutical carrier,
wherein the composition comprises an effective amount of a mixture to induce a protective or therapeutic immune response against infection lentiviral.

11. Composition according to claim 10 **characterized in that** the Gag polypeptide is a Gag polypeptide of HIV; the Vif polypeptide is the Vif polypeptide of HIV; and, the Nef polypeptide is the Nef polypeptide of HIV.

12. Method for inducing a protective or therapeutic immune response against HIV **characterized in that** the method comprises administering the composition defined in any one of claims 1 to 11 to an individual who needs it.

13. Method according to claim 12 **characterized by** further comprises administering an initial dose of the composition ("priming") prior to administering the composition of any one of claims 7-18, where the initial dose comprises a DNA encoding one or more of the HIV polypeptides or fragments thereof and/or the initial dosage of the composition comprises rBCG that expresses one or more of the polypeptides of HIV or fragments thereof.

14. Method according to claim 12 **characterized in that** the composition be administered in two or more subsequent times, waiting for at least 4-12 weeks before the subsequent administration.

15. Method according to claim 12 **characterized in that** the protective or therapeutic immune response against HIV includes a response of CD4+ T cell and / or a response in CD8+ Tcell.

16. Vaccine to induce a protective immune response or a therapeutic response against HIV **characterized in that** said vaccine comprises: (A) a recombinant yellow fever virus which expresses a heterologous polypeptide comprising at least a fragment of a lentiviral polypeptide and, (B) a pharmaceutical carrier;
wherein the composition comprises an effective amount of a recombinant yellow fever virus to induce a therapeutic or protective immune response against infection with lentivirus.

17. Vaccine to induce a protective immune response or therapeutic response against HIV **characterized in that** said vaccine comprises a mixture of recombinant yellow fever virus, said mixture comprising: (A) a first recombinant yellow fever virus which expresses a first heterologous polypeptide comprising at least a fragment of a lentiviral Gag polypeptide; (B) a second recombinant yellow fever virus expressing a second heterologous polypeptide comprising at least a fragment of a lentiviral Vif polypeptide; (C) a third recombinant yellow fever virus that expresses a third heterologous polypeptide comprising at least a fragment of a lentiviral Nef polypeptide; and,(D) a pharmaceutical carrier.

18. 45-269 fragment of the Gag protein used for drawing a heterologous cassette of SIV **characterized in that** the fragment comprises SEQ ID NO:1.

19. Recombinant protein of 45-269 amino acids from the Gag protein **characterized by** comprising SEQ ID NO:2.

20. Recombinant gene of Gag 45-269 protein of SIV **characterized in that** it comprises SEQ ID NO:6.

21. Nucleotide sequence of recombinant yellow fever virus which expresses Gag 45-269 protein **characterized in that** it comprises SEQ ID NO:7.

22. Precursor polyprotein containing the recombinant protein of amino acids 45-269 from the SIV Gag protein **characterized by** comprising SEQ ID NO:8.

23. Method for producing lentivirus proteins or related polypeptides **characterized in that** said method comprises the steps of: (A) introducing at least one vector or a vector system in the host cell, said vector or vector system including a nucleic acid sequence encoding at least an immunogenic protein of lentivirus or the related polypeptide, a nucleic acid sequence having at least 90% identity with sequence SED ID NO:6 or SEQ ID NO:7 or the encoded protein having at least 90% identity with the protein defined by SEQ ID NOs:1, 2, 8 or 9 (or at least 95 %, 96%, 97% or 99% identity with the sequences referred to); (B) expressing the nucleic acid sequence into the host cell and, (C) producing the antigenic protein encoded by lentivirus or related polypeptide within the host cell.

24. Method according to claim 23 **characterized in that** it comprises introducing two or more vectors or vector systems.

25. Method according to claim 23 **characterized in that** the vector or vector system include a nucleic acid sequence encoding two or more immunogenic proteins of lentiviruses or related polypeptides.


SEQUENCE LISTING

<110>  Fundação Oswaldo Cruz e outros

<120>  LENTIVIRUS VACCINE BASED ON THE RECOMBINANT YELLOW FEVER VACCINE
       VIRUS

<130>  P1880

<140>  PCT/BR2010/000430
<141>  2010-12-22

<150>  BR PI0914507-9
<151>  2009-12-23

<160>  9

<170>  PatentIn version 3.5

<210>  1
<211>  510
<212>  PRT
<213>  Simian immunodeficiency virus

<220>
<221>  misc_feature
<222>  (489)..(489)
<223>  Xaa can be any naturally occurring amino acid

<400>  1

Met Gly Ala Arg Asn Ser Val Leu Ser Gly Lys Lys Ala Asp Glu Leu
1               5                   10                  15

Glu Lys Ile Arg Leu Arg Pro Asn Gly Lys Lys Lys Tyr Met Leu Lys
            20                  25                  30

His Val Val Trp Ala Ala Asn Glu Leu Asp Arg Phe Gly Leu Ala Glu
        35                  40                  45

Ser Leu Leu Glu Asn Lys Glu Gly Cys Gln Lys Ile Leu Ser Val Leu
    50                  55                  60

Ala Pro Leu Val Pro Thr Gly Ser Glu Asn Leu Lys Ser Leu Tyr Asn
65                  70                  75                  80

Thr Val Cys Val Ile Trp Cys Ile His Ala Glu Glu Lys Val Lys His
                85                  90                  95

Thr Glu Glu Ala Lys Gln Ile Val Gln Arg His Leu Val Val Glu Thr
            100                 105                 110

Gly Thr Thr Glu Thr Met Pro Lys Thr Ser Arg Pro Thr Ala Pro Ser
        115                 120                 125

Ser Gly Arg Gly Gly Asn Tyr Pro Val Gln Gln Ile Gly Gly Asn Tyr

130                 135                 140

Val His Leu Pro Leu Ser Pro Arg Thr Leu Asn Ala Trp Val Lys Leu
145                 150                 155                 160

Ile Glu Glu Lys Lys Phe Gly Ala Glu Val Val Pro Gly Phe Gln Ala
                165                 170                 175

Leu Ser Glu Gly Cys Thr Pro Tyr Asp Ile Asn Gln Met Leu Asn Cys
            180                 185                 190

Val Gly Asp His Gln Ala Ala Met Gln Ile Ile Arg Asp Ile Ile Asn
        195                 200                 205

Glu Glu Ala Ala Asp Trp Asp Leu Gln His Pro Gln Pro Ala Pro Gln
    210                 215                 220

Gln Gly Gln Leu Arg Glu Pro Ser Gly Ser Asp Ile Ala Gly Thr Thr
225                 230                 235                 240

Ser Ser Val Asp Glu Gln Ile Gln Trp Met Tyr Arg Gln Gln Asn Pro
                245                 250                 255

Ile Pro Val Gly Asn Ile Tyr Arg Arg Trp Ile Gln Leu Gly Leu Gln
            260                 265                 270

Lys Cys Val Arg Met Tyr Asn Pro Thr Asn Ile Leu Asp Val Lys Gln
        275                 280                 285

Gly Pro Lys Glu Pro Phe Gln Ser Tyr Val Asp Arg Phe Tyr Lys Ser
    290                 295                 300

Leu Arg Ala Glu Gln Thr Asp Ala Ala Val Lys Asn Trp Met Thr Gln
305                 310                 315                 320

Thr Leu Leu Ile Gln Asn Ala Asn Pro Asp Cys Lys Leu Val Leu Lys
                325                 330                 335

Gly Leu Gly Val Asn Pro Thr Leu Glu Glu Met Leu Thr Ala Cys Gln
            340                 345                 350

Gly Val Gly Gly Pro Gly Gln Lys Ala Arg Leu Met Ala Glu Ala Leu
        355                 360                 365

Lys Glu Ala Leu Ala Pro Val Pro Ile Pro Phe Ala Ala Ala Gln Gln
    370                 375                 380

Arg Gly Pro Arg Lys Thr Ile Lys Cys Trp Asn Cys Gly Lys Glu Gly
385                 390                 395                 400

His Ser Ala Arg Gln Cys Arg Ala Pro Arg Arg Gln Gly Cys Trp Lys
                405                 410                 415

Cys Gly Lys Met Asp His Val Met Ala Lys Cys Pro Asp Arg Gln Ala
            420                 425                 430

Gly Phe Leu Gly Leu Gly Pro Trp Gly Lys Lys Pro Arg Asn Phe Pro
        435                 440                 445

Met Ala Gln Val His Gln Gly Leu Met Pro Thr Ala Pro Pro Glu Asp
    450                 455                 460

Pro Ala Val Asp Leu Leu Lys Asn Tyr Met Gln Leu Gly Lys Gln Gln
465                 470                 475                 480

Arg Glu Lys Gln Arg Glu Ser Arg Xaa Lys Pro Tyr Lys Glu Val Thr
                485                 490                 495

Glu Asp Leu Leu His Leu Asn Ser Leu Phe Gly Gly Asp Gln
            500                 505                 510

<210>  2
<211>  307
<212>  PRT
<213>  Simian immunodeficiency virus

<400>  2

Asp Gln Gly Ser Ala Ile Asn Phe Gly Arg Gly Leu Ala Glu Ser Leu
1               5                   10                  15

Leu Glu Asn Lys Glu Gly Cys Gln Lys Ile Leu Ser Val Leu Ala Pro
            20                  25                  30

Leu Val Pro Thr Gly Ser Glu Asn Leu Lys Ser Leu Tyr Asn Thr Val
        35                  40                  45

Cys Val Ile Trp Cys Ile His Ala Glu Glu Lys Val Lys His Thr Glu
    50                  55                  60

Glu Ala Lys Gln Ile Val Gln Arg His Leu Val Val Glu Thr Gly Thr
65                  70                  75                  80

Thr Glu Thr Met Pro Lys Thr Ser Arg Pro Thr Ala Pro Ser Ser Gly
                85                  90                  95

Arg Gly Gly Asn Tyr Pro Val Gln Gln Ile Gly Gly Asn Tyr Val His
            100                 105                 110

Leu Pro Leu Ser Pro Arg Thr Leu Asn Ala Trp Val Lys Leu Ile Glu
        115                 120                 125

Glu Lys Lys Phe Gly Ala Glu Val Val Pro Gly Phe Gln Ala Leu Ser
    130                 135                 140

Glu Gly Cys Thr Pro Tyr Asp Ile Asn Gln Met Leu Asn Cys Val Gly
145                 150                 155                 160

Asp His Gln Ala Ala Met Gln Ile Ile Arg Asp Ile Ile Asn Glu Glu
                165                 170                 175

Ala Ala Asp Trp Asp Leu Gln His Pro Gln Pro Ala Pro Gln Gln Gly
            180                 185                 190

Gln Leu Arg Glu Pro Ser Gly Ser Asp Ile Ala Gly Thr Thr Ser Ser
        195                 200                 205

Val Asp Glu Gln Ile Gln Trp Met Tyr Arg Gln Gln Asn Pro Ile Pro
    210                 215                 220

Val Gly Asn Ile Tyr Arg Arg Trp Ile Gln Leu Lys Glu Ser Ser Ile
225                 230                 235                 240

Gly Thr Ser Leu Gly Lys Ala Val His Gln Val Phe Gly Ser Val Tyr
                245                 250                 255

Thr Thr Met Phe Gly Gly Val Ser Trp Met Ile Arg Ile Leu Ile Gly
            260                 265                 270

Phe Leu Val Leu Trp Ile Gly Thr Asn Ser Arg Asn Thr Ser Met Ala
        275                 280                 285

Met Thr Cys Ile Ala Val Gly Gly Ile Thr Leu Phe Leu Gly Phe Thr
    290                 295                 300

Val Gly Ala
305

<210>  3
<211>  10
<212>  PRT
<213>  Yellow fever virus

<400>  3

Asp Gln Gly Ser Ala Ile Asn Phe Gly Arg
1               5                   10

<210>  4
<211>  6

<212>  PRT
<213>  Yellow fever virus

<400>  4

Lys Glu Ser Ser Ile Gly
1               5


<210>  5
<211>  66
<212>  PRT
<213>  Dengue virus type 4

<400>  5

Thr Ser Leu Gly Lys Ala Val His Gln Val Phe Gly Ser Val Tyr Thr
1               5                   10                  15


Thr Met Phe Gly Gly Val Ser Trp Met Ile Arg Ile Leu Ile Gly Phe
            20                  25                  30


Leu Val Leu Trp Ile Gly Thr Asn Ser Arg Asn Thr Ser Met Ala Met
        35                  40                  45


Thr Cys Ile Ala Val Gly Gly Ile Thr Leu Phe Leu Gly Phe Thr Val
    50                  55                  60


Gly Ala
65


<210>  6
<211>  924
<212>  DNA
<213>  Simian immunodeficiency virus

<400>  6
gatcaaggaa gcgccatcaa cttcggccga ggactggctg aatcactgct ggaaaacaaa      60

gaaggatgtc agaaaatcct gtcagtgctg gctccactgg tgccaacagg atcagaaaac     120

ctgaagtcac tgtacaacac agtgtgtgtg atctggtgta tccatgctga agaaaaagtg     180

aagcatacag aagaggccaa gcagatcgtg cagaggcatc tggtggtgga aacagggaca     240

acagaaacaa tgccaaagac atcaaggcca acagctccat catcaggaag ggggggaaac     300

tacccagtgc agcagatcgg agggaactac gtgcatctgc cactgtcacc aaggacactg     360

aacgcttggg tgaaactgat cgaagaaaag aaatttggag ctgaagtggt gccaggattt     420

caggctctgt cagaaggatg tacaccatac gacatcaacc agatgctgaa ctgtgtggga     480

gatcatcagg ctgctatgca gatcattagg gacatcatca acgaagaagc tgcagattgg     540

gatctgcaac atccacagcc agctccacag cagggacagc tgagggaacc atcaggatca     600

gacattgctg gaacaacatc atcagtggac gaacagatcc agtggatgta caggcagcag     660

aacccaatcc cagtgggaaa catctacaga agatggatcc agctgaaaga gggaagctca     720

ataggaacat cattgggaaa ggctgtgcac caggtttttg gaagtgtgta tacaaccatg 780

tttggaggag tctcatggat gattagaatc ctaattgggt tcttagtgtt gtggattggc 840

acgaactcca ggaacacttc aatggctatg acgtgcatag ctgttggagg aatcactctg 900

tttctgggct tcacagttgg cgcc 924

<210> 7
<211> 11785
<212> DNA
<213> Simian immunodeficiency virus

<400> 7
agtaaatcct gtgtgctaat tgaggtgcat tggtctgcaa atcgagttgc taggcaataa 60

acacatttgg attaatttta atcgttcgtt gagcgattag cagagaactg accagaacat 120

gtctggtcgt aaagctcagg gaaaaaccct gggcgtcaat atggtacgac gaggagttcg 180

ctccttgtca aacaaaataa aacaaaaaac aaaacaaatt ggaaacagac ctggaccttc 240

aagaggtgtt caaggattta tctttttctt tttgttcaac attttgactg gaaaaaagat 300

cacagcccac ctaaagaggt tgtggaaaat gctggaccca agacaaggct tggctgttct 360

aaggaaagtc aagagagtgg tggccagttt gatgagagga ttgtcctcaa ggaaacgccg 420

ttcccatgat gttctgactg tgcaattcct aattttggga atgctgttga tgaccggtgg 480

agtgaccttg gtgcggaaaa acagatggtt gctcctaaat gtgacatctg aggacctcgg 540

gaaaacattc tctgtgggca caggcaactg cacaacaaac attttggaag ccaagtactg 600

gtgcccagac tcaatggaat acaactgtcc caatctcagt ccaagagagg agccagatga 660

cattgattgc tggtgctatg gggtggaaaa cgttagagtc gcatatggta agtgtgactc 720

agcaggcagg tctaggaggt caagaagggc cattgacttg cctacgcatg aaaaccatgg 780

tttgaagacc cggcaagaaa aatggatgac tggaagaatg ggtgaaaggc aactccaaaa 840

gattgagaga tggttcgtga ggaacccctt ttttgcagtg acggctctga ccattgccta 900

ccttgtggga agcaacatga cgcaacgagt cgtgattgcc ctactggtct tggctgttgg 960

tccggcctac tcagctcact gcattggaat tactgacagg gatttcattg agggggtgca 1020

tggaggaact tgggtttcag ctaccctgga gcaagacaag tgtgtcactg ttatggcccc 1080

tgacaagcct tcattggaca tctcactaga gacagtagcc attgatagac ctgctgaggc 1140

gaggaaagtg tgttacaatg cagttctcac tcatgtgaag attaatgaca agtgccccag 1200

cactggagag gcccacctag ctgaagagaa cgaaggggac aatgcgtgca agcgcactta 1260

ttctgataga ggctggggca atggctgtgg cctatttggg aaagggagca ttgtggcatg 1320

cgccaaattc acttgtgcca aatccatgag tttgtttgag gttgatcaga ccaaaattca 1380

gtatgtcatc agagcacaat tgcatgtagg ggccaagcag gaaaattgga ataccagcat 1440

taagactctc aagtttgatg ccctgtcagg ctcccaggaa gtcgagttca ttgggtatgg 1500

aaaagctaca ctggaatgcc aggtgcaaac tgcggtggac tttggtaaca gttacatcgc 1560

tgagatggaa acagagagct ggatagtgga cagacagtgg gcccaggact tgaccctgcc 1620

atggcagagt ggaagtggcg gggtgtggag agagatgcat catcttgtcg aatttgaacc 1680

tccgcatgcc gccactatca gagtactggc cctgggaaac caggaaggct ccttgaaaac 1740

agctcttact ggcgcaatga gggttacaaa ggacacaaat gacaacaacc tttacaaact 1800

acatggtgga catgtttctt gcagagtgaa attgtcagct ttgacactca aggggacatc 1860

ctacaaaata tgcactgaca aaatgttttt tgtcaagaac ccaactgaca ctggccatgg 1920

cactgttgtg atgcaggtga aagtgtcaaa aggaaccccc tgcaggattc cagtgatagt 1980

agctgatgat cttacagcgg caatcaataa aggcattttg gttacagtta accccatcgc 2040

ctcaaccaat gatgatgaag tgctgattga ggtgaaccca ccttttggag acagctacat 2100

tatcgttggg agaggagatt cacgtctcac ttaccagtgg cacaaagagg gaagctcaat 2160

aggaaagttg ttcactcaga ccatgaaagg cgtggaacgc ctggccgtca tgggagacac 2220

cgcctgggat ttcagctccg ctggagggtt cttcacttcg gttgggaaag gaattcatac 2280

ggtgtttggc tctgcctttc aggggctatt tggcggcttg aactggataa caaaggtcat 2340

catgggggcg gtactcatat gggttggcat caacacaaga aacatgacaa tgtccatgag 2400

catgatcttg gtaggagtga tcatgatgtt tttgtctcta ggagttgggg cggatcaagg 2460

aagcgccatc aacttcggcc gaggactggc tgaatcactg ctggaaaaca aagaaggatg 2520

tcagaaaatc ctgtcagtgc tggctccact ggtgccaaca ggatcagaaa acctgaagtc 2580

actgtacaac acagtgtgtg tgatctggtg tatccatgct gaagaaaaag tgaagcatac 2640

agaagaggcc aagcagatcg tgcagaggca tctggtggtg gaaacaggga caacagaaac 2700

aatgccaaag acatcaaggc caacagctcc atcatcagga aggggggaaa actacccagt 2760

gcagcagatc ggagggaact acgtgcatct gccactgtca ccaaggacac tgaacgcttg 2820

ggtgaaactg atcgaagaaa agaaatttgg agctgaagtg gtgccaggat ttcaggctct 2880

gtcagaagga tgtacaccat acgacatcaa ccagatgctg aactgtgtgg gagatcatca 2940

ggctgctatg cagatcatta gggacatcat caacgaagaa gctgcagatt gggatctgca 3000

acatccacag ccagctccac agcagggaca gctgagggaa ccatcaggat cagacattgc 3060

tggaacaaca tcatcagtgg acgaacagat ccagtggatg tacaggcagc agaacccaat 3120

cccagtggga aacatctaca gaagatggat ccagctgaaa gagggaagct caataggaac 3180

atcattggga aaggctgtgc accaggtttt tggaagtgtg tatacaacca tgtttggagg 3240

agtctcatgg atgattagaa tcctaattgg gttcttagtg ttgtggattg gcacgaactc 3300

caggaacact tcaatggcta tgacgtgcat agctgttgga ggaatcactc tgtttctggg 3360

cttcacagtt ggcgccgatc aaggatgcgc catcaacttt ggcaagagag agctcaagtg 3420

cggagatggt atcttcatat ttagagactc tgatgactgg ctgaacaagt actcatacta 3480

tccagaagat cctgtgaagc ttgcatcaat agtgaaagcc tctttcgaag aagggaagtg 3540

tggcctaaat tcagttgact cccttgagca tgagatgtgg agaagcaggg cagatgagat 3600

taataccatt tttgaggaaa acgaggtgga catttctgtt gtcgtgcagg atccaaagaa 3660

tgtttaccag agaggaactc atccattttc cagaattcgg gatggtctgc agtatggttg 3720

gaagacttgg ggtaagaacc ttgtgttctc cccagggagg aagaatggaa gcttcatcat 3780

agatggaaag tccaggaaag aatgcccgtt ttcaaaccgg gtctggaatt ctttccagat 3840

agaggagttt gggacgggag tgttcaccac acgcgtgtac atggacgcag tctttgaata 3900

caccatagac tgcgatggat ctatcttggg tgcagcggtg aacggaaaaa agagtgccca 3960

tggctctcca acatttggga tgggaagtca tgaagtaaat gggacatgga tgatccacac 4020

cttggaggca ttagattaca aggagtgtga gtggccactg acacatacga ttggaacatc 4080

agttgaagag agtgaaatgt tcatgccgag atcaatcgga ggcccagtta gctctcacaa 4140

tcatatccct ggatacaagg ttcagacgaa cggaccttgg atgcaggtac cactagaagt 4200

gaagagagaa gcttgcccag ggactagcgt gatcattgat ggcaactgtg atggacgggg 4260

aaaatcaacc agatccacca cggatagcgg gaaagttatt cctgaatggt gttgccgctc 4320

ctgcacaatg ccgcctgtga gcttccatgg tagtgatggg tgttggtatc ccatggaaat 4380

taggccaagg aaaacgcatg aaagccatct ggtgcgctcc tgggttacag ctggagaaat 4440

acatgctgtc ccttttggtt tggtgagcat gatgatagca atggaagtgg tcctaaggaa 4500

aagacaggga ccaaagcaaa tgttggttgg aggagtagtg ctcttgggag caatgctggt 4560

cgggcaagta actctccttg atttgctgaa actcacagtg gctgtgggat tgcatttcca 4620

tgagatgaac aatggaggag acgccatgta tatggcgttg attgctgcct tttcaatcag 4680

accagggctg ctcatcggct ttgggctcag gaccctatgg agccctcggg aacgccttgt 4740

gctgacccta ggagcagcca tggtggagat tgccttgggt ggcgtgatgg gcggcctgtg 4800

gaagtatcta aatgcagttt ctctctgcat cctgacaata aatgctgttg cttctaggaa 4860

agcatcaaat accatcttgc ccctcatggc tctgttgaca cctgtcacta tggctgaggt 4920

gagacttgcc gcaatgttct tttgtgccat ggttatcata ggggtccttc accagaattt 4980

caaggacacc tccatgcaga agactatacc tctggtggcc ctcacactca catcttacct 5040

gggcttgaca caaccttttt tgggcctgtg tgcatttctg gcaacccgca tatttgggcg 5100

aaggagtatc ccagtgaatg aggcactcgc agcagctggt ctagtgggag tgctggcagg 5160

actggctttt caggagatgg agaacttcct tggtccgatt gcagttggag gactcctgat 5220

gatgctggtt agcgtggctg ggagggtgga tgggctagag ctcaagaagc ttggtgaagt 5280

ttcatgggaa gaggaggcgg agatcagcgg gagttccgcc cgctatgatg tggcactcag 5340

tgaacaaggg gagttcaagc tgctttctga agagaaagtg ccatgggacc aggttgtgat 5400

gacctcgctg gccttggttg gggctgccct ccatccattt gctcttctgc tggtccttgc 5460

tgggtggctg tttcatgtca ggggagctag gagaagtggg gatgtcttgt gggatattcc 5520

cactcctaag atcatcgagg aatgtgaaca tctggaggat gggatttatg gcatattcca 5580

gtcaaccttc ttgggggcct cccagcgagg agtgggagtg gcacagggag gggtgttcca 5640

cacaatgtgg catgtcacaa gaggagcttt ccttgtcagg aatggcaaga agttgattcc 5700

atcttgggct tcagtaaagg aagaccttgt cgcctatggt ggctcatgga agttggaagg 5760

cagatgggat ggagaggaag aggtccagtt gatcgcggct gttccaggaa agaacgtggt 5820

caacgtccag acaaaaccga gcttgttcaa agtgaggaat gggggagaaa tcggggctgt 5880

cgctcttgac tatccgagtg gcacttcagg atctcctatt gttaacagga acggagaggt 5940

gattgggctg tacggcaatg gcatccttgt cggtgacaac tccttcgtgt ccgccatatc 6000

ccagactgag gtgaaggaag aaggaaagga ggagctccaa gagatcccga caatgctaaa 6060

gaaaggaatg acaactgtcc ttgattttca tcctggagct gggaagacaa gacgtttcct 6120

cccacagatc ttggccgagt gcgcacggag acgcttgcgc actcttgtgt tggcccccac 6180

cagggttgtt ctttctgaaa tgaaggaggc ttttcacggc ctggacgtga aattccacac 6240

acaggctttt tccgctcacg gcagcgggag agaagtcatt gatgccatgt gccatgccac 6300

cctaacttac aggatgttgg aaccaactag ggttgttaac tgggaagtga tcattatgga 6360

tgaagcccat tttttggatc cagctagcat agccgctaga ggttgggcag cgcacagagc 6420

tagggcaaat gaaagtgcaa caatcttgat gacagccaca ccgcctggga ctagtgatga 6480

atttccacat tcaaatggtg aaatagaaga tgttcaaacg gacataccca gtgagccctg 6540

gaacacaggg catgactgga tcctggctga caaaaggccc acggcatggt tccttccatc 6600

catcagagct gcaaatgtca tggctgcctc tttgcgtaag gctggaaaga gtgtggtggt 6660

cctgaacagg aaaacctttg agagagaata ccccacgata aagcagaaga aacctgactt 6720

tatattggcc actgacatag ctgaaatggg agccaacctt tgcgtggagc gagtgctgga 6780

ttgcaggacg gcttttaagc ctgtgcttgt ggatgaaggg aggaaggtgg caataaaagg 6840

gccacttcgt atctccgcat cctctgctgc tcaaaggagg gggcgcattg ggagaaatcc 6900

caacagagat ggagactcat actactattc tgagcctaca agtgaaaata atgcccacca 6960

cgtctgctgg ttggaggcct caatgctctt ggacaacatg gaggtgaggg gtggaatggt 7020

cgccccactc tatggcgttg aaggaactaa aacaccagtt tcccctggtg aaatgagact 7080

gagggatgac cagaggaaag tcttcagaga actagtgagg aattgtgacc tgcccgtttg 7140

gctttcgtgg caagtggcca aggctggttt gaagacgaat gatcgtaagt ggtgttttga 7200

aggccctgag gaacatgaga tcttgaatga cagcggtgaa acagtgaagt gcagggctcc 7260

tggaggagca aagaagcctc tgcgcccaag gtggtgtgat gaaagggtgt catctgacca 7320

gagtgcgctg tctgaattta ttaagtttgc tgaaggtagg aggggagctg ctgaagtgct 7380

agttgtgctg agtgaactcc ctgatttcct ggctaaaaaa ggtggagagg caatggatac 7440

catcagtgtg ttcctccact ctgaggaagg ctctagggct taccgcaatg cactatcaat 7500

gatgcctgag gcaatgacaa tagtcatgct gtttatactg gctggactac tgacatcggg 7560

aatggtcatc tttttcatgt ctcccaaagg catcagtaga atgtctatgg cgatgggcac 7620

aatggccggc tgtggatatc tcatgttcct tggaggcgtc aaacccactc acatctccta 7680

tgtcatgctc atattctttg tcctgatggt ggttgtgatc cccgagccag ggcaacaaag 7740

gtccatccaa gacaaccaag tggcatacct cattattggc atcctgacgc tggtttcagc 7800

ggtggcagcc aacgagctag gcatgctgga gaaaaccaaa gaggacctct ttgggaagaa 7860

gaacttaatt ccatctagtg cttcaccctg gagttggccg gatcttgacc tgaagccagg 7920

agctgcctgg acagtgtacg ttggcattgt tacaatgctc tctccaatgt tgcaccactg 7980

gatcaaagtc gaatatggca acctgtctct gtctggaata gcccagtcag cctcagtcct 8040

ttctttcatg gacaagggga taccattcat gaagatgaat atctcggtca taatgctgct 8100

ggtcagtggc tggaattcaa taacagtgat gcctctgctc tgtggcatag ggtgcgccat 8160

gctccactgg tctctcattt tacctggaat caaagcgcag cagtcaaagc ttgcacagag 8220

aagggtgttc catggcgttg ccaagaaccc tgtggttgat gggaatccaa cagttgacat 8280

tgaggaagct cctgaaatgc ctgcccttta tgagaagaaa ctggctctat atctccttct 8340

tgctctcagc ctagcttctg ttgccatgtg cagaacgccc ttttcattgg ctgaaggcat 8400

tgtcctagca tcagctgcct tagggccgct catagaggga aacaccagcc ttctttggaa 8460

tggacccatg gctgtctcca tgacaggagt catgaggggg aatcactatg cttttgtggg 8520

agtcatgtac aatctatgga agatgaaaac tggacgccgg gggagcgcga atggaaaaac 8580

tttgggtgaa gtctggaaga gggaactgaa tctgttggac aagcgacagt ttgagttgta 8640

taaaaggacc gacattgtgg aggtggatcg tgatacggca cgcaggcatt tggccgaagg 8700

gaaggtggac accggggtgg cggtctccag ggggaccgca aagttaaggt ggttccatga 8760

gcgtggctat gtcaagctgg aaggtagggt gattgacctg gggtgtggcc gcggaggctg 8820

gtgttactac gctgctgcgc aaaaggaagt gagtggggtc aaaggattta ctcttggaag 8880

agacggccat gagaaaccca tgaatgtgca aagtctggga tggaacatca tcaccttcaa 8940

ggacaaaact gatatccacc gcctagaacc agtgaaatgt gacacccttt tgtgtgacat 9000

tggagagtca tcatcgtcat cggtcacaga gggggaaagg accgtgagag ttcttgatac 9060

tgtagaaaaa tggctggctt gtggggttga caacttctgt gtgaaggtgt tagctccata 9120

catgccagat gttcttgaga aactggaatt gctccaaagg aggtttggcg gaacagtgat 9180

caggaaccct ctctccagga attccactca tgaaatgtac tacgtgtctg gagcccgcag 9240

caatgtcaca tttactgtga accaaacatc ccgcctcctg atgaggagaa tgaggcgtcc 9300

aactggaaaa gtgaccctgg aggctgacgt catcctccca attgggacac gcagtgttga 9360
gacagacaag ggacccctgg acaaagaggc catagaagaa agggttgaga ggataaaatc 9420
tgagtacatg acctcttggt tttatgacaa tgacaacccc tacaggacct ggcactactg 9480
tggctcctat gtcacaaaaa cctcaggaag tgcggcgagc atggtaaatg gtgttattaa 9540
aattctgaca tatccatggg acaggataga ggaggtcacc agaatggcaa tgactgacac 9600
aacccctttt ggacagcaaa gagtgtttaa agaaaaagtt gacaccagag caaaggatcc 9660
accagcggga actaggaaga tcatgaaagt tgtcaacagg tggctgttcc gccacctggc 9720
cagagaaaag agccccagac tgtgcacaaa ggaagaattt attgcaaaag tccgaagtca 9780
tgcagccatt ggagcttacc tggaagaaca agaacagtgg aagactgcca atgaggctgt 9840
ccaagaccca aagttctggg aactggtgga tgaagaaagg aagctgcacc aacaaggcag 9900
gtgtcggact tgtgtgtaca acatgatggg gaaaagagag aagaagctgt cagagtttgg 9960
gaaagcaaag ggaagccgtg ccatatggta tatgtggctg ggagcgcggt atcttgagtt 10020
tgaggccctg ggattcctga atgaggacca ttgggcttcc agggaaaact caggaggagg 10080
agtggaaggc attggcttac aatacctagg atatgtgatc agagacctgg ctgcaatgga 10140
tggtggtgga ttctacgcgg atgacaccgc tggatgggac acgcgcatca cagaggcaga 10200
ccttgatgat gaacaggaga tcttgaacta catgagccca catcacaaaa aactggcaca 10260
agcagtgatg gaaatgacat acaagaacaa agtggtgaaa gtgttgagac cagccccagg 10320
agggaaagcc tacatggatg tcataagtcg acgagaccag agaggatccg ggcaggtagt 10380
gacttatgct ctgaacacca tcaccaactt gaaagtccaa ttgatcagaa tggcagaagc 10440
agagatggtg atacatcacc aacatgttca agattgtgat gaatcagttc tgaccaggct 10500
ggaggcatgg ctcactgagc acggatgtaa cagactgaag aggatggcgg tgagtggaga 10560
cgactgtgtg gtccggccca tcgatgacag gttcggcctg gccctgtccc atctcaacgc 10620
catgtccaag gttagaaagg acatatctga atggcagcca tcaaaagggt ggaatgattg 10680
ggagaatgtg cccttctgtt cccaccactt ccatgaacta cagctgaagg atggcaggag 10740
gattgtggtg ccttgccgag aacaggacga gctcattggg agaggaaggg tgtctccagg 10800
aaacggctgg atgatcaagg aaacagcttg cctcagcaaa gcctatgcca acatgtggtc 10860
actgatgtat tttcacaaaa gggacatgag gctactgtca ttggctgttt cctcagctgt 10920
tcccacctca tgggttccac aaggacgcac aacatggtcg attcatggga aaggggagtg 10980
gatgaccacg gaagacatgc ttgaggtgtg gaacagagta tggataacca acaacccaca 11040
catgcaggac aagacaatgg tgaaaaaatg gagagatgtc ccttatctaa ccaagagaca 11100
agacaagctg tgcggatcac tgattggaat gaccaatagg gccacctggg cctcccacat 11160
ccatttagtc atccatcgta tccgaacgct gattggacag gagaaataca ctgactacct 11220

aacagtcatg gacaggtatt ctgtggatgc tgacctgcaa ctgggtgagc ttatctgaaa 11280

caccatctaa caggaataac cgggatacaa accacgggtg gagaaccgga ctccccacaa 11340

cctgaaaccg ggatataaac cacggctgga gaaccgggct ccgcacttaa aatgaaacag 11400

aaaccgggat aaaaactacg gatggagaac cggactccac acattgagac agaagaagtt 11460

gtcagcccag aaccccacac gagttttgcc actgctaagc tgtgaggcag tgcaggctgg 11520

gacagccgac ctccaggttg cgaaaaacct ggtttctggg acctcccacc ccagagtaaa 11580

aagaacggag cctccgctac caccctccca cgtggtggta gaaagacggg gtctagaggt 11640

tagaggagac cctccaggga acaaatagtg ggaccatatt gacgccaggg aaagaccgga 11700

gtggttctct gcttttcctc cagaggtctg tgagcacagt ttgctcaaga ataagcagac 11760

ctttggatga caaacacaaa accac 11785

<210> 8
<211> 3718
<212> PRT
<213> Simian immunodeficiency virus

<400> 8

Ser Gly Arg Lys Ala Gln Gly Lys Thr Leu Gly Val Asn Met Val Arg
1               5                   10                  15

Arg Gly Val Arg Ser Leu Ser Asn Lys Ile Lys Gln Lys Thr Lys Gln
            20                  25                  30

Ile Gly Asn Arg Pro Gly Pro Ser Arg Gly Val Gln Gly Phe Ile Phe
        35                  40                  45

Phe Phe Leu Phe Asn Ile Leu Thr Gly Lys Lys Ile Thr Ala His Leu
    50                  55                  60

Lys Arg Leu Trp Lys Met Leu Asp Pro Arg Gln Gly Leu Ala Val Leu
65                  70                  75                  80

Arg Lys Val Lys Arg Val Val Ala Ser Leu Met Arg Gly Leu Ser Ser
                85                  90                  95

Arg Lys Arg Arg Ser His Asp Val Leu Thr Val Gln Phe Leu Ile Leu
            100                 105                 110

Gly Met Leu Leu Met Thr Gly Gly Val Thr Leu Val Arg Lys Asn Arg
        115                 120                 125

Trp Leu Leu Leu Asn Val Thr Ser Glu Asp Leu Gly Lys Thr Phe Ser
    130                 135                 140

Val Gly Thr Gly Asn Cys Thr Thr Asn Ile Leu Glu Ala Lys Tyr Trp

145                 150                 155                 160

Cys Pro Asp Ser Met Glu Tyr Asn Cys Pro Asn Leu Ser Pro Arg Glu
                165                 170                 175

Glu Pro Asp Asp Ile Asp Cys Trp Cys Tyr Gly Val Glu Asn Val Arg
            180                 185                 190

Val Ala Tyr Gly Lys Cys Asp Ser Ala Gly Arg Ser Arg Arg Ser Arg
        195                 200                 205

Arg Ala Ile Asp Leu Pro Thr His Glu Asn His Gly Leu Lys Thr Arg
    210                 215                 220

Gln Glu Lys Trp Met Thr Gly Arg Met Gly Glu Arg Gln Leu Gln Lys
225                 230                 235                 240

Ile Glu Arg Trp Phe Val Arg Asn Pro Phe Phe Ala Val Thr Ala Leu
                245                 250                 255

Thr Ile Ala Tyr Leu Val Gly Ser Asn Met Thr Gln Arg Val Val Ile
            260                 265                 270

Ala Leu Leu Val Leu Ala Val Gly Pro Ala Tyr Ser Ala His Cys Ile
        275                 280                 285

Gly Ile Thr Asp Arg Asp Phe Ile Glu Gly Val His Gly Gly Thr Trp
    290                 295                 300

Val Ser Ala Thr Leu Glu Gln Asp Lys Cys Val Thr Val Met Ala Pro
305                 310                 315                 320

Asp Lys Pro Ser Leu Asp Ile Ser Leu Glu Thr Val Ala Ile Asp Arg
                325                 330                 335

Pro Ala Glu Ala Arg Lys Val Cys Tyr Asn Ala Val Leu Thr His Val
            340                 345                 350

Lys Ile Asn Asp Lys Cys Pro Ser Thr Gly Glu Ala His Leu Ala Glu
        355                 360                 365

Glu Asn Glu Gly Asp Asn Ala Cys Lys Arg Thr Tyr Ser Asp Arg Gly
    370                 375                 380

Trp Gly Asn Gly Cys Gly Leu Phe Gly Lys Gly Ser Ile Val Ala Cys
385                 390                 395                 400

Ala Lys Phe Thr Cys Ala Lys Ser Met Ser Leu Phe Glu Val Asp Gln
                405                 410                 415

Thr Lys Ile Gln Tyr Val Ile Arg Ala Gln Leu His Val Gly Ala Lys
            420                 425                 430

Gln Glu Asn Trp Asn Thr Ser Ile Lys Thr Leu Lys Phe Asp Ala Leu
        435                 440                 445

Ser Gly Ser Gln Glu Val Glu Phe Ile Gly Tyr Gly Lys Ala Thr Leu
    450                 455                 460

Glu Cys Gln Val Gln Thr Ala Val Asp Phe Gly Asn Ser Tyr Ile Ala
465                 470                 475                 480

Glu Met Glu Thr Glu Ser Trp Ile Val Asp Arg Gln Trp Ala Gln Asp
                485                 490                 495

Leu Thr Leu Pro Trp Gln Ser Gly Ser Gly Gly Val Trp Arg Glu Met
            500                 505                 510

His His Leu Val Glu Phe Glu Pro Pro His Ala Ala Thr Ile Arg Val
        515                 520                 525

Leu Ala Leu Gly Asn Gln Glu Gly Ser Leu Lys Thr Ala Leu Thr Gly
    530                 535                 540

Ala Met Arg Val Thr Lys Asp Thr Asn Asp Asn Asn Leu Tyr Lys Leu
545                 550                 555                 560

His Gly Gly His Val Ser Cys Arg Val Lys Leu Ser Ala Leu Thr Leu
                565                 570                 575

Lys Gly Thr Ser Tyr Lys Ile Cys Thr Asp Lys Met Phe Phe Val Lys
            580                 585                 590

Asn Pro Thr Asp Thr Gly His Gly Thr Val Val Met Gln Val Lys Val
        595                 600                 605

Ser Lys Gly Thr Pro Cys Arg Ile Pro Val Ile Val Ala Asp Asp Leu
    610                 615                 620

Thr Ala Ala Ile Asn Lys Gly Ile Leu Val Thr Val Asn Pro Ile Ala
625                 630                 635                 640

Ser Thr Asn Asp Asp Glu Val Leu Ile Glu Val Asn Pro Pro Phe Gly
                645                 650                 655

Asp Ser Tyr Ile Ile Val Gly Arg Gly Asp Ser Arg Leu Thr Tyr Gln
            660                 665                 670

Trp His Lys Glu Gly Ser Ser Ile Gly Lys Leu Phe Thr Gln Thr Met
        675                 680                 685

Lys Gly Val Glu Arg Leu Ala Val Met Gly Asp Thr Ala Trp Asp Phe
    690                 695                 700

Ser Ser Ala Gly Gly Phe Phe Thr Ser Val Gly Lys Gly Ile His Thr
705                 710                 715                 720

Val Phe Gly Ser Ala Phe Gln Gly Leu Phe Gly Gly Leu Asn Trp Ile
                725                 730                 735

Thr Lys Val Ile Met Gly Ala Val Leu Ile Trp Val Gly Ile Asn Thr
            740                 745                 750

Arg Asn Met Thr Met Ser Met Ser Met Ile Leu Val Gly Val Ile Met
        755                 760                 765

Met Phe Leu Ser Leu Gly Val Gly Ala Asp Gln Gly Ser Ala Ile Asn
    770                 775                 780

Phe Gly Arg Gly Leu Ala Glu Ser Leu Leu Glu Asn Lys Glu Gly Cys
785                 790                 795                 800

Gln Lys Ile Leu Ser Val Leu Ala Pro Leu Val Pro Thr Gly Ser Glu
                805                 810                 815

Asn Leu Lys Ser Leu Tyr Asn Thr Val Cys Val Ile Trp Cys Ile His
            820                 825                 830

Ala Glu Glu Lys Val Lys His Thr Glu Glu Ala Lys Gln Ile Val Gln
        835                 840                 845

Arg His Leu Val Val Glu Thr Gly Thr Thr Glu Thr Met Pro Lys Thr
    850                 855                 860

Ser Arg Pro Thr Ala Pro Ser Ser Gly Arg Gly Gly Asn Tyr Pro Val
865                 870                 875                 880

Gln Gln Ile Gly Gly Asn Tyr Val His Leu Pro Leu Ser Pro Arg Thr
                885                 890                 895

Leu Asn Ala Trp Val Lys Leu Ile Glu Glu Lys Lys Phe Gly Ala Glu
            900                 905                 910

Val Val Pro Gly Phe Gln Ala Leu Ser Glu Gly Cys Thr Pro Tyr Asp
        915                 920                 925

Ile Asn Gln Met Leu Asn Cys Val Gly Asp His Gln Ala Ala Met Gln
    930                 935                 940

Ile Ile Arg Asp Ile Ile Asn Glu Glu Ala Ala Asp Trp Asp Leu Gln
945                 950                 955                 960

His Pro Gln Pro Ala Pro Gln Gln Gly Gln Leu Arg Glu Pro Ser Gly
                965                 970                 975

Ser Asp Ile Ala Gly Thr Thr Ser Ser Val Asp Glu Gln Ile Gln Trp
            980                 985                 990

Met Tyr Arg Gln Gln Asn Pro Ile  Pro Val Gly Asn Ile  Tyr Arg Arg
        995                 1000                 1005

Trp Ile  Gln Leu Lys Glu Gly  Ser Ser Ile Gly Thr  Ser Leu Gly
    1010                 1015                 1020

Lys Ala  Val His Gln Val Phe  Gly Ser Val Tyr Thr  Thr Met Phe
    1025                 1030                 1035

Gly Gly  Val Ser Trp Met Ile  Arg Ile Leu Ile Gly  Phe Leu Val
    1040                 1045                 1050

Leu Trp  Ile Gly Thr Asn Ser  Arg Asn Thr Ser Met  Ala Met Thr
    1055                 1060                 1065

Cys Ile  Ala Val Gly Gly Ile  Thr Leu Phe Leu Gly  Phe Thr Val
    1070                 1075                 1080

Gly Ala  Asp Gln Gly Cys Ala  Ile Asn Phe Gly Lys  Arg Glu Leu
    1085                 1090                 1095

Lys Cys  Gly Asp Gly Ile Phe  Ile Phe Arg Asp Ser  Asp Asp Trp
    1100                 1105                 1110

Leu Asn  Lys Tyr Ser Tyr Tyr  Pro Glu Asp Pro Val  Lys Leu Ala
    1115                 1120                 1125

Ser Ile  Val Lys Ala Ser Phe  Glu Glu Gly Lys Cys  Gly Leu Asn
    1130                 1135                 1140

Ser Val  Asp Ser Leu Glu His  Glu Met Trp Arg Ser  Arg Ala Asp
    1145                 1150                 1155

Glu Ile  Asn Thr Ile Phe Glu  Glu Asn Glu Val Asp  Ile Ser Val
    1160                 1165                 1170

Val Val  Gln Asp Pro Lys Asn  Val Tyr Gln Arg Gly  Thr His Pro

1175                1180                1185

Phe Ser Arg Ile Arg Asp Gly Leu Gln Tyr Gly Trp Lys Thr Trp
    1190                1195                1200

Gly Lys Asn Leu Val Phe Ser Pro Gly Arg Lys Asn Gly Ser Phe
    1205                1210                1215

Ile Ile Asp Gly Lys Ser Arg Lys Glu Cys Pro Phe Ser Asn Arg
    1220                1225                1230

Val Trp Asn Ser Phe Gln Ile Glu Glu Phe Gly Thr Gly Val Phe
    1235                1240                1245

Thr Thr Arg Val Tyr Met Asp Ala Val Phe Glu Tyr Thr Ile Asp
    1250                1255                1260

Cys Asp Gly Ser Ile Leu Gly Ala Ala Val Asn Gly Lys Lys Ser
    1265                1270                1275

Ala His Gly Ser Pro Thr Phe Trp Met Gly Ser His Glu Val Asn
    1280                1285                1290

Gly Thr Trp Met Ile His Thr Leu Glu Ala Leu Asp Tyr Lys Glu
    1295                1300                1305

Cys Glu Trp Pro Leu Thr His Thr Ile Gly Thr Ser Val Glu Glu
    1310                1315                1320

Ser Glu Met Phe Met Pro Arg Ser Ile Gly Gly Pro Val Ser Ser
    1325                1330                1335

His Asn His Ile Pro Gly Tyr Lys Val Gln Thr Asn Gly Pro Trp
    1340                1345                1350

Met Gln Val Pro Leu Glu Val Lys Arg Glu Ala Cys Pro Gly Thr
    1355                1360                1365

Ser Val Ile Ile Asp Gly Asn Cys Asp Gly Arg Gly Lys Ser Thr
    1370                1375                1380

Arg Ser Thr Thr Asp Ser Gly Lys Val Ile Pro Glu Trp Cys Cys
    1385                1390                1395

Arg Ser Cys Thr Met Pro Pro Val Ser Phe His Gly Ser Asp Gly
    1400                1405                1410

Cys Trp Tyr Pro Met Glu Ile Arg Pro Arg Lys Thr His Glu Ser
    1415                1420                1425

His Leu Val Arg Ser Trp Val Thr Ala Gly Glu Ile His Ala Val
    1430                1435                1440

Pro Phe Gly Leu Val Ser Met Met Ile Ala Met Glu Val Val Leu
    1445                1450                1455

Arg Lys Arg Gln Gly Pro Lys Gln Met Leu Val Gly Gly Val Val
    1460                1465                1470

Leu Leu Gly Ala Met Leu Val Gly Gln Val Thr Leu Leu Asp Leu
    1475                1480                1485

Leu Lys Leu Thr Val Ala Val Gly Leu His Phe His Glu Met Asn
    1490                1495                1500

Asn Gly Gly Asp Ala Met Tyr Met Ala Leu Ile Ala Ala Phe Ser
    1505                1510                1515

Ile Arg Pro Gly Leu Leu Ile Gly Phe Gly Leu Arg Thr Leu Trp
    1520                1525                1530

Ser Pro Arg Glu Arg Leu Val Leu Thr Leu Gly Ala Ala Met Val
    1535                1540                1545

Glu Ile Ala Leu Gly Gly Val Met Gly Gly Leu Trp Lys Tyr Leu
    1550                1555                1560

Asn Ala Val Ser Leu Cys Ile Leu Thr Ile Asn Ala Val Ala Ser
    1565                1570                1575

Arg Lys Ala Ser Asn Thr Ile Leu Pro Leu Met Ala Leu Leu Thr
    1580                1585                1590

Pro Val Thr Met Ala Glu Val Arg Leu Ala Ala Met Phe Phe Cys
    1595                1600                1605

Ala Met Val Ile Ile Gly Val Leu His Gln Asn Phe Lys Asp Thr
    1610                1615                1620

Ser Met Gln Lys Thr Ile Pro Leu Val Ala Leu Thr Leu Thr Ser
    1625                1630                1635

Tyr Leu Gly Leu Thr Gln Pro Phe Leu Gly Leu Cys Ala Phe Leu
    1640                1645                1650

Ala Thr Arg Ile Phe Gly Arg Arg Ser Ile Pro Val Asn Glu Ala
    1655                1660                1665

Leu Ala Ala Ala Gly Leu Val Gly Val Leu Ala Gly Leu Ala Phe
    1670                1675                1680

Gln Glu Met Glu Asn Phe Leu Gly Pro Ile Ala Val Gly Gly Leu
    1685                1690                1695

Leu Met Met Leu Val Ser Val Ala Gly Arg Val Asp Gly Leu Glu
    1700                1705                1710

Leu Lys Lys Leu Gly Glu Val Ser Trp Glu Glu Glu Ala Glu Ile
    1715                1720                1725

Ser Gly Ser Ser Ala Arg Tyr Asp Val Ala Leu Ser Glu Gln Gly
    1730                1735                1740

Glu Phe Lys Leu Leu Ser Glu Glu Lys Val Pro Trp Asp Gln Val
    1745                1750                1755

Val Met Thr Ser Leu Ala Leu Val Gly Ala Ala Leu His Pro Phe
    1760                1765                1770

Ala Leu Leu Leu Val Leu Ala Gly Trp Leu Phe His Val Arg Gly
    1775                1780                1785

Ala Arg Arg Ser Gly Asp Val Leu Trp Asp Ile Pro Thr Pro Lys
    1790                1795                1800

Ile Ile Glu Glu Cys Glu His Leu Glu Asp Gly Ile Tyr Gly Ile
    1805                1810                1815

Phe Gln Ser Thr Phe Leu Gly Ala Ser Gln Arg Gly Val Gly Val
    1820                1825                1830

Ala Gln Gly Gly Val Phe His Thr Met Trp His Val Thr Arg Gly
    1835                1840                1845

Ala Phe Leu Val Arg Asn Gly Lys Lys Leu Ile Pro Ser Trp Ala
    1850                1855                1860

Ser Val Lys Glu Asp Leu Val Ala Tyr Gly Gly Ser Trp Lys Leu
    1865                1870                1875

Glu Gly Arg Trp Asp Gly Glu Glu Glu Val Gln Leu Ile Ala Ala
    1880                1885                1890

Val Pro Gly Lys Asn Val Val Asn Val Gln Thr Lys Pro Ser Leu
    1895                1900                1905

Phe Lys Val Arg Asn Gly Gly Glu Ile Gly Ala Val Ala Leu Asp
    1910                1915                1920

Tyr Pro Ser Gly Thr Ser Gly Ser Pro Ile Val Asn Arg Asn Gly
    1925                1930                1935

Glu Val Ile Gly Leu Tyr Gly Asn Gly Ile Leu Val Gly Asp Asn
    1940                1945                1950

Ser Phe Val Ser Ala Ile Ser Gln Thr Glu Val Lys Glu Glu Gly
    1955                1960                1965

Lys Glu Glu Leu Gln Glu Ile Pro Thr Met Leu Lys Lys Gly Met
    1970                1975                1980

Thr Thr Val Leu Asp Phe His Pro Gly Ala Gly Lys Thr Arg Arg
    1985                1990                1995

Phe Leu Pro Gln Ile Leu Ala Glu Cys Ala Arg Arg Arg Leu Arg
    2000                2005                2010

Thr Leu Val Leu Ala Pro Thr Arg Val Val Leu Ser Glu Met Lys
    2015                2020                2025

Glu Ala Phe His Gly Leu Asp Val Lys Phe His Thr Gln Ala Phe
    2030                2035                2040

Ser Ala His Gly Ser Gly Arg Glu Val Ile Asp Ala Met Cys His
    2045                2050                2055

Ala Thr Leu Thr Tyr Arg Met Leu Glu Pro Thr Arg Val Val Asn
    2060                2065                2070

Trp Glu Val Ile Ile Met Asp Glu Ala His Phe Leu Asp Pro Ala
    2075                2080                2085

Ser Ile Ala Ala Arg Gly Trp Ala Ala His Arg Ala Arg Ala Asn
    2090                2095                2100

Glu Ser Ala Thr Ile Leu Met Thr Ala Thr Pro Pro Gly Thr Ser
    2105                2110                2115

Asp Glu Phe Pro His Ser Asn Gly Glu Ile Glu Asp Val Gln Thr
    2120                2125                2130

Asp Ile Pro Ser Glu Pro Trp Asn Thr Gly His Asp Trp Ile Leu
    2135                2140                2145

Ala Asp Lys Arg Pro Thr Ala Trp Phe Leu Pro Ser Ile Arg Ala

2150                    2155                    2160

Ala Asn Val Met Ala Ala Ser Leu Arg Lys Ala Gly Lys Ser Val
    2165                    2170                    2175

Val Val Leu Asn Arg Lys Thr Phe Glu Arg Glu Tyr Pro Thr Ile
    2180                    2185                    2190

Lys Gln Lys Lys Pro Asp Phe Ile Leu Ala Thr Asp Ile Ala Glu
    2195                    2200                    2205

Met Gly Ala Asn Leu Cys Val Glu Arg Val Leu Asp Cys Arg Thr
    2210                    2215                    2220

Ala Phe Lys Pro Val Leu Val Asp Glu Gly Arg Lys Val Ala Ile
    2225                    2230                    2235

Lys Gly Pro Leu Arg Ile Ser Ala Ser Ser Ala Ala Gln Arg Arg
    2240                    2245                    2250

Gly Arg Ile Gly Arg Asn Pro Asn Arg Asp Gly Asp Ser Tyr Tyr
    2255                    2260                    2265

Tyr Ser Glu Pro Thr Ser Glu Asn Asn Ala His His Val Cys Trp
    2270                    2275                    2280

Leu Glu Ala Ser Met Leu Leu Asp Asn Met Glu Val Arg Gly Gly
    2285                    2290                    2295

Met Val Ala Pro Leu Tyr Gly Val Glu Gly Thr Lys Thr Pro Val
    2300                    2305                    2310

Ser Pro Gly Glu Met Arg Leu Arg Asp Asp Gln Arg Lys Val Phe
    2315                    2320                    2325

Arg Glu Leu Val Arg Asn Cys Asp Leu Pro Val Trp Leu Ser Trp
    2330                    2335                    2340

Gln Val Ala Lys Ala Gly Leu Lys Thr Asn Asp Arg Lys Trp Cys
    2345                    2350                    2355

Phe Glu Gly Pro Glu Glu His Glu Ile Leu Asn Asp Ser Gly Glu
    2360                    2365                    2370

Thr Val Lys Cys Arg Ala Pro Gly Gly Ala Lys Lys Pro Leu Arg
    2375                    2380                    2385

Pro Arg Trp Cys Asp Glu Arg Val Ser Ser Asp Gln Ser Ala Leu
    2390                    2395                    2400

Ser Glu Phe Ile Lys Phe Ala Glu Gly Arg Arg Gly Ala Ala Glu
    2405                2410                2415

Val Leu Val Val Leu Ser Glu Leu Pro Asp Phe Leu Ala Lys Lys
    2420                2425                2430

Gly Gly Glu Ala Met Asp Thr Ile Ser Val Phe Leu His Ser Glu
    2435                2440                2445

Glu Gly Ser Arg Ala Tyr Arg Asn Ala Leu Ser Met Met Pro Glu
    2450                2455                2460

Ala Met Thr Ile Val Met Leu Phe Ile Leu Ala Gly Leu Leu Thr
    2465                2470                2475

Ser Gly Met Val Ile Phe Phe Met Ser Pro Lys Gly Ile Ser Arg
    2480                2485                2490

Met Ser Met Ala Met Gly Thr Met Ala Gly Cys Gly Tyr Leu Met
    2495                2500                2505

Phe Leu Gly Gly Val Lys Pro Thr His Ile Ser Tyr Val Met Leu
    2510                2515                2520

Ile Phe Phe Val Leu Met Val Val Val Ile Pro Glu Pro Gly Gln
    2525                2530                2535

Gln Arg Ser Ile Gln Asp Asn Gln Val Ala Tyr Leu Ile Ile Gly
    2540                2545                2550

Ile Leu Thr Leu Val Ser Ala Val Ala Ala Asn Glu Leu Gly Met
    2555                2560                2565

Leu Glu Lys Thr Lys Glu Asp Leu Phe Gly Lys Lys Asn Leu Ile
    2570                2575                2580

Pro Ser Ser Ala Ser Pro Trp Ser Trp Pro Asp Leu Asp Leu Lys
    2585                2590                2595

Pro Gly Ala Ala Trp Thr Val Tyr Val Gly Ile Val Thr Met Leu
    2600                2605                2610

Ser Pro Met Leu His His Trp Ile Lys Val Glu Tyr Gly Asn Leu
    2615                2620                2625

Ser Leu Ser Gly Ile Ala Gln Ser Ala Ser Val Leu Ser Phe Met
    2630                2635                2640

Asp Lys Gly Ile Pro Phe Met Lys Met Asn Ile Ser Val Ile Met
    2645                2650                2655

Leu Leu Val Ser Gly Trp Asn Ser Ile Thr Val Met Pro Leu Leu
    2660                2665                2670

Cys Gly Ile Gly Cys Ala Met Leu His Trp Ser Leu Ile Leu Pro
    2675                2680                2685

Gly Ile Lys Ala Gln Gln Ser Lys Leu Ala Gln Arg Arg Val Phe
    2690                2695                2700

His Gly Val Ala Lys Asn Pro Val Val Asp Gly Asn Pro Thr Val
    2705                2710                2715

Asp Ile Glu Glu Ala Pro Glu Met Pro Ala Leu Tyr Glu Lys Lys
    2720                2725                2730

Leu Ala Leu Tyr Leu Leu Leu Ala Leu Ser Leu Ala Ser Val Ala
    2735                2740                2745

Met Cys Arg Thr Pro Phe Ser Leu Ala Glu Gly Ile Val Leu Ala
    2750                2755                2760

Ser Ala Ala Leu Gly Pro Leu Ile Glu Gly Asn Thr Ser Leu Leu
    2765                2770                2775

Trp Asn Gly Pro Met Ala Val Ser Met Thr Gly Val Met Arg Gly
    2780                2785                2790

Asn His Tyr Ala Phe Val Gly Val Met Tyr Asn Leu Trp Lys Met
    2795                2800                2805

Lys Thr Gly Arg Arg Gly Ser Ala Asn Gly Lys Thr Leu Gly Glu
    2810                2815                2820

Val Trp Lys Arg Glu Leu Asn Leu Leu Asp Lys Arg Gln Phe Glu
    2825                2830                2835

Leu Tyr Lys Arg Thr Asp Ile Val Glu Val Asp Arg Asp Thr Ala
    2840                2845                2850

Arg Arg His Leu Ala Glu Gly Lys Val Asp Thr Gly Val Ala Val
    2855                2860                2865

Ser Arg Gly Thr Ala Lys Leu Arg Trp Phe His Glu Arg Gly Tyr
    2870                2875                2880

Val Lys Leu Glu Gly Arg Val Ile Asp Leu Gly Cys Gly Arg Gly
    2885                2890                2895

Gly Trp Cys Tyr Tyr Ala Ala Ala Gln Lys Glu Val Ser Gly Val
    2900                2905                2910

Lys Gly Phe Thr Leu Gly Arg Asp Gly His Glu Lys Pro Met Asn
    2915                2920                2925

Val Gln Ser Leu Gly Trp Asn Ile Ile Thr Phe Lys Asp Lys Thr
    2930                2935                2940

Asp Ile His Arg Leu Glu Pro Val Lys Cys Asp Thr Leu Leu Cys
    2945                2950                2955

Asp Ile Gly Glu Ser Ser Ser Ser Ser Val Thr Glu Gly Glu Arg
    2960                2965                2970

Thr Val Arg Val Leu Asp Thr Val Glu Lys Trp Leu Ala Cys Gly
    2975                2980                2985

Val Asp Asn Phe Cys Val Lys Val Leu Ala Pro Tyr Met Pro Asp
    2990                2995                3000

Val Leu Glu Lys Leu Glu Leu Leu Gln Arg Arg Phe Gly Gly Thr
    3005                3010                3015

Val Ile Arg Asn Pro Leu Ser Arg Asn Ser Thr His Glu Met Tyr
    3020                3025                3030

Tyr Val Ser Gly Ala Arg Ser Asn Val Thr Phe Thr Val Asn Gln
    3035                3040                3045

Thr Ser Arg Leu Leu Met Arg Arg Met Arg Arg Pro Thr Gly Lys
    3050                3055                3060

Val Thr Leu Glu Ala Asp Val Ile Leu Pro Ile Gly Thr Arg Ser
    3065                3070                3075

Val Glu Thr Asp Lys Gly Pro Leu Asp Lys Glu Ala Ile Glu Glu
    3080                3085                3090

Arg Val Glu Arg Ile Lys Ser Glu Tyr Met Thr Ser Trp Phe Tyr
    3095                3100                3105

Asp Asn Asp Asn Pro Tyr Arg Thr Trp His Tyr Cys Gly Ser Tyr
    3110                3115                3120

Val Thr Lys Thr Ser Gly Ser Ala Ala Ser Met Val Asn Gly Val

3125                3130                3135

Ile Lys Ile Leu Thr Tyr Pro Trp Asp Arg Ile Glu Glu Val Thr
    3140                3145                3150

Arg Met Ala Met Thr Asp Thr Thr Pro Phe Gly Gln Gln Arg Val
    3155                3160                3165

Phe Lys Glu Lys Val Asp Thr Arg Ala Lys Asp Pro Pro Ala Gly
    3170                3175                3180

Thr Arg Lys Ile Met Lys Val Val Asn Arg Trp Leu Phe Arg His
    3185                3190                3195

Leu Ala Arg Glu Lys Ser Pro Arg Leu Cys Thr Lys Glu Glu Phe
    3200                3205                3210

Ile Ala Lys Val Arg Ser His Ala Ala Ile Gly Ala Tyr Leu Glu
    3215                3220                3225

Glu Gln Glu Gln Trp Lys Thr Ala Asn Glu Ala Val Gln Asp Pro
    3230                3235                3240

Lys Phe Trp Glu Leu Val Asp Glu Glu Arg Lys Leu His Gln Gln
    3245                3250                3255

Gly Arg Cys Arg Thr Cys Val Tyr Asn Met Met Gly Lys Arg Glu
    3260                3265                3270

Lys Lys Leu Ser Glu Phe Gly Lys Ala Lys Gly Ser Arg Ala Ile
    3275                3280                3285

Trp Tyr Met Trp Leu Gly Ala Arg Tyr Leu Glu Phe Glu Ala Leu
    3290                3295                3300

Gly Phe Leu Asn Glu Asp His Trp Ala Ser Arg Glu Asn Ser Gly
    3305                3310                3315

Gly Gly Val Glu Gly Ile Gly Leu Gln Tyr Leu Gly Tyr Val Ile
    3320                3325                3330

Arg Asp Leu Ala Ala Met Asp Gly Gly Gly Phe Tyr Ala Asp Asp
    3335                3340                3345

Thr Ala Gly Trp Asp Thr Arg Ile Thr Glu Ala Asp Leu Asp Asp
    3350                3355                3360

Glu Gln Glu Ile Leu Asn Tyr Met Ser Pro His His Lys Lys Leu
    3365                3370                3375

Ala Gln Ala Val Met Glu Met Thr Tyr Lys Asn Lys Val Val Lys
    3380                3385                3390

Val Leu Arg Pro Ala Pro Gly Gly Lys Ala Tyr Met Asp Val Ile
    3395                3400                3405

Ser Arg Arg Asp Gln Arg Gly Ser Gly Gln Val Val Thr Tyr Ala
    3410                3415                3420

Leu Asn Thr Ile Thr Asn Leu Lys Val Gln Leu Ile Arg Met Ala
    3425                3430                3435

Glu Ala Glu Met Val Ile His His Gln His Val Gln Asp Cys Asp
    3440                3445                3450

Glu Ser Val Leu Thr Arg Leu Glu Ala Trp Leu Thr Glu His Gly
    3455                3460                3465

Cys Asn Arg Leu Lys Arg Met Ala Val Ser Gly Asp Asp Cys Val
    3470                3475                3480

Val Arg Pro Ile Asp Asp Arg Phe Gly Leu Ala Leu Ser His Leu
    3485                3490                3495

Asn Ala Met Ser Lys Val Arg Lys Asp Ile Ser Glu Trp Gln Pro
    3500                3505                3510

Ser Lys Gly Trp Asn Asp Trp Glu Asn Val Pro Phe Cys Ser His
    3515                3520                3525

His Phe His Glu Leu Gln Leu Lys Asp Gly Arg Arg Ile Val Val
    3530                3535                3540

Pro Cys Arg Glu Gln Asp Glu Leu Ile Gly Arg Gly Arg Val Ser
    3545                3550                3555

Pro Gly Asn Gly Trp Met Ile Lys Glu Thr Ala Cys Leu Ser Lys
    3560                3565                3570

Ala Tyr Ala Asn Met Trp Ser Leu Met Tyr Phe His Lys Arg Asp
    3575                3580                3585

Met Arg Leu Leu Ser Leu Ala Val Ser Ser Ala Val Pro Thr Ser
    3590                3595                3600

Trp Val Pro Gln Gly Arg Thr Thr Trp Ser Ile His Gly Lys Gly
    3605                3610                3615

Glu Trp Met Thr Thr Glu Asp Met Leu Glu Val Trp Asn Arg Val
    3620                3625                3630

Trp Ile Thr Asn Asn Pro His Met Gln Asp Lys Thr Met Val Lys
    3635                3640                3645

Lys Trp Arg Asp Val Pro Tyr Leu Thr Lys Arg Gln Asp Lys Leu
    3650                3655                3660

Cys Gly Ser Leu Ile Gly Met Thr Asn Arg Ala Thr Trp Ala Ser
    3665                3670                3675

His Ile His Leu Val Ile His Arg Ile Arg Thr Leu Ile Gly Gln
    3680                3685                3690

Glu Lys Tyr Thr Asp Tyr Leu Thr Val Met Asp Arg Tyr Ser Val
    3695                3700                3705

Asp Ala Asp Leu Gln Leu Gly Glu Leu Ile
    3710                3715

<210>  9
<211>  508
<212>  PRT
<213>  Human immunodeficiency virus

<400>  9

Met Gly Ala Arg Ala Ser Val Leu Ser Gly Gly Lys Leu Asp Lys Trp
1               5                   10                  15

Glu Lys Ile Arg Leu Arg Pro Gly Gly Lys Lys Thr Tyr Gln Leu Lys
            20                  25                  30

His Ile Val Trp Ala Ser Arg Glu Leu Glu Arg Phe Ala Val Asn Pro
        35                  40                  45

Gly Leu Leu Glu Thr Gly Gly Gly Cys Lys Gln Ile Leu Val Gln Leu
    50                  55                  60

Gln Pro Ser Leu Gln Thr Gly Ser Glu Glu Leu Lys Ser Leu Tyr Asn
65                  70                  75                  80

Ala Val Ala Thr Leu Tyr Cys Val His Gln Gly Ile Glu Val Arg Asp
                85                  90                  95

Thr Lys Glu Ala Leu Asp Lys Ile Glu Glu Glu Gln Asn Lys Ser Lys
            100                 105                 110

Lys Lys Ala Gln Gln Ala Ala Ala Asp Thr Gly Asn Ser Ser Gln Val

115                 120                 125

Ser Gln Asn Tyr Pro Ile Val Gln Asn Leu Gln Gly Gln Met Val His
    130                 135                 140

Gln Ala Ile Ser Pro Arg Thr Leu Asn Ala Trp Val Lys Val Ile Glu
145                 150                 155                 160

Glu Lys Ala Phe Ser Pro Glu Val Ile Pro Met Phe Ser Ala Leu Ser
                165                 170                 175

Glu Gly Ala Thr Pro Gln Asp Leu Asn Thr Met Leu Asn Thr Val Gly
            180                 185                 190

Gly His Gln Ala Ala Met Gln Met Leu Lys Glu Thr Ile Asn Glu Glu
        195                 200                 205

Ala Ala Glu Trp Asp Arg Leu His Pro Ala His Ala Gly Pro Asn Ala
    210                 215                 220

Pro Gly Gln Met Arg Glu Pro Arg Gly Ser Asp Ile Ala Gly Thr Thr
225                 230                 235                 240

Ser Thr Leu Gln Glu Gln Ile Gly Trp Met Thr Ser Asn Pro Pro Val
                245                 250                 255

Pro Val Gly Glu Ile Tyr Lys Arg Trp Ile Ile Leu Gly Leu Asn Lys
            260                 265                 270

Ile Val Arg Met Tyr Ser Pro Val Ser Ile Leu Asp Ile Arg Gln Gly
        275                 280                 285

Pro Lys Glu Pro Phe Arg Asp Tyr Val Asp Arg Phe Tyr Lys Thr Leu
    290                 295                 300

Arg Ala Glu Gln Ala Ser Gln Asp Val Lys Asn Trp Met Thr Glu Thr
305                 310                 315                 320

Leu Leu Val Gln Asn Ala Asn Pro Asp Cys Lys Thr Ile Leu Lys Ala
                325                 330                 335

Leu Gly Pro Ala Ala Thr Leu Glu Glu Met Met Thr Ala Cys Gln Gly
            340                 345                 350

Val Gly Gly Pro Ser His Lys Ala Arg Ile Leu Ala Glu Ala Met Ser
        355                 360                 365

Gln Val Thr Ser Pro Ala Asn Ile Met Met Gln Arg Gly Asn Phe Arg
    370                 375                 380

Asn Gln Arg Lys Thr Ile Lys Cys Phe Asn Cys Gly Lys Glu Gly His
385                 390                 395                 400

Leu Ala Arg His Cys Arg Ala Pro Arg Lys Lys Gly Cys Trp Lys Cys
                405                 410                 415

Gly Arg Glu Gly His Gln Met Lys Asp Cys Thr Glu Arg Gln Ala Asn
            420                 425                 430

Phe Leu Gly Lys Ile Trp Pro Ser His Lys Gly Arg Pro Gly Asn Phe
        435                 440                 445

Leu Gln Ser Arg Pro Glu Pro Thr Ala Pro Pro Glu Glu Ser Phe Arg
    450                 455                 460

Phe Gly Glu Glu Thr Thr Thr Pro Pro Gln Lys Gln Glu Pro Leu Pro
465                 470                 475                 480

Ser Gln Lys Gln Glu Thr Ile Asp Lys Asp Leu Tyr Pro Leu Ala Ser
                485                 490                 495

Leu Lys Ser Leu Phe Gly Asn Asp Pro Ser Leu Gln
            500                 505